# EUROPÄISCHE PATENTANMELDUNG

(11) **EP 1 842 573 A2**
(43) Veröffentlichungstag der Anmeldung: **10.10.2007**
(21) Anmeldenummer: 07005873.0
(22) Anmeldetag: 22.03.2007
(51) Int. Cl.: A61Q 5/10

(54) **Aufhell- und/oder Färbemittel mit verbessertem Produktempfinden**

(30) Priorität: 06.04.2006 DE 102006016580
(71) Anmelder: Henkel Kommanditgesellschaft auf Aktien, 40589 Düsseldorf (DE)
(72) Erfinder: Döring, Thomas, 41542 Dormagen (DE); Bossmann, Britta, 40699 Erkrath (DE); Hollenberg, Detlev, 40699 Erkrath (DE)

(57) **Zusammenfassung**

Mittel zum Färben und/oder Aufhellen von Keratinfasern, insbesondere menschlichen Haaren, die- jeweils bezogen auf ihr Gewicht - 0,001 bis 10 Gew.-% eines oder mehrerer Oxidationsfarbstoffvorprodukte und/oder direktziehender Farbstoffe und 0,001 bis 5 Gew.-% mindestens einer ein Gefühl von Wärme erzeugenden Substanz enthalten, führen zu einer Verringerung des Irritationspotentials und zu einer verbesserten sensorischen Wahrnehmung dieser Mittel.

## Beschreibung

Die vorliegende Erfindung betrifft Mittel zum Färben und/oder Aufhellen keratinischer Fasern, d.h. Mittel zur Anwendung auf Keratinfasern, insbesondere menschlichen Haaren, und deren Verwendung.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Gleichzeitig besteht aber auch vielfach der Wunsch, die Haarfarbe zu ändern und zusammen mit der Haarfärbung auch eine Aufhellung des zu färbenden Haares zu erreichen.

Konventionelle Haarfärbemittel bestehen in der Regel aus mindestens einer Entwickler- und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Vor ihrer Anwendung auf menschliches Haar werden Haarfärbe- und/oder -aufhellungsmittel in fester oder pastöser Form mit verdünnter wäßriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 40 Minuten. Es ist naheliegend, daß bei den Benutzern dieser Haarfarben oder Blondiermittel ein Bedürfnis besteht, diese Einwirkungszeit zu verringern.

Weder die pastenförmigen noch die pulverförmigen Färbe- und/oder Blondiermittel, die heute auf dem Markt sind, können als optimal angesehen werden. Während die Färbe- und/oder Blondierwirkung auf dem Haar als auf die Verbraucherbedürfnisse zugeschnitten bezeichnet werden kann, bestehen doch noch eine Reihe von Nachteilen und Problemen sowohl bei Herstellung als auch bei der Handhabung dieser Mittel.

Nicht zuletzt durch die starke Beanspruchung der Haare, beispielsweise durch das Färben oder Dauerwellen als auch durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Färbung und/oder Aufhellung oder Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate wirken im allgemeinen bevorzugt an der Haaroberfläche. So sind Wirkstoffe bekannt, welche dem Haar Glanz, Halt, Fülle, bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann. Auch hierfür gibt es Wirkstoffe, die von ihrer Molekülgröße klein genug sind, um in den Cortex penetrieren zu können und zu einer innerstrukturellen Stabilisierung führen.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen. So führt die Anwendung von Aufhell- oder Färbemitteln gelegentlich zu Irritationen der Kopfhaut, die sich in stechenden Empfindungen oder in Juckreiz äußern können. Zusätzlich wird die Abkühlung der Kopfhaut - bedingt durch die langen Anwendungszeiten - als unangenehm empfunden.

Es wurde nun gefunden, daß sich bestimmte Wirkstoffkombinationen mit besonderem Vorteil in Haarfärbe- und -aufhellungsmittel einarbeiten lassen und zu einer Verringerung des Irritationspotentials und zu einer verbesserten sensorischen Wahrnehmung dieser Mittel führen.

Gegenstand der Erfindung ist in einer ersten Ausführungsform ein Mittel zum Färben und/oder Aufhellen von Keratinfasern, insbesondere menschlichen Haaren, enthaltend - jeweils bezogen auf sein Gewicht -
a) 0,001 bis 10 Gew.-% eines oder mehrerer Oxidationsfarbstoffvorprodukte und/oder direktziehender Farbstoffe;
b) 0,001 bis 5 Gew.-% mindestens einer ein Gefühl von Wärme erzeugenden Substanz.

Erfindungsgemäße Mittel können als Mittel zum Färben und/oder als Mittel zum Aufhellen keratinischer Fasern konfektioniert werden. Sind nur direktziehende Farbstoffe vorhanden, ist beispielsweise auch die Formulierung eines Färbeshampoos möglich. Unter keratinischen bzw. keratinhaltigen Fasern werden im Rahmen dieser Anmeldung Pelze, Wolle, Federn und insbesondere menschliche Haare verstanden.

Bevorzugte erfindungsgemäße Mittel sind Färbemittel, d.h. Mittel zur Veränderung der Farbe keratinischer Fasern. Unter diesen sind insbesondere die sogenannten Oxidationsfärbemittel bevorzugt. Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.

Erfindungsgemäß bevorzugte Mittel zum Färben und/oder Aufhellen keratinischer Fasern sind demnach dadurch gekennzeichnet, daß sie mindestens ein Oxidationsfarbstoffvorprodukt vom Entwicklertyp und/oder Kupplertyp enthalten.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.
Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthält das Färbemittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 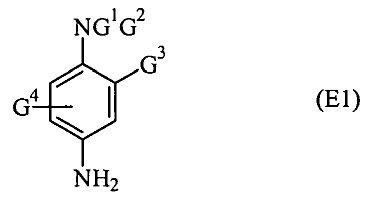 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Ct- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung verwendet werden können, kann man insbesondere die Verbindungen nennen, die der folgenden Formel (E2) entsprechen, sowie ihre physiologisch verträglichen Salze: 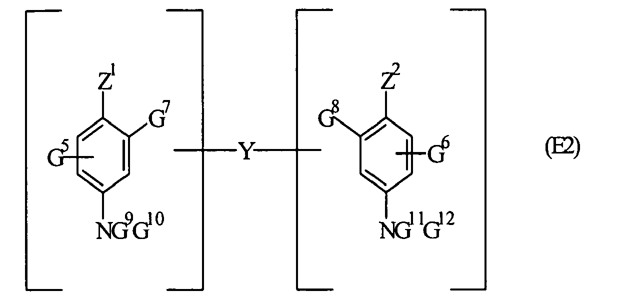 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 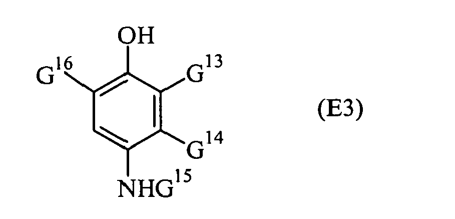 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 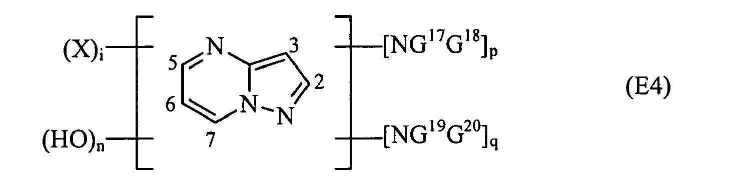 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

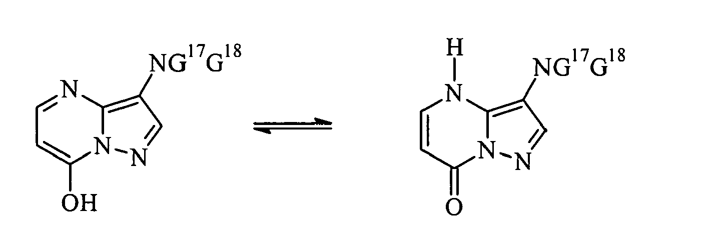

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß besonders bevorzugte Oxidationsfärbemittel sind dadurch gekennzeichnet, daß die Entwicklerkomponente ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diaminobenzol, 3-Methyl-4-aminophenol und 2-Methylamino-4-aminophenol, p-Phenylendiamin, 2-(ß-Hydroxyethyl)-p- phenylendiamin, N,N-Bis-(ß-hydroxyethyl)-p-phenylendiamin, N,N'-bis-(ß-Hydroxyethyl)-N,N'-bis- (4'-aminophenyl)-1,3-diamino-propan-2-ol, Bis-(2-hydroxy-5-aminophenyl)- methan, N,N'-Bis-(4'-aminophenyl)-1,4-diazacycloheptan, 1,10-Bis-(2',5'-diaminophenyl)-1,4,7,10-tetraoxadecan, p-Aminophenol, 4- Amino-3-fluorphenol, 4-Amino-2-aminomethylphenol, 4- Amino-2- ((diethylamino)methyl)phenol, o-Aminophenol, 2-Amino-4-methylphenol, 2-Amino-5- methylphenol, 2-Amino-4-chlorphenol, 2,4,5,6- Tetraaminopyrimidin, 4-Hydroxy-2,5,6- triaminopyrimidin, 2-Hydroxy-4,5,6- triaminopyrimidin, 2-Dimethylamino-4,5,6-triaminopyrimidin, 2,4- Dihydroxy-5,6-diaminopyrimidin, 2,5,6-Triaminopyrimidin, 1-(2'- Hydroxy-5'-aminobenzyl)-imidazolidin-2-on, 4,5-Diamino-1-(2'-hydroxyethyl) pyrazol und *N*-(4-Amino-3-methylphenyl)-*N*-[3-(1*H*-imidazol-1-yl)propyl]amin trihydrochlorid.

Erfindungsgemäß besonders bevorzugte Mittel zum Färben und/oder Aufhellen keratinischer Fasern sind dadurch gekennzeichnet, daß sie als Oxidationsfarbstoffvorprodukt mindestens eine Entwicklerkomponente enthalten, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol.

Die erfindungsgemäßen Färbemittel können weiterhin mindestens eine Kupplerkomponente enthalten.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIIa), 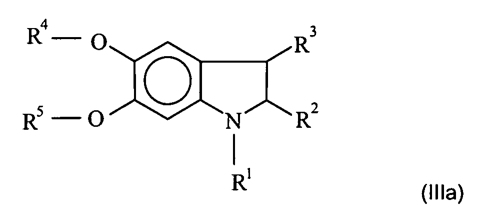 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIIb), 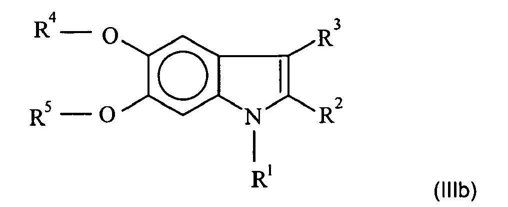 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Erfindungsgemäß besonders bevorzugte Oxidationsfärbemittel sind dadurch gekennzeichnet, daß die Kupplerkomponente ausgewählt ist aus m-Phenylendiaminderivaten, Naphtholen, Resorcin und Resorcinderivaten, Pyrazolonen, m-Aminophenolen und substituierten Pyridinderivaten, wobei bevorzugte Mittel Resorcin, 3-Amino-2-methylamino-6-methoxypyridin, 3-Amino-6-methylphenol, 3-Amino-2-hydroxypyridin, 1,3-Bis-(2,4-diaminophenoxy)propan, 2,7-Dihydroxynaphthalin, 2-Methylresorcin, 5-Methyl-resorcin, 2,5-Dimethylresorcin und 4-Chlorresorcin 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 3-Amino-4-(2-methoxyethoxy)-5-methylphenylamin und/oder 2-[(3-Morpholin-4-ylphenyl)amino]ethanol dihydrochlorid enthalten.

Erfindungsgemäße bevorzugte Mittel zum Färben und/oder Aufhellen keratinischer Fasern sind dadurch gekennzeichnet, daß sie als Oxidationsfarbstoffvorprodukt mindestens eine Kupplerkomponente enthalten, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

Zusammenfassend sind erfindungsgemäße Mittel zum Färben und/oder Aufhellen keratinischer Fasern bevorzugt, die als Oxidationsfarbstoffvorprodukt mindestens eine Entwicklerkomponente und/oder mindestens eine Kupplerkomponente enthalten, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol und bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

Vorzugsweise werden Kuppler- und Entwicklerkomponenten in einem bestimmten Verhältnis zueinander eingesetzt. Hier sind erfindungsgemäße Oxidationsfärbemittel bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Zusätzlich können die Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Erfindungsgemäß bevorzugte Mittel zum Färben und/oder Aufhellen keratinischer Fasern sind dadurch gekennzeichnet, daß sie mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

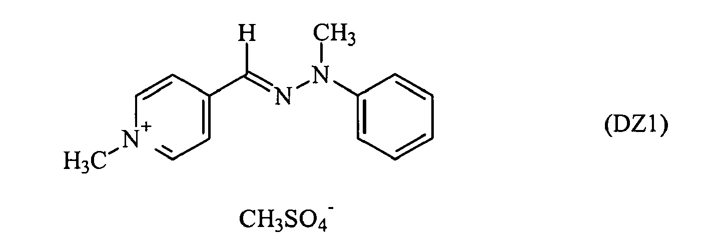

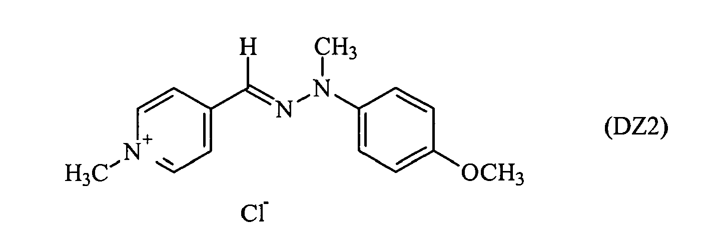

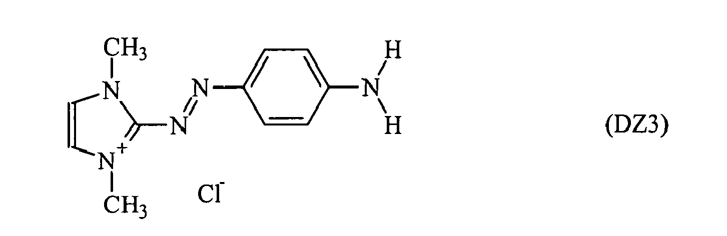

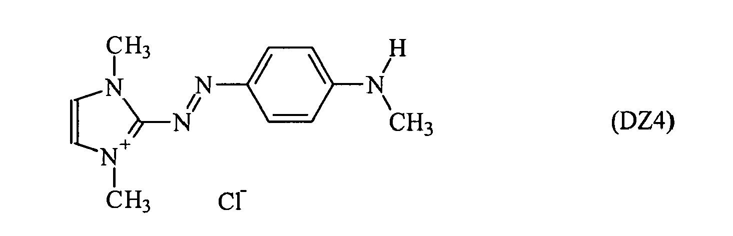

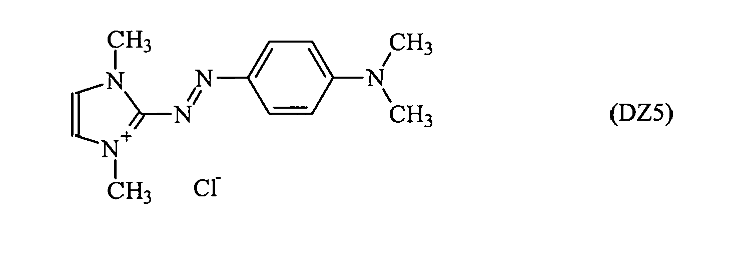

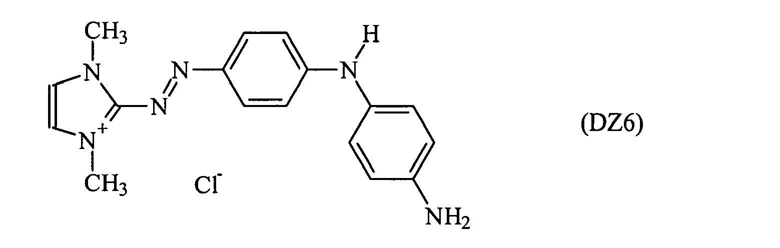

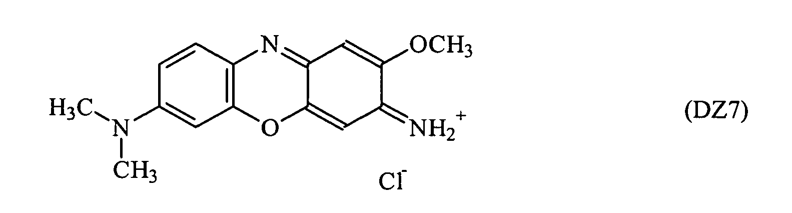

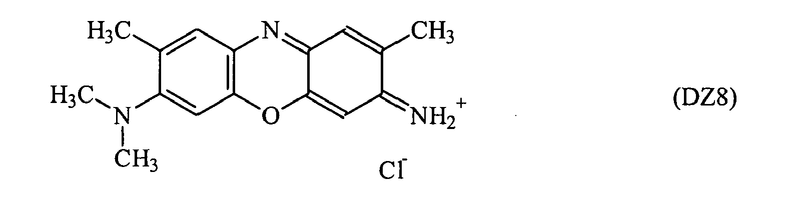

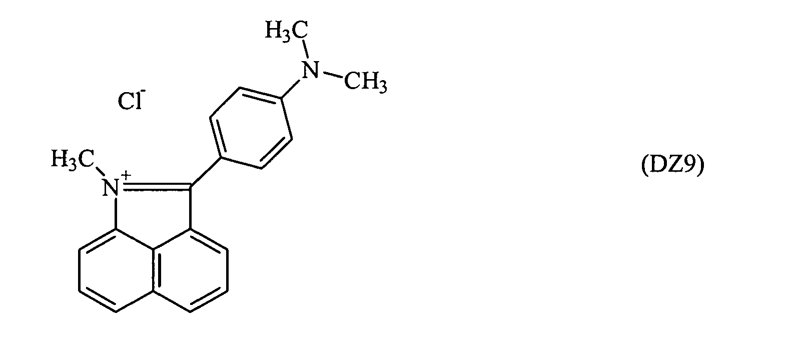

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Zusammenfassend sind erfindungsgemäße Mittel zum Färben und/oder Aufhellen keratinischer Fasern bevorzugt, die mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylam ino-1-hydroxy-4-nitrobenzol.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Eine Färbung keratinischer Fasern kann auch mittels Farbstoffen erfolgen, die in einer oxidativ katalysierten Reaktion von C,H-aciden Verbindungen mit reaktiven Carbonylverbindungen gebildet werden.

In einer weiteren Ausführungsform enthält das erfindungsgemäße Mittel daher eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung
c) von Wasser unter Bildung von Hydraten als Additionsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxybenzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxybenzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)-hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methyl-acridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1 -naphthyl)-3-methylbenzothiazolium-, 5-Chlor-2-(4'-formylphenyl)-3-methylbenzothiazolium-, 2-(4'-Formylphenyl)-3,5-dimethylbenzothiazoliumbenzolsulfonat, -p-toluolsulfonat, -methansulfonat, -perchlorat, -sulfat, -chlorid, -bromid, -iodid, - tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden in den erfindungsgemäßen Mitteln Benzaldehyd, Zimtaldehyd und Naphthaldehyd sowie deren Derivate, insbesondere mit einem oder mehreren Hydroxy-, Alkoxy- oder Aminosubstituenten, als reaktive Carbonylverbindung verwendet. Dabei werden wiederum die Verbindungen gemäß Formel (Ca-1) bevorzugt, 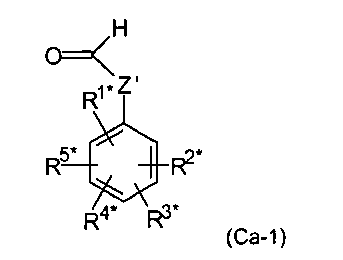 worin
- R^{1*}, R^{2*} und R^{3*} stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxygruppe, eine Sulfonsäuregruppe, eine Sulfonamidogruppe, eine Sulfonamidgruppe, eine Carbamoylgruppe, eine C₂-C₆-Acylgruppe oder eine Nitrogruppe,
- Z' steht für eine direkte Bindung oder eine Vinylengruppe,
- R⁴* und R⁵* stehen für ein Wasserstoffatom oder bilden gemeinsam, zusammen mit dem Restmolekül einen 5- oder 6-gliederigen aromatischen oder aliphatischen Ring.

Die Derivate der Benzaldehyde, Naphthaldehyde bzw. Zimtaldehyde der reaktiven Carbonylverbindung gemäß Komponente C werden besonders bevorzugt ausgewählt aus bestimmten Aldehyden. Hier sind erfindungsgemäße Mittel bevorzugt, die zusätzlich mindestens eine reaktive Carbonylverbindung enthalten, die ausgewählt wird, aus der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxybenzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

Erfindungsgemäß bevorzugte Mittel sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer reaktive Carbonylverbindung(en) enthalten.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methylnaphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenylpyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methylbenzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methylbenzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Geeignete Verbindungen mit primärer oder sekundärerAminogruppe als Komponente B sind z.B. primäre aromatische Amine wie N,N-Dimethyl-, N,N-Diethyl-, N- (2- Hydroxyethyl)-N-ethyl-, N,N-Bis-(2-hydroxyethyl)-, N-(2-Methoxyethyl-), 2,3-, 2,4-, 2,5- Dichlor-p-phenylendiamin, 2-Chlor-p-phenylendiamin, 2,5-Dihydroxy-4- morpholinoanilin- dihydrobromid, 2-, 3-, 4-Aminophenol, 2-Aminomethyl-4-aminophenoi, 2- Hydroxymethyl- 4-aminophenoi, o-, p-Phenylendiamin, o-Toluyiendiamin, 2,5-Diaminotoluol, -phenot, - phenethol, 4-Amino-3-methylphenoi, 2-(2,5-Diaminophenyl)-ethanoi, 2,4- Diaminophen- oxyethanoi, 2-(2,5-Diaminophenoxy)-ethanol, 4-Methylamino-, 3-Amino-4- (2'- hydroxyethyloxy)-, 3,4-Methylendiamino-, 3,4-Methylendioxyanilin, 3- Amino-2,4-dichlor-, 4-Methylamino-, 2-Methyl-5-amino-, 3-Methyl-4-amino-, 2-Methyl-5-(2-hydro- xyethylamino)-, 6-Methyl-3-amino-2-chlor-, 2-Methyl-5-amino-4-chlor-, 3, 4-Methylendioxy-, 5-(2-Hydroxyethylamino)-4-methoxy-2-methyl-, 4-Amino-2- hydroxymethylphenol, 1,3-Diamino-2,4-dimethoxybenzol, 2-, 3-, 4-Aminobenzoesäure, - phenylessigsäure, 2,3-, 2,4-, 2,5-, 3,4-, 3,5-Diaminobenzoesäure, 4-, 5-Aminosalicyisäure, 3- Amino-4-hydroxy-, 4-Amino-3-hydroxybenzoesäure, 2-, 3-, 4-Aminobenzolsulfonsäure, 3-Amino- 4- hydroxybenzolsulfonsäure, 4-Amino-3-hydroxynaphthalin-1 -sulfonsäure, 6- Amino-7- hydroxynaphthalin-2-sulfonsäure, 7-Amino-4-hydroxynaphthalin-2- sulfonsäure, 4-Amino- 5-hydroxynaphthalin-2,7-disulfonsäure, 3-Amino-2-naphthoesäure, 3- Aminophthalsäure, 5-Aminoisophthalsäure, 1,3,5-, 1,2,4-Triaminobenzol, 1,2,4,5- Tetraaminobenzol, 2,4,5- Triaminophenol, Pentaaminobenzol, Hexaaminobenzol, 2,4,6-Triaminoresorcin, 4,5- Diaminobrenzcatechin, 4,6-Diaminopyrogaliol, 3,5-Diamino-4-hydroxybrenzcatechin, aromatische Aniline bzw. Phenole mit einem weiteren aromatischen Rest, wie sie in der nachstehenden Formel dargestellt sind

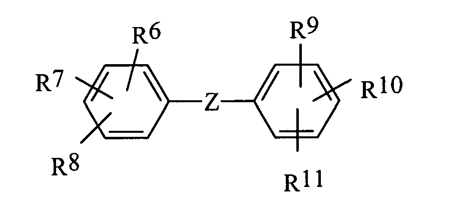

in der R6 für eine Hydroxy- oder eine Aminogruppe, die durch C1-4-Alkyl, Cl.4- Hydroxyalkyl- oderC1-4-Alkoxy-C1-4-alkyl substituiert sein kann, steht, R 7 , R", R9, R'O und RI' für Wasserstoff, eine Hydroxy- oder eine Aminogruppe, die durch eine Cl-4-Aikyl-, Cl-4-Hydroxyalkyl, CI-4-Aminoalkyl- oder CI-4-Alkoxy- C1-4-alkylgruppe sub- stituiert sein kann, für eine Carbondoer Sulfonsäuregruppe stehen, und Z für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel 111 Q-(CH2-p-CH2-Q')1 > (111) in der P eine direkte Bindung, eine CH2- oder CHOH-Gruppe bedeutet, Q und Q' unabhängig voneinander für ein Sauerstoffatom, eine NR 12 - Gruppe, worin R 12 Wasserstoff, eine Cl-,-Alkyl-, oder Hydroxy-C 1-4-alkylgruppe bedeutet, die Gruppe 0-(CH2)p-NH oder NH-(CH2)p,-0, worin p und p' 2 oder 3 sind, stehen und o eine Zahl von 1 bis 4 bedeutet, wie beispielsweise 4,4'-Diaminostilben, 4,4'-Diaminostilben-2,2'-disulfonsäure-mono- oder -di-Na-Salz, 4-Amino-4'-dimethylaminostilben, 4,4'-Diaminodiphenylmethan, -sulfid, -sulfoxid, -amin, 4,4'-Diaminodiphenyiamin-2-sulfonsäure, 4,4'-Diaminobenzophenon, - diphenylether, 3,3',4,4'-Tetraaminodiphenyl, 3,3',4,4'-Tetraaminobenzophenon, 1,3-Bis- (2,4-diaminophenoxy)-propan, 1,8-Bis-(2,5-diaminophenoxy)-3,6-dioxaoctan, 1,3-Bis-(4- aminophenylamino)-propan, -2-propanoi, 1,3-Bis-[N-(4-aminophenyl)-2-hydroxyethyla- mino]-2-propanol, N,N-Bis-[2-(4-aminophenoxy)-ethyl]-methylamin, N- Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z.B. 2-, 3-, 4-Amino-, 2- Amino-3-hydroxy-, 2,6-Diamino-, 2,5-Diamino-, 2,3-Diamino-, 2- Dimethylamino-5-amino-, 2-Methylamino-3-amino-6-methoxy-, 2,3-Diamino-6-methoxy-, 2,6-Dimethoxy- 3,5-dia- mino-, 2,4,5-Triamino-, 2,6-Dihydroxy-3,4-dimethylpyridin, 2,4-Dihydroxy- 5,6-diamino-, 4,5,6-Triamino-, 4-Hydroxy-2,5,6-triamino-, 2-Hydroxy-4,5,6-triamino-, 2, 4,5,6-Tetraamino-, 2-Methylamino-4,5,6-triamino-, 2,4-, 4,5-Diamino-, 2-Amino-4- methoxy-6-methyl- pyrimidin, 3,5-Diaminopyrazol, -1,2,4-triazol, 3-Amino-, 3-Amino-5- hydroxypyrazol, 2-,3-, 8-Aminochinolin, 4-Amino-chinaidin, 2-, 6-Aminonicotinsäure, 5- Aminoisochinolin, 5-, 6- Aminoindazol, 5-, 7-Amino-benzimidazol, - benzothiazol, 2,5-Dihydroxy-4- morpholinoani- lin sowie Indole und Indoline, sowie deren physiologisch verträgliche Salze, sein. Bevorzugte Beispiele für Indol- bzw. Indolinderivate 5,6-Dihydroxyindol, N- Methyl-5,6-dihy- droxyindol, N-Ethyl-5,6-dihydroxyindol, N-Propyl-5,6-dihydroxyindol, N- Butyl-5,6-dihy- droxyindol, 5,6-Dihydroxyindol-2-carbonsäure, 6-Hydroxyindol, 6- Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure. 6-Hydroxyindolin, 6-Aminoindolin und 4-Aminoindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Als Aminosäuren kommen bevorzugt alle natürlich vorkommenden und synthetischen (alpha-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Es können aber auch andere Aminosäuren verwendet werden, wie z. B. 6- Aminocapronsäure.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemittein ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Geeignete aromatische Hydroxyverbindungen sind z.B. 2-, 4-, 5- Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2- Hydroxyethyl)-, 3,4-Methylendioxyphenol, 2,4-, 3,4-Dihydroxybenzoesäure, - phenylessigsäure, Gallussäure, 2,4,6-Trihydroxybenzoesäure, -acetophenon, 2-, 4- Chlorresorcin, 1-Naphthol, 1,5-, 2,3-, 2,7-Dihydroxynaphthalin, 6- Dimethylamino-4- hydroxy-2-naphthalinsulfonsäure, 3,6-Dihydroxy-2,7-naphthalinsulfonsäure.

Als CH-aktive Verbindungen können beispielhaft genannt werden 1,2,3,3- Tetramethyl- 3H-indoliumiodid, 1,2,3,3-Tetramethyl-3H-indolium-p-toluoisulfonat, 1,2, 3,3-Tetramethyl- 3H-indolium-methansulfonat, Fischersche Base (1,3,3-Trimethyl-2- methylenindolin), 2,3- Dimethyl-benzothiazoliumiodid, 2,3-Dimethyl-benzothiazolium-p- toluolsulfonat, Rhodanin, Rhodanin-3-essigsäure, 1 -Ethyl-2-chinaldiniumiodid, 1 -Methyl-2- chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

Bevorzugt werden die CH-aciden Verbindungen aus den Formeln (II) und/oder (III) und/oder (IV) ausgewählt 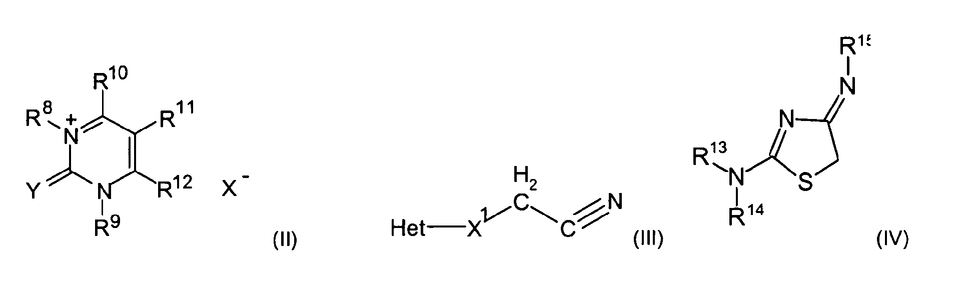 worin
- R⁸ und R⁹ stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₘ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe,
wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R¹⁰ und R¹² stehen unabhängig voneinander für ein Wasserstoffatom oder eine C₁-C₆-Alkylgruppe, wobei mindestens einer der Reste R¹⁰ und R¹² eine C₁-C₆-Alkylgruppe bedeutet,
- R¹¹ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}R^{IV}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R¹¹ zusammen mit einem der Reste R¹⁰ oder R¹² einen 5- oder 6-gliedrigen aromatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}R^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
- R¹³ und R¹⁴ bilden entweder zusammen mit dem Stickstoffatom einen gesättigten oder ungesättigten 5- oder 6-gliedrigen Ring oder stehen unabhängig voneinander für eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-(CH₂)ₘ-, worin R^{I} und R^{II} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe,
wobei R^{I} und R^{II} gemeinsam mit dem Stickstoffatom einen 5-, 6- oder 7-gliedrigen Ring bilden können und m steht für eine Zahl 2, 3, 4, 5 oder 6, und
- R¹⁵ ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrigen Ring bilden können und n steht für eine Zahl 2, 3, 4, 5 oder 6
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
- X⁻ steht für ein physiologisch verträgliches Anion,
- Het steht für einen gegebenenfalls substituierten Heteroaromaten,
- X¹ steht für eine direkte Bindung oder eine Carbonylgruppe.

Gleichwirkend zu den Verbindungen der Formel II sind deren Enaminformen. Es wird hier ausdrücklich auf die Druckschrift WO-A1-2004/022016 verwiesen, auf die vollinhaltlich Bezug genommen wird.

Mindestens eine Gruppe R¹⁰ oder R¹² gemäß Formel II steht zwingend für eine C₁-C₆-Alkylgruppe. Diese Alkylgruppe trägt an deren alpha-Kohlenstoffatom bevorzugt mindestens zwei Wasserstoffatome. Besonders bevorzugte Alkylgruppen sind die Methyl-, Ethyl-, Propyl-, n-Butyl-, iso-Butyl, n-Pentyl-, neo-Pentyl-, n-Hexylgruppe. Ganz besonders bevorzugt stehen R¹⁰ und R¹² unabhängig voneinander für Wasserstoff oder eine Methylgruppe, wobei mindestens eine Gruppe R¹⁰ oder R¹² eine Methylgruppe bedeutet.

In einer bevorzugten Ausführungsform steht Y für ein Sauerstoff- oder ein Schwefelatom, besonders bevorzugt für ein Sauerstoffatom.

Der Rest R⁸ wird bevorzugt ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

R¹¹ steht bevorzugt für ein Wasserstoffatom.

Besonders bevorzugt stehen die Reste R⁹, R¹⁰ und R¹² für eine Methylgruppe, der Rest R¹¹ für ein Wasserstoffatom, Y für ein Sauerstoff- oder ein Schwefelatom und der Rest R⁸ wird ausgewählt aus einer (C₁-C₆)-Alkylgruppe (besonders bevorzugt einer Methylgruppe), einer C₂-C₆-Alkenylgruppe (insbesondere einer Allylgruppe), einer Hydroxy-(C₂- bis C₆)-alkylgruppe, insbesondere einer 2-Hydroxyethylgruppe, oder einer gegebenenfalls substituierten Benzylgruppe.

Vorzugsweise sind die Verbindungen gemäß Formel II ausgewählt aus einer oder mehrerer Verbindungen der Gruppe von Salzen mit physiologisch verträglichem Gegenion X⁻, die gebildet wird aus Salzen des
1,2-Dihydro-1,3,4,6-tetramethyl-2-oxo-pyrimidiniums,
1,2-Dihydro-1,3-diethyl-4,6-dimethyl-2-oxo-pyrimidiniums,
1,2-Dihydro-1,3-dipropyl-4,6-dimethyl-2-oxo-pyrimidiniums,
1,2-Dihydro-1,3-di(2-hydroxyethyl)-4,6-dimethyl-2-oxo-pyrimidiniums,
1,2-Dihydro-1,3-diphenyl-4,6-dimethyl-2-oxo-pyrimidiniums,
1,2-Dihydro-1,3,4-trimethyl-2-oxo-pyrimidiniums,
1,2-Dihydro-1,3-diethyl-4-methyl-2-oxo-pyrimidiniums,
1,2-Dihydro-1,3-dipropyl-4-methyl-2-oxo-pyrimidiniums,
1,2-Dihydro-1,3-di(2-hydroxyethyl)-4-methyl-2-oxo-pyrimidiniums,
1,2-Dihydro-1,3-diphenyl-4-methyl-2-oxo-pyrimidiniums,
1-Allyl-1,2-dihydro-3,4,6-trimethyl-2-oxo-pyrimidiniums,
1,2-Dihydro-1-(2-hydroxyethyl)-3,4,6-trimethyl-2-oxo-pyrimidiniums,
1,2-Dihydro-1,3,4,6-tetramethyl-2-thioxo-pyrimidiniums,
1,2-Dihydro-1,3-diethyl-4,6-dimethyl-2-thioxo-pyrimidiniums,
1,2-Dihydro-1,3-dipropyl-4,6-dimethyl-2-thioxo-pyrimidiniums,
1,2-Dihydro-1,3-di(2-hydroxyethyl)-4,6-dimethyl-2-thioxo-pyrimidiniums,
1,2-Dihydro-1,3-diphenyl-4,6-dimethyl-2-thioxo-pyrimidiniums,
1,2-Dihydro-1,3,4-trimethyl-2-thioxo-pyrimidiniums,
1,2-Dihydro-1,3-diethyl-4-methyl-2-thioxo-pyrimidiniums,
1,2-Dihydro-1,3-dipropyl-4-methyl-2-thioxo-pyrimidiniums,
1,2-Dihydro-1,3-di(2-hydroxyethyl)-4-methyl-2-thioxo-pyrimidiniums,
1,2-Dihydro-1,3-diphenyl-4-methyl-2-thioxo-pyrimidiniums,
1,2-Dihydro-3,4-dimethyl-2-oxo-chinazoliniums und
1,2-Dihydro-3,4-dimethyl-2-thioxo-chinazoliniums.

Ganz besonders bevorzugte erfindungsgemäße Mittel sind dadurch gekennzeichnet, dass sie als CH-acide Verbindung
Salze des 1,2-Dihydro-1,3,4,6-tetramethyl-2-oxopyrimidiniums,
Salze des 1,2-Dihydro-1,3,4-trimethyl-2-oxopyrimidiniums,
Salze des 1,2-Dihydro-1,3,4,6-tetramethyl-2-thioxopyrimidiniums,
Salze des 1-Allyl-1,2-dihydro-3,4,6-trimethyl-2-oxopyrimidiniums,
Salze des 1,2-Dihydro-1-(2-hydroxyethyl)-3,4,6-trimethyl-2-oxopyrimidiniums, 2-(Cyanomethyl)benzimidazol,
4,5-Dihydro-4-imino-2-(1-piperidinyl)-thiazol und/oder dessen Hydrochlorid,
4,5-Dihydro-4-imino-2-(4-morpholinyl)-thiazol und/oder dessen Hydrochlorid,
4,5-Dihydro-4-imino-2-(1-pyrrolidinyl)-thiazol und/oder dessen Hydrochlorid
enthalten.

X⁻ steht in Formel (II) sowie in obigen Listen bevorzugt für Halogenid, Benzolsulfonat, p-Toluolsulfonat, C₁-C₄-Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt werden die Anionen Chlorid, Bromid, Iodid, Hydrogensulfat oder p-Toluolsulfonat als X- eingesetzt.

Der Rest Het gemäß Formel (III) steht bevorzugt für das Molekülfragment mit der Formel (V), 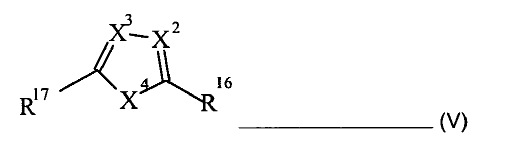 worin
- R¹⁶ und R¹⁷ stehen unabhängig voneinander für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe, eine Gruppe R^{VIII}R^{IX}N-(CH₂)ₘ-, worin R^{VIII} und R^{IX} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{VIII} und R^{IX} gemeinsam mit dem Stickstoffatom einen 5-, 6- oder 7-gliedrigen Ring bilden können und m steht für eine Zahl 0, 1, 2, 3 oder 4,
wobei R¹⁶ und/oder R¹⁷ einen an den Ring des Restmoleküls ankondensierten, gegebenenfalls substituierten aromatischen oder heteroaromatischen, 5- oder 6-Ring bilden können
- X² und X³ stehen unabhängig voneinander für ein Stickstoffatom oder eine Gruppe CR¹⁵,
wobei R¹⁵ steht für ein Wasserstoffatom, eine Hydroxygruppe, ein Halogenatom, eine Nitrogruppe, eine lineare oder zyklische C₁-C₆-Alkylgruppe, eine C₂-C₆-Alkenylgruppe, eine gegebenenfalls substituierte Arylgruppe, eine Cyanmethylgruppe, eine Cyanmethylcarbonylgruppe, eine gegebenenfalls substituierte Heteroarylguppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxygruppe, eine C₁-C₆-Alkoxycarbonylgruppe, eine C₁-C₆-Alkoxy-C₂-C₆-alkylgruppe, eine C₁-C₆-Sulfoalkylgruppe, eine C₁-C₆-Carboxyalkylgruppe und eine Gruppe R^{X}R^{XI}N-(CH₂)ₙ-, worin R^{X} und R^{XI} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{X} und R^{XI} gemeinsam mit dem Stickstoffatom einen 5-, 6- oder 7-gliedrigen Ring bilden können und n steht für eine Zahl 0, 1, 2, 3 oder 4,
wobei mindestens einer der Substituenten X² und X³ zusammen mit dem Restmolekül einen ankondensierten gegebenenfalls substituierten aromatischen 5- oder 6-Ring bilden kann,
- X⁴ steht für ein Sauerstoffatom, ein Schwefelatom, einer Vinylengruppe oder eine Gruppe N-H, wobei die beiden letztgenannten Gruppen unabhängig voneinander gegebenenfalls mit einer linearen oder zyklischen C₁-C₆-Alkylgruppe, einer C₂-C₆-Alkenylgruppe, einer gegebenenfalls substituierten Arylgruppe, einer gegebenenfalls substituierten Heteroarylguppe, einer Aryl-C₁-C₆-alkylgruppe, einer C₂-C₆-Hydroxyalkylgruppe, einer C₂-C₆-Polyhydroxyalkylgruppe, einer C₁-C₆-Alkoxy-C₂-C₆-alkylgruppe, einer C₁-C₆-Sulfoalkylgruppe, einer C₁-C₆-Carboxyalkylgruppe, einer Gruppe R^{XII}R^{XIII}N-(CH₂)ₚ- steht, worin R^{XII} und R^{XIII} stehen unabhängig voneinander für ein Wasserstoffatom, eine lineare oder zyklische C₁-C₆-Alkylgruppe, eine C₂-C₆-Alkenylgruppe, eine C₁-C₆-Hydroxyalkylgruppe oder eine Aryl-C₁-C₄-alkylgruppe, wobei R^{XII} und R^{XIII} gemeinsam mit dem Stickstoffatom einen 5-, 6- oder 7-gliedrigen Ring bilden können und p steht für eine Zahl 0, 1, 2, 3 oder 4, substituiert sein können,
mit der Maßgabe, daß, wenn X⁴ für eine Vinylengruppe steht, mindestens eine der Gruppen X² oder X³ ein Stickstoffatom bedeutet.

Die Bindung des heterozyklischen Rings gemäß Formel (V) zum Molekülfragment -X¹-CH₂-C≡N unter Erhalt der erfindungsgemäßen Verbindung gemäß Formel (III) erfolgt an den Ring des Heterozyklusses und ersetzt ein an diesen Ring gebundenes Wasserstoffatom. Folglich ist es zwingend notwendig, daß die Substituenten R¹⁶, R¹⁷, X², X³ und X⁴ derart gewählt werden müssen, daß mindestens einer dieser Substituenten eine entsprechende Bindungsbildung ermöglicht. Folglich ist es zwingend, daß mindestens einer der Reste R¹⁶ oder R¹⁷ die Bindung zum Molekülfragment -X¹-CH₂-C≡N ausbildet, wenn X⁴ ein Sauerstoffatom oder ein Schwefelatom ist und X² und X³ ein Stickstoffatom bedeuten.

Der Rest Het gemäß Formel (III) wird besonders bevorzugt abgeleitet von den Heteroaromaten Furan, Thiophen, Pyrrol, Isoxazol, Isothiazol, Imidazol, Oxazol, Thiazol, Pyridin, Pyridazin, Pyrimidin, Pyrazin, 1,2,3-Triazin, 1,2,4-Triazin, 1,3,5-Triazin, Benzopyrrol, Benzofuran, Benzothiophen, Benzimidazol, Benzoxazol, Indazol, Benzoisoxazol, Benzoisothiazol, Indol, Chinolin, Isochinolin, Cinnolin, Phthalazin, Chinazolin, Chinoxalin, Acridin, Benzochinolin, Benzoisochinolin, Benzothiazol, Phenazin, Benzocinnolin, Benzochinazolin, Benzochinoxalin, Phenoxazin, Phenothiazin, Nephthyridin, Phenanthrolin, Indolizin, Chinolizin, Carbolin, Purin, Pteridin und Cumarin, wobei die vorgenannten Heteroaromaten mit mindestens einer Gruppe ausgewählt aus einem Halogenatom, einer Nitrogruppe, einer Thiogruppe, einer Thio-(C₁-C₆)-alkylgruppe, einer Heteroarylgruppe, einer Arylgruppe, einer (C₁-C₆)-Alkylgruppe, einer (C₁-C₆)-Alkoxygruppe, einer Hydroxygruppe, einer (C₂-C₆)-Hydroxyalkylgruppe, einer (C₂-C₆)-Polyhydroxyalkylgruppe, einer (C₁-C₆)-Alkoxyl-(C₁-C₆)-alkylgruppe, einer Aryl-(C₁-C₆)-alkylgruppe, einer Aminogruppe, einer (C₁-C₆)-Monoalkylaminogruppe, einer (C₁-C₆)-Dialkylaminogruppe, eine Dialkylaminoalkylgruppe -(CH₂)ₙ-NR'R'', worin n eine ganze Zahl von 2 und 6 ist und R' und R" unabhängig voneinander eine lineare oder verzweigte Alkylgruppe bedeutet, welche gegebenenfalls zusammen einen Ring bilden können, substituiert sein können.

Vorzugsweise sind die Verbindungen gemäß Formel (III) ausgewählt aus der Gruppe bestehend aus 2-(2-Furoyl)-acetonitril, 2-(5-Brom-2-furoyl)-acetonitril, 2-(5-Methyl-2-trifluormethyl-3-furoyl)-acetonitril, 3-(2,5-Dimethyl-3-furyl)-3-oxopropanitril, 2-(2-Thenoyl)-acetonitril, 2-(3-Thenoyl)-acetonitril, 2-(5-Fluor-2-thenoyl)-acetonitril, 2-(5-Chlor-2-thenoyl)-acetonitril, 2-(5-Brom-2-thenoyl)-acetonitril, 2-(5-Methyl-2-thenoyl)-acetonitril, 2-(2,5-Dimethylpyrrol-3-oyl)-acetonitril, 2-(1,2,5-Trimethylpyrrol-3-oyl)-acetonitril, 1*H*-Benzimidazol-2-ylacetonitril, 1*H*-Benzothiazol-2-ylacetonitril, 2-(Pyrid-2-yl)-acetonitril, 2,6-Bis(cyanmethyl)-pyridin, 2-(Indol-3-oyl)-acetonitril, 2-(2-Methyl-indol-3-oyl)-acetonitril, 8-Cyanacetyl-7-methoxy-4-methylcumarin, 2-(2-Isopropyl-5,6-benzochinolin-4-oyl)-acetonitril, 2-(2-Phenyl-5,6-benzochinolin-4-oyl)-acetonitril, 2-(Chinoxalin-2-yl)-acetonitril, 2-(Cumaron-2-yl)-acetonitril, 6,7-Dichlor-5-(cyanoacetyl)-2,3-dihydro-1-benzofuran-2-carbonsäure-tert.-butylester, 2-(6-Hydroxy-4,7-dimethoxy-1-benzofuran-5-oyl)-acetonitril und 2-(1-Phenyl-1,4-dihydrothiochromeno[4,3-c]pyrazol-3-oyl)-acetonitril. Besonders bevorzugt ist 1H-Benzimidazol-2-ylacetonitril [2-(Cyanomethyl)benzimidazol].

Erfindungsgemäß bevorzugte Mittel sind dadurch gekennzeichnet, daß sie als Haarfärbemittel formuliert sind, die bezogen auf ihr Gewicht 0,01 bis 20 Gew.-%, vorzugsweise 0,05 bis 15 Gew.-%, besonders bevorzugt 0,1 bis 12,5 Gew.-% und insbesondere 0,2 bis 10 Gew.-% einer oder mehrerer CH-acider Verbindung(en) enthalten.

Als weiteren Inhaltstoff enthalten die erfindungsgemäßen Mittel mindestens eine ein Gefühl von Wärme erzeugenden Substanz in Mengen von 0,001 bis 5 Gew.-%, jeweils bezogen auf das Mittel. Erfindungsgemäß bevorzugte Mittel zum Färben und/oder Aufhellen keratinischer Fasern sind dadurch gekennzeichnet, daß sie 0,01 bis 4 Gew.-%, vorzugsweise 0,05 bis 3,5 Gew.-%, besonders bevorzugt 0,1 bis 3 Gew.-% und insbesondere 0,2 bis 2,5 Gew.-% mindestens einer ein Gefühl von Wärme erzeugenden Substanz enthält.

Als ein Gefühl von Wärme erzeugende Substanz kann eine Reihe von Stoffen eingesetzt werden. Bevorzugt sind beispielsweise Ether von Vanillin oder Isovanillin, die durch die nachstehenden Formeln wiedergegeben werden:

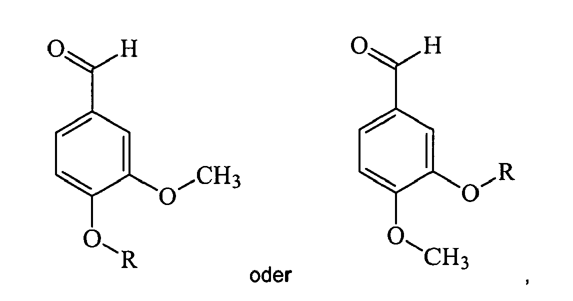

in denen R für einen Alkyl-, Alkenyl- oder Alkinylrest steht, der gegebenenfalls weiter substituiert sein kann, oder für einen Aryl- oder Heteroarylrest, der gegebenenfalls weiter substituiert sein kann.

Erfindungsgemäß bevorzugte Mittel zum Färben und/oder Aufhellen keratinischer Fasern sind dadurch gekennzeichnet, daß sie als ein Gefühl von Wärme erzeugende Substanz mindestens einen Ether von Vanillylakohol enthalten, vorzugsweise Vanillylalkohol methyl-ether und/oder Vanillylalkohol ethyl-ether, und/oder Vanillylalkohol n-propyl-ether und/oder Vanillylalkohol isopropyl-ether und/oder Vanillylalkohol n-butyl-ether und/oder Vanillylalkohol isobutyl-ether und/oder Vanillylalkohol n-amyl-ether und/oder Vanillylalkohol isoamyl-ether und/oder Vanillylalkohol n-hexyl-ether.

Weitere erfindungsgemäß bevorzugt einsetzbare Substanzen, die ein Gefühl von Wärme erzeugen, sind Nicotinsäureester, die durch die nachstehende Formel wiedergegeben werden: 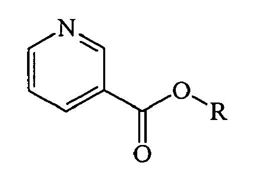 in der R für einen Alkyl-, Alkenyl- oder Alkinylrest steht, der gegebenenfalls weiter substituiert sein kann, oder für einen Aryl- oder Heteroarylrest, der gegebenenfalls weiter substituiert sein kann. Bevorzugt ist R ausgewählt aus Methyl-, Ethyl-, n-Propyl-, iso-Propyl, n-Butyl-, iso-Butyl-, tert-Butyl, n-Pentyl, n-Hexyl, Benzyl- oder Myristylresten.

Weitere erfindungsgemäß bevorzugt einsetzbare Substanzen, die ein Gefühl von Wärme erzeugen, sind Gingerol, das durch die nachstehende Formel wiedergegeben wird: 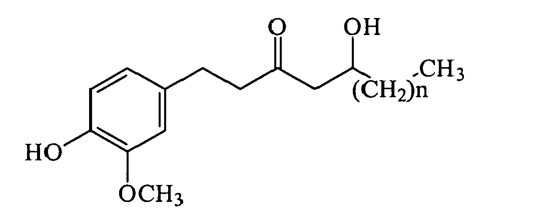 und in der n für 2, 3 oder 4 steht sowie Shoagol, das durch die nachstehende Formel wiedergegeben wird: 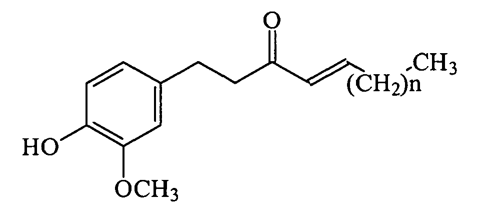 und in der n für 2, 3 oder 4 steht sowie Zingeron, das durch die nachstehende Formel wiedergegeben wird:

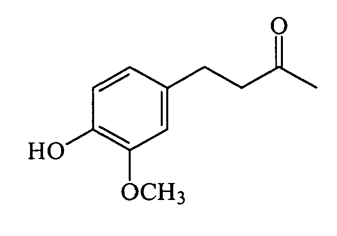

Erfindungsgemäß einsetzbar sind ferner Paradol, Capsaicin 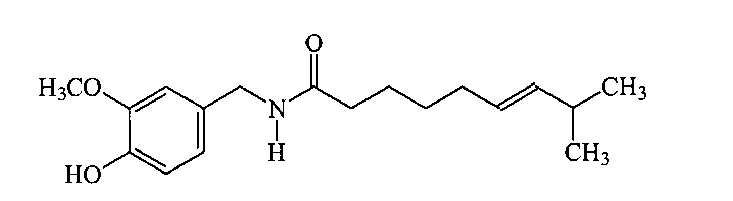 und/oder Dihydrocapsaicin 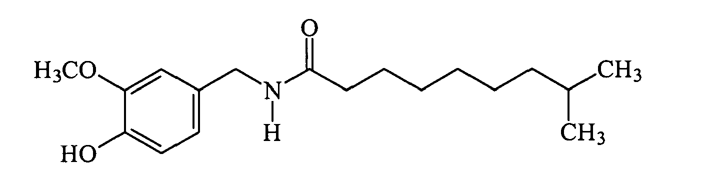 und/oder Homocapsaicin 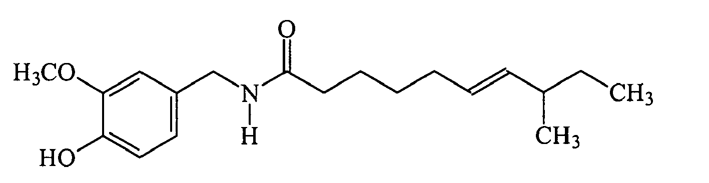 und/oder Homodihydrocapsaicin 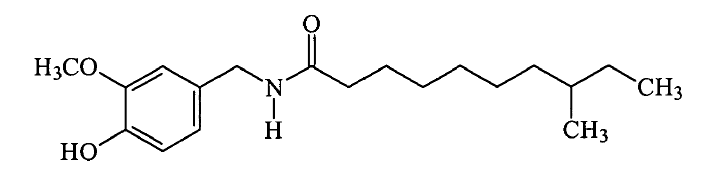 und/oder Norcapsaicin 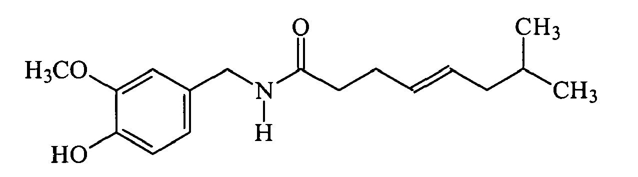 und/oder Nordihydrocapsaicin

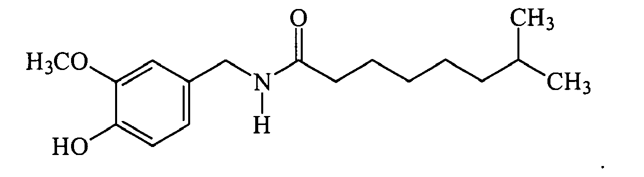

Zusammenfassend sind erfindungsgemäße Mittel zum Färben und/oder Aufhellen keratinischer Fasern bevorzugt, die als ein Gefühl von Wärme erzeugende Substanz mindestens eine Verbindung aus der Gruppe
- der Nicotinsäureester, vorzugsweise Methylnicotinat und/oder Ethylnicotinat und/oder Benzylnicotinat und/oder Butoxyethylnicotinat und/oder Hexylnicotinat und/oder Isopropylnicotinat und/oder Myristylnicotinat und/oder Thurfylniconinat und/oder
- Gingerol und/oder
- Shogaol und/oder
- Paradol und/oder
- Zingeron und/oder
- Capsaicin und/oder Capsaicin-haltiger Extrakte und/oder
- Dihydrocapsaicin und/oder
- Nordihydrocapsaicin und/oder
- Homocapsaicin und/oder
- Homodihydrocapsaicin und/oder
- Norcapsaicin und/oder
- Nordihydrocapsaicin
enthalten.

Als ein Gefühl von Wärme erzeugende Substanz können weiterhin bevorzugt N-Alkyl-substituierte Amide von ungesättigten Carbonsäuren eingesetzt werden, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid

- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid

- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid

- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid

- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid

- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid

- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid

- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid

- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid

- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid

- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt): 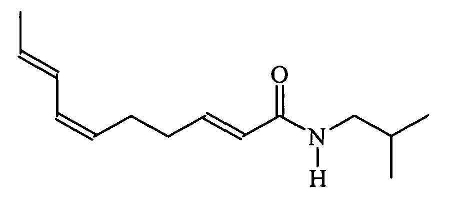 2E,4E,8Z-Decatriensäure-N-isobutylamid (N-Isobutyl-2E,4E,8Z-decatrienamid, auch Isoaffinin genannt): 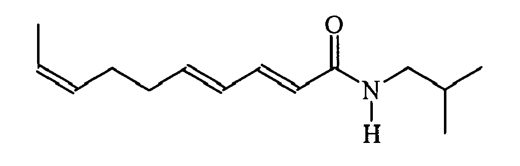 2E,7Z,9E-Undecatriensäure-N-isobutylamid (N-Isobutyl-2E,7Z,9E-undecatrienamid): 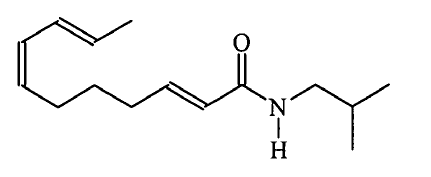 2E,4Z-Decadiensäure-N-isobutylamid (cis-Pellitorin): 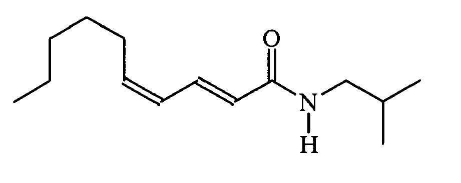 2E,4E-Decadiensäure-N-isobutylamid (trans-Pellitorin):

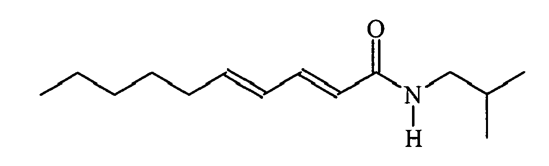

Ferulasäureamide, beispielsweise
Ferulasäure-N-Vanillylamid: 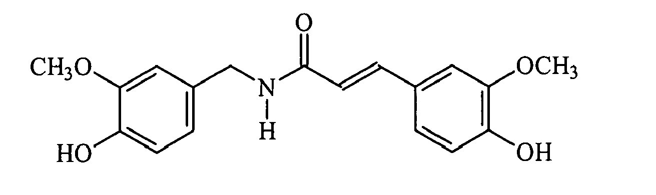 N-[2-(4-Hydroxy-3-methoxyphenyl)ethyl]-3-(4-hydroxy-3-methoxy-phenyl)- (2*E*)-propensäureamid (trans-Feruloylmethoxytyramin): 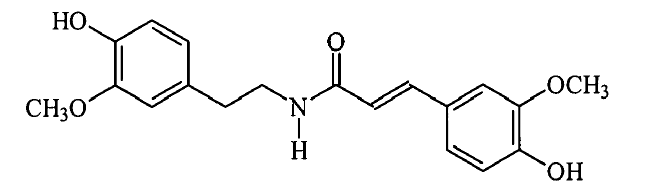 *N*-[2-(4-Hydroxy-3-methoxyphenyl)ethyl]-3-(4-hydroxy-3-methoxy-phenyl)-(2 Z)-propensäureamid (cis-Feruloylmethoxytyramin): 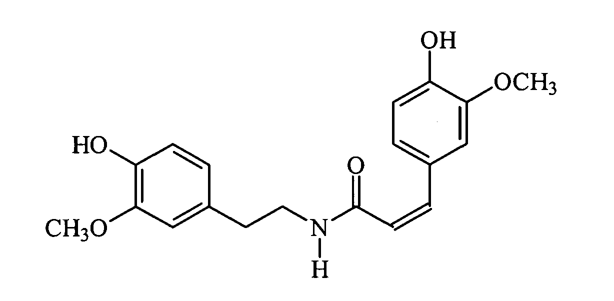 *N*-[2-(4- Hydroxy-3-methoxyphenyl)ethyl]-3-(4-hydroxy-3-methoxy-phenyl)- propansäureamid (Dihydroferuloylmethoxytyramin): 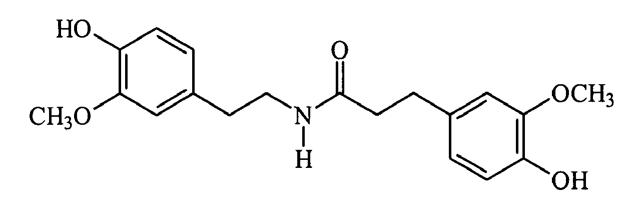 *N*-[2-(3,4- Dihydroxyphenyl)ethyl]-3-(4-hydroxy-3-methoxy-phenyl)-(2*E*)- propensäureamid (trans-Feruloyldopamin): 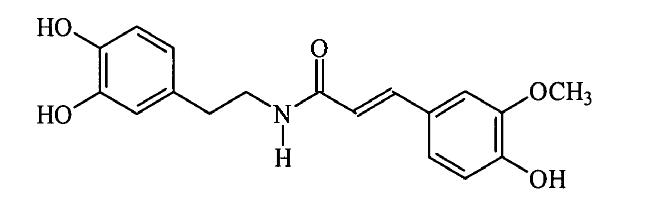 *N*-[2-(3,4-Dihydroxyphenyl) ethyl]-3-(4-hydroxy-3-methoxy-phenyl)-(2*Z*)-propensäureamid (cis-Feruloyldopamin): 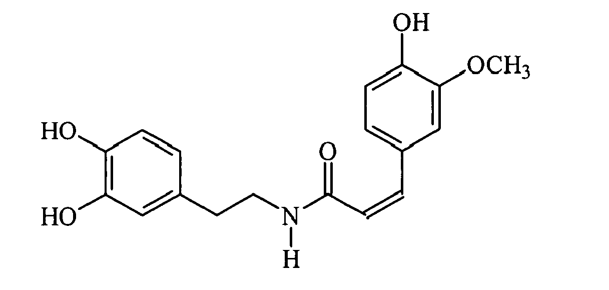 *N*-[2-(4-Hydroxyphenyl)ethyl]-3-(3,4- dihydroxyphenyl)-(2*E*)-propensäureamid (trans-Caffeoyltyramin): 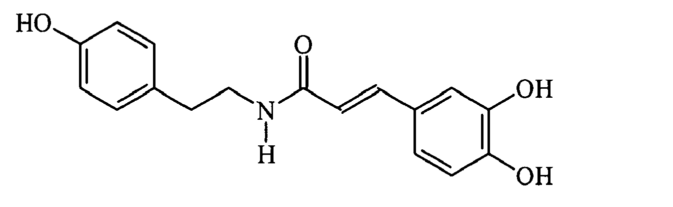 *N*-[2-(4-Hydroxyphenyl)ethyl]-3-(3,4-dihydroxyphenyl)-(2*Z*)- propensäureamid (cis-Caffeoyltyramin): 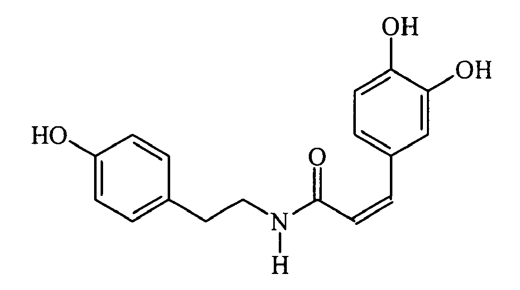 *N*-[2-(3,4-Dimethoxyphenyl) ethyl]-3-(3,4-dimethoxyphenyl)-(2*E*)-propensäureamid (trans-Rubenamin): 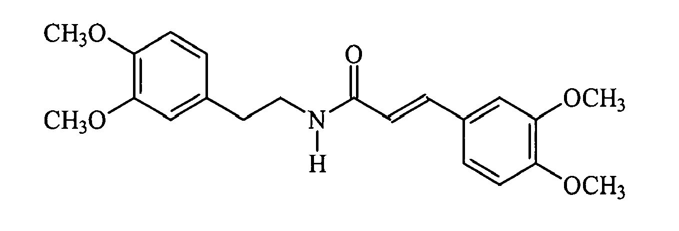 *N*-[2-(3,4-dimethoxyphenyl)ethyl]-3-(3,4- dimethoxy-phenyl)-(2*Z*)-propensäureamid (cis-Rubenamin):

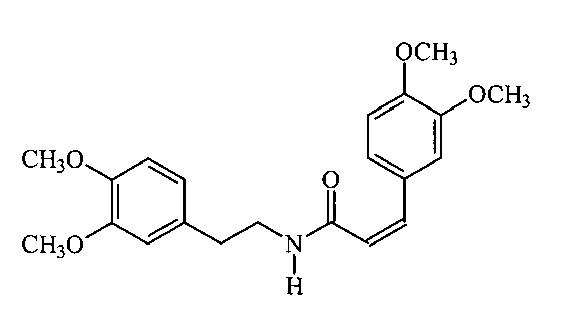

Weitere im Rahmen der vorliegenden Erfindung mit besonderem Vorzug einsetzbare Substanzen, die ein Gefühl von Wärme erzeugen sind beispielsweise Extrakte aus Naturpflanzen. Solche pflanzlichen Extrakte können alle physiologisch unbedenklichen pflanzlichen Extrakte sein, die einen warmen sensorischen Eindruck hervorrufen. Bevorzugt als ein Gefühl von Wärme erzeugende pflanzliche Extrakte sind beispielsweise Pfefferextrakt (*Piper ssp.,* insbesondere *Piper nigrum*), Wasserpfefferextrakt (*Polygonum ssp.* ,insbesondere *Polygonum hydropiper*), Extrakte aus *Allium* ssp.(insbesondere Zwiebel und Knoblauchextrakte), Extrakte aus Rettich (*Raphanus ssp.*), Meerrettichextrakte (*Cochlearia armoracia*), Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Anacyclus ssp.,* insbesondere *Anacyclus pyrethrum*L.), Sonnenhutextrakte ( *Echinaceae ssp.),* Extrakten aus Szechuan-Pfeffer *(Zanthoxylum ssp.,* insbesondere *Zanthoxylum piperitum*), Spilanthesextrakt (*Spilanthes ssp.,* insbesondere *Spilanthes acmella*), Chiliextrakt *(Capsicum ssp.,* insbesondere *Capsicum frutescens* ), Paradieskörner-Extrakt ( *Aframomum* ssp.,insbesondere *Aframomum melegueta*[Rose] K. Schum.),Ingwerextrakt *(Zingiber* ssp.,insbesondere *Zingiber officinale*) und Galangaextrakt *(Kaempferia galanga* oder *Alpinia galanga*).

Einsetzbar ist auch N-Ethyl-p-menthan-3-carboxamid (N-Ethyl-5-Methyl-2-isopropylcyclohexancarboxamid):

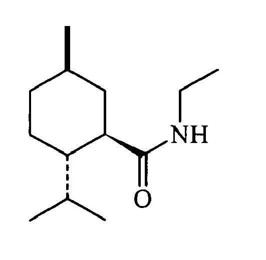

Andere ein Gefühl von Wärme erzeugende Substanzen können z.B. sein Capsaicin, Dihydrocapsaicin, Gingerol, Paradol, Shogaol, Piperin, Carbonsäure-N- vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3-methoxybenzylalkohol, insbesonders 4-Hydroxy-3-methoxybenzyl-n-butylether, Alkylether von 3-Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Zusammenfassend sind bevorzugte erfindungsgemäße Mittel zum Färben und/oder Aufhellen keratinischer Fasern dadurch gekennzeichnet, daß sie als ein Gefühl von Wärme erzeugende Substanz mindestens einen Scharfstoff aus der Gruppe der N-Alkyl-substituierte Amide von ungesättigten Carbonsäuren, vorzugsweise
- 2E,6Z,8E-Decatriensäure-N-isobutylamid (Spilanthol) und/oder
- 2E,4E,8Z-Decatriensäure-N-isobutylamid und/oder
- 2E,7Z,9E-Undecatriensäure-N-isobutylamid und/oder
- 2E,4Z-Decadiensäure-N-isobutylamid (cis-Pellitorin) und/oder
- 2E,4E-Decadiensäure-N-isobutylamid (trans-Pellitorin) und/oder
- Ferulasäure-N-Vanillylamid und/oder
- N-[2-(4-Hydroxy-3-methoxyphenyl)ethyl]-3-(4-hydroxy-3-methoxyphenyl)- (2*E*)-propensäureamid (trans-Feruloylmethoxytyramin) und/oder
- N-[2- (4-Hydroxy-3-methoxyphenyl)ethyl]-3-(4-hydroxy-3-methoxyphenyl)-(2 Z)-propensäureamid (cis-Feruloylmethoxytyramin) und/oder
- *N*-[2-(4- Hydroxy-3-methoxyphenyl)ethyl]-3-(4-hydroxy-3-methoxyphenyl)- propansäureamid (Dihydroferuloylmethoxytyramin) und/oder
- N-[2-(3,4- Dihydroxyphenyl)ethyl]-3-(4-hydroxy-3-methoxy-phenyl)-(2*E*)- propensäureamid (trans-Feruloyldopamin) und/oder
- N-[2-(3,4-Dihydroxyphenyl) ethyl]-3-(4-hydroxy-3-methoxy-phenyl)-(2*Z*)-propensäureamid (cis- Feruloyldopamin) und/oder
- N-[2-(4-Hydroxyphenyl)ethyl]-3-(3,4- dihydroxyphenyl)-(2*E*)-propensäureamid (trans-Caffeoyltyramin) und/oder
- *N*-[2-(4-Hydroxyphenyl)ethyl]-3-(3,4-dihydroxyphenyl)-(2*Z*)-propensäureamid (cis-Caffeoyltyramin) und/oder
- N-[2-(3,4-Dimethoxyphenyl) ethyl]-3-(3,4-dimethoxyphenyl)-(2*E*)-propensäureamid (trans- Rubenamin) und/oder
- N-[2-(3,4-dimethoxyphenyl)ethyl]-3-(3,4- dimethoxy-phenyl)-(2*Z*)-propensäureamid (cis-Rubenamin)
enthalten.

Zusätzlich zu den genannten Scharfstoffen oder an ihrer Stelle können auch weitere ein Gefühl von Wärme erzeugende Substanzen in die erfindungsgemäßen Mittel eingearbeitet werden.

Als besonders geeignet haben sich im Rahmen der vorliegenden Erfindung alkylsubstituierte Dioxane der Formel 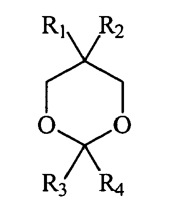 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃, - CH(CH₃)₂. Einzelne Spezies, die der vorstehend genannten Formel genügen, sind hinsichtlich ihrer Reste R1, R2, R3 und R4 in der nachstehenden Tabelle 1 aufgeführt. Diese sind erfindungsgemäß besonders bevorzugt.

**Tabelle 1: Bevorzugte alkylsubstituierte Dioxane:**

| **Nr.** | **R1** | **R2** | **R3** | **R4** |
|---|---|---|---|---|
| 1 | -H | -CH₃ | -H | -H |
| 2 | -H | -CH₂CH₃ | -H | -H |
| 3 | -CH₃ | -CH₃ | -H | -H |
| 4 | -CH₃ | -CH₂CH₃ | -H | -H |
| 5 | -CH₂CH₃ | -CH₂CH₃ | -H | -H |
| 6 | -H | -CH₃ | -H | -CH₃ |
| 7 | -H | -CH₃ | -H | -CH₂CH₃ |
| 8 | -H | -CH₃ | -H | -CH₂CH₂CH₃ |
| 9 | -H | -CH₃ | -H | -CH(CH₃)₂ |
| 10 | -H | -CH₃ | -CH₃ | -CH₃ |
| 11 | -H | -CH₃ | -CH₃ | -CH₂CH₃ |
| 12 | -H | -CH₃ | -CH₃ | -CH₂CH₂CH₃ |
| 13 | -H | -CH₃ | -CH₃ | -CH(CH₃)₂ |
| 14 | -H | -CH₃ | -CH₂CH₃ | -CH₂CH₃ |
| 15 | -H | -CH₃ | -CH₂CH₃ | -CH₂CH₂CH₃ |
| 16 | -H | -CH₃ | -CH₂CH₃ | -CH(CH₃)₂ |
| 17 | -H | -CH₃ | -CH₂CH₂CH₃ | -CH₂CH₂CH₃ |
| 18 | -H | -CH₃ | -CH₂CH₂CH₃ | -CH(CH₃)₂ |
| 19 | -H | -CH₃ | -CH(CH₃)₂ | -CH(CH₃)₂ |
| 20 | -H | -CH₂CH₃ | -H | -CH₃ |
| 21 | -H | -CH₂CH₃ | -H | -CH₂CH₃ |
| 22 | -H | -CH₂CH₃ | -H | -CH₂CH₂CH₃ |
| 23 | -H | -CH₂CH₃ | -H | -CH(CH₃)₂ |
| 24 | -H | -CH₂CH₃ | -CH₃ | -CH₃ |
| 25 | -H | -CH₂CH₃ | -CH₃ | -CH₂CH₃ |
| 26 | -H | -CH₂CH₃ | -CH₃ | -CH₂CH₂CH₃ |
| 27 | -H | -CH₂CH₃ | -CH₃ | -CH(CH₃)₂ |
| 28 | -H | -CH₂CH₃ | -CH₂CH₃ | -CH₂CH₃ |
| 29 | -H | -CH₂CH₃ | -CH₂CH₃ | -CH₂CH₂CH₃ |
| 30 | -H | -CH₂CH₃ | -CH₂CH₃ | -CH(CH₃)₂ |
| 31 | -H | -CH₂CH₃ | -CH₂CH₂CH₃ | -CH₂CH₂CH₃ |
| 32 | -H | -CH₂CH₃ | -CH₂CH₂CH₃ | -CH(CH₃)₂ |
| 33 | -H | -CH₂CH₃ | -CH(CH₃)₂ | -CH(CH₃)₂ |
| 34 | -CH₃ | -CH₃ | -H | -CH₃ |
| 35 | -CH₃ | -CH₃ | -H | -CH₂CH₃ |
| 36 | -CH₃ | -CH₃ | -H | -CH₂CH₂CH₃ |
| 37 | -CH₃ | -CH₃ | -H | -CH(CH₃)₂ |
| 38 | -CH₃ | -CH₃ | -CH₃ | -CH₃ |
| 39 | -CH₃ | -CH₃ | -CH₃ | -CH₂CH₃ |
| 40 | -CH₃ | -CH₃ | -CH₃ | -CH₂CH₂CH₃ |
| 41 | -CH₃ | -CH₃ | -CH₃ | -CH(CH₃)₂ |
| 42 | -CH₃ | -CH₃ | -CH₂CH₃ | -CH₂CH₃ |
| 43 | -CH₃ | -CH₃ | -CH₂CH₃ | -CH₂CH₂CH₃ |
| 44 | -CH₃ | -CH₃ | -CH₂CH₃ | -CH(CH₃)₂ |
| 45 | -CH₃ | -CH₃ | -CH₂CH₂CH₃ | -CH₂CH₂CH₃ |
| 46 | -CH₃ | -CH₃ | -CH₂CH₂CH₃ | -CH(CH₃)₂ |
| 47 | -CH₃ | -CH₃ | -CH(CH₃)₂ | -CH(CH₃)₂ |
| 48 | -CH₃ | -CH₂CH₃ | -H | -CH₃ |
| 49 | -CH₃ | -CH₂CH₃ | -H | -CH₂CH₃ |
| 50 | -CH₃ | -CH₂CH₃ | -H | -CH₂CH₂CH₃ |
| 51 | -CH₃ | -CH₂CH₃ | -H | -CH(CH₃)₂ |
| 52 | -CH₃ | -CH₂CH₃ | -CH₃ | -CH₃ |
| 53 | -CH₃ | -CH₂CH₃ | -CH₃ | -CH₂CH₃ |
| 54 | -CH₃ | -CH₂CH₃ | -CH₃ | -CH₂CH₂CH₃ |
| 55 | -CH₃ | -CH₂CH₃ | -CH₃ | -CH(CH₃)₂ |
| 56 | -CH₃ | -CH₂CH₃ | -CH₂CH₃ | -CH₂CH₃ |
| 57 | -CH₃ | -CH₂CH₃ | -CH₂CH₃ | -CH₂CH₂CH₃ |
| 58 | -CH₃ | -CH₂CH₃ | -CH₂CH₃ | -CH(CH₃)₂ |
| 59 | -CH₃ | -CH₂CH₃ | -CH₂CH₂CH₃ | -CH₂CH₂CH₃ |
| 60 | -CH₃ | -CH₂CH₃ | -CH₂CH₂CH₃ | -CH(CH₃)₂ |
| 61 | -CH₃ | -CH₂CH₃ | -CH(CH₃)₂ | -CH(CH₃)₂ |
| 62 | -CH₂CH₃ | -CH₂CH₃ | -H | -CH₃ |
| 63 | -CH₂CH₃ | -CH₂CH₃ | -H | -CH₂CH₃ |
| 64 | -CH₂CH₃ | -CH₂CH₃ | -H | -CH₂CH₂CH₃ |
| 65 | -CH₂CH₃ | -CH₂CH₃ | -H | -CH(CH₃)₂ |
| 66 | -CH₂CH₃ | -CH₂CH₃ | -CH₃ | -CH₃ |
| 67 | -CH₂CH₃ | -CH₂CH₃ | -CH₃ | -CH₂CH₃ |
| 68 | -CH₂CH₃ | -CH₂CH₃ | -CH₃ | -CH₂CH₂CH₃ |
| 69 | -CH₂CH₃ | -CH₂CH₃ | -CH₃ | -CH(CH₃)₂ |
| 70 | -CH₂CH₃ | -CH₂CH₃ | -CH₂CH₃ | -CH₂CH₃ |
| 71 | -CH₂CH₃ | -CH₂CH₃ | -CH₂CH₃ | -CH₂CH₂CH₃ |
| 72 | -CH₂CH₃ | -CH₂CH₃ | -CH₂CH₃ | -CH(CH₃)₂ |
| 73 | -CH₂CH₃ | -CH₂CH₃ | -CH₂CH₂CH₃ | -CH₂CH₂CH₃ |
| 74 | -CH₂CH₃ | -CH₂CH₃ | -CH₂CH₂CH₃ | -CH(CH₃)₂ |
| 75 | -CH₂CH₃ | -CH₂CH₃ | -CH(CH₃)₂ | -CH(CH₃)₂ |

Als weiterhin besonders geeignet haben sich im Rahmen der vorliegenden Erfindung Phenylester der Formel 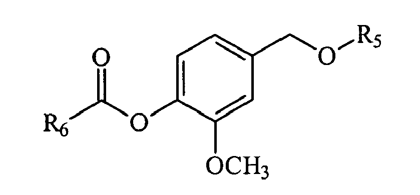 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Einzelne Spezies, die der vorstehend genannten Formel genügen, sind hinsichtlich ihrer Reste R5 und R6 in der nachstehenden Tabelle 2 aufgeführt. Diese sind erfindungsgemäß besonders bevorzugt.

**Tabelle 2: Bevorzugte Phenylester**

| **Nr.** | **R5** | **R6** |
|---|---|---|
| 1 | -CH₃ | -CH₃ |
| 2 | -CH₂CH₃ | -CH₃ |
| 3 | -CH₂CH₂CH₃ | -CH₃ |
| 4 | -CH(CH₃)₂ | -CH₃ |
| 5 | -CH₂CH₂CH₂CH₃ | -CH₃ |
| 6 | -CH(CH₃)CH₂CH₃ | -CH₃ |
| 7 | -CH₂CH(CH₃)₂ | -CH₃ |
| 8 | -C(CH₃)₃ | -CH₃ |
| 9 | -CH₂(CH₂)₃CH₃ | -CH₃ |
| 10 | -CH(CH₃)(CH₂)₂CH₃ | -CH₃ |
| 11 | -CH₂CH(CH₃)CH₂CH₃ | -CH₃ |
| 12 | -(CH₂)₂CH(CH₃)₂ | -CH₃ |
| 13 | -CH₂(CH₂)₄CH₃ | -CH₃ |
| 14 | -CH₂(CH₂)₅CH₃ | -CH₃ |
| 15 | -CH₂(CH₂)₆CH₃ | -CH₃ |
| 16 | -CH=CH₂ | -CH₃ |
| 17 | -CH=CHCH₃ | -CH₃ |
| 18 | -CH=C(CH₃)₂ | -CH₃ |
| 19 | -C(CH₃)=CH₂ | -CH₃ |
| 20 | -C(CH₃)=CHCH₃ | -CH₃ |
| 21 | -C(CH₃)=C(CH₃)₂ | -CH₃ |
| 22 | -CH=CH-CH₂CH₃ | -CH₃ |
| 23 | -CH₂CH=CH-CH₃ | -CH₃ |
| 24 | -(CH₂)₂CH=CH₂ | -CH₃ |
| 25 | -CH=CH-(CH₂)₂CH₃ | -CH₃ |
| 26 | -CH₂CH=CH-CH₂CH₃ | -CH₃ |
| 27 | -(CH₂)₂CH=CH-CH₃ | -CH₃ |
| 28 | -(CH₂)₃CH=CH₂ | -CH₃ |
| 29 | -CH=CH-(CH₂)₃CH₃ | -CH₃ |
| 30 | -CH₂CH=CH-(CH₂)₂CH₃ | -CH₃ |
| 31 | -(CH₂)₂CH=CH-CH₂CH₃ | -CH₃ |
| 32 | -(CH₂)₃CH=CH-CH₃ | -CH₃ |
| 33 | -(CH₂)₄CH=CH₂ | -CH₃ |
| 34 | -CH=CH-(CH₂)₃CH₃ | -CH₃ |
| 35 | -CH₂CH=CH-(CH₂)₃CH₃ | -CH₃ |
| 36 | -(CH₂)₂CH=CH-(CH₂)₂CH₃ | -CH₃ |
| 37 | -(CH₂)₃CH=CH-CH₂CH₃ | -CH₃ |
| 38 | -(CH₂)₄CH=CH-CH₃ | -CH₃ |
| 39 | -(CH₂)₅CH=CH₂ | -CH₃ |
| 40 | -CH=CH-(CH₂)₅CH₃ | -CH₃ |
| 41 | -CH₂CH=CH-(CH₂)₄CH₃ | -CH₃ |
| 42 | -(CH₂)₂CH=CH-(CH₂)₃CH₃ | -CH₃ |
| 43 | -(CH₂)₃CH=CH-(CH₂)₂CH₃ | -CH₃ |
| 44 | -(CH₂)₄CH=CH-CH₂CH₃ | -CH₃ |
| 45 | -(CH₂)₅CH=CH-CH₃ | -CH₃ |
| 46 | -(CH₂)₆CH=CH₂ | -CH₃ |
| 47 | -CH₃ | -CH₂CH₃ |
| 48 | -CH₂CH₃ | -CH₂CH₃ |
| 49 | -CH₂CH₂CH₃ | -CH₂CH₃ |
| 50 | -CH(CH₃)₂ | -CH₂CH₃ |
| 51 | -CH₂CH₂CH₂CH₃ | -CH₂CH₃ |
| 52 | -CH(CH₃)CH₂CH₃ | -CH₂CH₃ |
| 53 | -CH₂CH(CH₃)₂ | -CH₂CH₃ |
| 54 | -C(CH₃)₃ | -CH₂CH₃ |
| 55 | -CH₂(CH₂)₃CH₃ | -CH₂CH₃ |
| 56 | -CH(CH₃)(CH₂)₂CH₃ | -CH₂CH₃ |
| 57 | -CH₂CH(CH₃)CH₂CH₃ | -CH₂CH₃ |
| 58 | -(CH₂)₂CH(CH₃)₂ | -CH₂CH₃ |
| 59 | -CH₂(CH₂)₄CH₃ | -CH₂CH₃ |
| 60 | -CH₂(CH₂)₅CH₃ | -CH₂CH₃ |
| 61 | -CH₂(CH₂)₆CH₃ | -CH₂CH₃ |
| 62 | -CH=CH₂ | -CH₂CH₃ |
| 63 | -CH=CHCH₃ | -CH₂CH₃ |
| 64 | -CH=C(CH₃)₂ | -CH₂CH₃ |
| 65 | -C(CH₃)=CH₂ | -CH₂CH₃ |
| 66 | -C(CH₃)=CHCH₃ | -CH₂CH₃ |
| 67 | -C(CH₃)=C(CH₃)₂ | -CH₂CH₃ |
| 68 | -CH=CH-CH₂CH₃ | -CH₂CH₃ |
| 69 | -CH₂CH=CH-CH₃ | -CH₂CH₃ |
| 70 | -(CH₂)₂CH=CH₂ | -CH₂CH₃ |
| 71 | -CH=CH-(CH₂)₂CH₃ | -CH₂CH₃ |
| 72 | -CH₂CH=CH-CH₂CH₃ | -CH₂CH₃ |
| 73 | -(CH₂)₂CH=CH-CH₃ | -CH₂CH₃ |
| 74 | -(CH₂)₃CH=CH₂ | -CH₂CH₃ |
| 75 | -CH=CH-(CH₂)₃CH₃ | -CH₂CH₃ |
| 76 | -CH₂CH=CH-(CH₂)₂CH₃ | -CH₂CH₃ |
| 77 | -(CH₂)₂CH=CH-CH₂CH₃ | -CH₂CH₃ |
| 78 | -(CH₂)₃CH=CH-CH₃ | -CH₂CH₃ |
| 79 | -(CH₂)₄CH=CH₂ | -CH₂CH₃ |
| 80 | -CH=CH-(CH₂)₃CH₃ | -CH₂CH₃ |
| 81 | -CH₂CH=CH-(CH₂)₃CH₃ | -CH₂CH₃ |
| 82 | -(CH₂)₂CH=CH-(CH₂)₂CH₃ | -CH₂CH₃ |
| 83 | -(CH₂)₃CH=CH-CH₂CH₃ | -CH₂CH₃ |
| 84 | -(CH₂)₄CH=CH-CH₃ | -CH₂CH₃ |
| 85 | -(CH₂)₅CH=CH₂ | -CH₂CH₃ |
| 86 | -CH=CH-(CH₂)₅CH₃ | -CH₂CH₃ |
| 87 | -CH₂CH=CH-(CH₂)₄CH₃ | -CH₂CH₃ |
| 88 | -(CH₂)₂CH=CH-(CH₂)₃CH₃ | -CH₂CH₃ |
| 89 | -(CH₂)₃CH=CH-(CH₂)₂CH₃ | -CH₂CH₃ |
| 90 | -(CH₂)₄CH=CH-CH₂CH₃ | -CH₂CH₃ |
| 91 | -(CH₂)₅CH=CH-CH₃ | -CH₂CH₃ |
| 92 | -(CH₂)₆CH=CH₂ | -CH₂CH₃ |
| 93 | -CH₃ | -CH₂CH₂CH₃ |
| 94 | -CH₂CH₃ | -CH₂CH₂CH₃ |
| 95 | -CH₂CH₂CH₃ | -CH₂CH₂CH₃ |
| 96 | -CH(CH₃)₂ | -CH₂CH₂CH₃ |
| 97 | -CH₂CH₂CH₂CH₃ | -CH₂CH₂CH₃ |
| 98 | -CH(CH₃)CH₂CH₃ | -CH₂CH₂CH₃ |
| 99 | -CH₂CH(CH₃)₂ | -CH₂CH₂CH₃ |
| 100 | -C(CH₃)₃ | -CH₂CH₂CH₃ |
| 101 | -CH₂(CH₂)₃CH₃ | -CH₂CH₂CH₃ |
| 102 | -CH(CH₃)(CH₂)₂CH₃ | -CH₂CH₂CH₃ |
| 103 | -CH₂CH(CH₃)CH₂CH₃ | -CH₂CH₂CH₃ |
| 104 | -(CH₂)₂CH(CH₃)₂ | -CH₂CH₂CH₃ |
| 105 | -CH₂(CH₂)₄CH₃ | -CH₂CH₂CH₃ |
| 106 | -CH₂(CH₂)₅CH₃ | -CH₂CH₂CH₃ |
| 107 | -CH₂(CH₂)₆CH₃ | -CH₂CH₂CH₃ |
| 108 | -CH=CH₂ | -CH₂CH₂CH₃ |
| 109 | -CH=CHCH₃ | -CH₂CH₂CH₃ |
| 110 | -CH=C(CH₃)₂ | -CH₂CH₂CH₃ |
| 111 | -C(CH₃)=CH₂ | -CH₂CH₂CH₃ |
| 112 | -C(CH₃)=CHCH₃ | -CH₂CH₂CH₃ |
| 113 | -C(CH₃)=C(CH₃)₂ | -CH₂CH₂CH₃ |
| 114 | -CH=CH-CH₂CH₃ | -CH₂CH₂CH₃ |
| 115 | -CH₂CH=CH-CH₃ | -CH₂CH₂CH₃ |
| 116 | -(CH₂)₂CH=CH₂ | -CH₂CH₂CH₃ |
| 117 | -CH=CH-(CH₂)₂CH₃ | -CH₂CH₂CH₃ |
| 118 | -CH₂CH=CH-CH₂CH₃ | -CH₂CH₂CH₃ |
| 119 | -(CH₂)₂CH=CH-CH₃ | -CH₂CH₂CH₃ |
| 120 | -(CH₂)₃CH=CH₂ | -CH₂CH₂CH₃ |
| 121 | -CH=CH-(CH₂)₃CH₃ | -CH₂CH₂CH₃ |
| 122 | -CH₂CH=CH-(CH₂)₂CH₃ | -CH₂CH₂CH₃ |
| 123 | -(CH₂)₂CH=CH-CH₂CH₃ | -CH₂CH₂CH₃ |
| 124 | -(CH₂)₃CH=CH-CH₃ | -CH₂CH₂CH₃ |
| 125 | -(CH₂)₄CH=CH₂ | -CH₂CH₂CH₃ |
| 126 | -CH=CH-(CH₂)₃CH₃ | -CH₂CH₂CH₃ |
| 127 | -CH₂CH=CH-(CH₂)₃CH₃ | -CH₂CH₂CH₃ |
| 128 | -(CH₂)₂CH=CH-(CH₂)₂CH₃ | -CH₂CH₂CH₃ |
| 129 | -(CH₂)₃CH=CH-CH₂CH₃ | -CH₂CH₂CH₃ |
| 130 | -(CH₂)₄CH=CH-CH₃ | -CH₂CH₂CH₃ |
| 131 | -(CH₂)₅CH=CH₂ | -CH₂CH₂CH₃ |
| 132 | -CH=CH-(CH₂)₅CH₃ | -CH₂CH₂CH₃ |
| 133 | -CH₂CH=CH-(CH₂)₄CH₃ | -CH₂CH₂CH₃ |
| 134 | -(CH₂)₂CH=CH-(CH₂)₃CH₃ | -CH₂CH₂CH₃ |
| 135 | -(CH₂)₃CH=CH-(CH₂)₂CH₃ | -CH₂CH₂CH₃ |
| 136 | -(CH₂)₄CH=CH-CH₂CH₃ | -CH₂CH₂CH₃ |
| 137 | -(CH₂)₅CH=CH-CH₃ | -CH₂CH₂CH₃ |
| 138 | -(CH₂)₆CH=CH₂ | -CH₂CH₂CH₃ |
| 139 | -CH₃ | -CH(CH₃)₂ |
| 140 | -CH₂CH₃ | -CH(CH₃)₂ |
| 141 | -CH₂CH₂CH₃ | -CH(CH₃)₂ |
| 142 | -CH(CH₃)₂ | -CH(CH₃)₂ |
| 143 | -CH₂CH₂CH₂CH₃ | -CH(CH₃)₂ |
| 144 | -CH(CH₃)CH₂CH₃ | -CH(CH₃)₂ |
| 145 | -CH₂CH(CH₃)₂ | -CH(CH₃)₂ |
| 146 | -C(CH₃)₃ | -CH(CH₃)₂ |
| 147 | -CH₂(CH₂)₃CH₃ | -CH(CH₃)₂ |
| 148 | -CH(CH₃)(CH₂)₂CH₃ | -CH(CH₃)₂ |
| 149 | -CH₂CH(CH₃)CH₂CH₃ | -CH(CH₃)₂ |
| 150 | -(CH₂)₂CH(CH₃)₂ | -CH(CH₃)₂ |
| 151 | -CH₂(CH₂)₄CH₃ | -CH(CH₃)₂ |
| 152 | -CH₂(CH₂)₅CH₃ | -CH(CH₃)₂ |
| 153 | -CH₂(CH₂)₆CH₃ | -CH(CH₃)₂ |
| 154 | -CH=CH₂ | -CH(CH₃)₂ |
| 155 | -CH=CHCH₃ | -CH(CH₃)₂ |
| 156 | -CH=C(CH₃)₂ | -CH(CH₃)₂ |
| 157 | -C(CH₃)=CH₂ | -CH(CH₃)₂ |
| 158 | -C(CH₃)=CHCH₃ | -CH(CH₃)₂ |
| 159 | -C(CH₃)=C(CH₃)₂ | -CH(CH₃)₂ |
| 160 | -CH=CH-CH₂CH₃ | -CH(CH₃)₂ |
| 161 | -CH₂CH=CH-CH₃ | -CH(CH₃)₂ |
| 162 | -(CH₂)₂CH=CH₂ | -CH(CH₃)₂ |
| 163 | -CH=CH-(CH₂)₂CH₃ | -CH(CH₃)₂ |
| 164 | -CH₂CH=CH-CH₂CH₃ | -CH(CH₃)₂ |
| 165 | -(CH₂)₂CH=CH-CH₃ | -CH(CH₃)₂ |
| 166 | -(CH₂)₃CH=CH₂ | -CH(CH₃)₂ |
| 167 | -CH=CH-(CH₂)₃CH₃ | -CH(CH₃)₂ |
| 168 | -CH₂CH=CH-(CH₂)₂CH₃ | -CH(CH₃)₂ |
| 169 | -(CH₂)₂CH=CH-CH₂CH₃ | -CH(CH₃)₂ |
| 170 | -(CH₂)₃CH=CH-CH₃ | -CH(CH₃)₂ |
| 171 | -(CH₂)₄CH=CH₂ | -CH(CH₃)₂ |
| 172 | -CH=CH-(CH₂)₃CH₃ | -CH(CH₃)₂ |
| 173 | -CH₂CH=CH-(CH₂)₃CH₃ | -CH(CH₃)₂ |
| 174 | -(CH₂)₂CH=CH-(CH₂)₂CH₃ | -CH(CH₃)₂ |
| 175 | -(CH₂)₃CH=CH-CH₂CH₃ | -CH(CH₃)₂ |
| 176 | -(CH₂)₄CH=CH-CH₃ | -CH(CH₃)₂ |
| 177 | -(CH₂)₅CH=CH₂ | -CH(CH₃)₂ |
| 178 | -CH=CH-(CH₂)₅CH₃ | -CH(CH₃)₂ |
| 179 | -CH₂CH=CH-(CH₂)₄CH₃ | -CH(CH₃)₂ |
| 180 | -(CH₂)₂CH=CH-(CH₂)₃CH₃ | -CH(CH₃)₂ |
| 181 | -(CH₂)₃CH=CH-(CH₂)₂CH₃ | -CH(CH₃)₂ |
| 182 | -(CH₂)₄CH=CH-CH₂CH₃ | -CH(CH₃)₂ |
| 183 | -(CH₂)₅CH=CH-CH₃ | -CH(CH₃)₂ |
| 184 | -(CH₂)₆CH=CH₂ | -CH(CH₃)₂ |
| 185 | -CH₃ | -CH₂CH₂CH₂CH₃ |
| 186 | -CH₂CH₃ | -CH₂CH₂CH₂CH₃ |
| 187 | -CH₂CH₂CH₃ | -CH₂CH₂CH₂CH₃ |
| 188 | -CH(CH₃)₂ | -CH₂CH₂CH₂CH₃ |
| 189 | -CH₂CH₂CH₂CH₃ | -CH₂CH₂CH₂CH₃ |
| 190 | -CH(CH₃)CH₂CH₃ | -CH₂CH₂CH₂CH₃ |
| 191 | -CH₂CH(CH₃)₂ | -CH₂CH₂CH₂CH₃ |
| 192 | -C(CH₃)₃ | -CH₂CH₂CH₂CH₃ |
| 193 | -CH₂(CH₂)₃CH₃ | -CH₂CH₂CH₂CH₃ |
| 194 | -CH(CH₃)(CH₂)₂CH₃ | -CH₂CH₂CH₂CH₃ |
| 195 | -CH₂CH(CH₃)CH₂CH₃ | -CH₂CH₂CH₂CH₃ |
| 196 | -(CH₂)₂CH(CH₃)₂ | -CH₂CH₂CH₂CH₃ |
| 197 | -CH₂(CH₂)₄CH₃ | -CH₂CH₂CH₂CH₃ |
| 198 | -CH₂(CH₂)₅CH₃ | -CH₂CH₂CH₂CH₃ |
| 199 | -CH₂(CH₂)₆CH₃ | -CH₂CH₂CH₂CH₃ |
| 200 | -CH=CH₂ | -CH₂CH₂CH₂CH₃ |
| 201 | -CH=CHCH₃ | -CH₂CH₂CH₂CH₃ |
| 202 | -CH=C(CH₃)₂ | -CH₂CH₂CH₂CH₃ |
| 203 | -C(CH₃)=CH₂ | -CH₂CH₂CH₂CH₃ |
| 204 | -C(CH₃)=CHCH₃ | -CH₂CH₂CH₂CH₃ |
| 205 | -C(CH₃)=C(CH₃)₂ | -CH₂CH₂CH₂CH₃ |
| 206 | -CH=CH-CH₂CH₃ | -CH₂CH₂CH₂CH₃ |
| 207 | -CH₂CH=CH-CH₃ | -CH₂CH₂CH₂CH₃ |
| 208 | -(CH₂)₂CH=CH₂ | -CH₂CH₂CH₂CH₃ |
| 209 | -CH=CH-(CH₂)₂CH₃ | -CH₂CH₂CH₂CH₃ |
| 210 | -CH₂CH=CH-CH₂CH₃ | -CH₂CH₂CH₂CH₃ |
| 211 | -(CH₂)₂CH=CH-CH₃ | -CH₂CH₂CH₂CH₃ |
| 212 | -(CH₂)₃CH=CH₂ | -CH₂CH₂CH₂CH₃ |
| 213 | -CH=CH-(CH₂)₃CH₃ | -CH₂CH₂CH₂CH₃ |
| 214 | -CH₂CH=CH-(CH₂)₂CH₃ | -CH₂CH₂CH₂CH₃ |
| 215 | -(CH₂)₂CH=CH-CH₂CH₃ | -CH₂CH₂CH₂CH₃ |
| 216 | -(CH₂)₃CH=CH-CH₃ | -CH₂CH₂CH₂CH₃ |
| 217 | -(CH₂)₄CH=CH₂ | -CH₂CH₂CH₂CH₃ |
| 218 | -CH=CH-(CH₂)₃CH₃ | -CH₂CH₂CH₂CH₃ |
| 219 | -CH₂CH=CH-(CH₂)₃CH₃ | -CH₂CH₂CH₂CH₃ |
| 220 | -(CH₂)₂CH=CH-(CH₂)₂CH₃ | -CH₂CH₂CH₂CH₃ |
| 221 | -(CH₂)₃CH=CH-CH₂CH₃ | -CH₂CH₂CH₂CH₃ |
| 222 | -(CH₂)₄CH=CH-CH₃ | -CH₂CH₂CH₂CH₃ |
| 223 | -(CH₂)₅CH=CH₂ | -CH₂CH₂CH₂CH₃ |
| 224 | -CH=CH-(CH₂)₅CH₃ | -CH₂CH₂CH₂CH₃ |
| 225 | -CH₂CH=CH-(CH₂)₄CH₃ | -CH₂CH₂CH₂CH₃ |
| 226 | -(CH₂)₂CH=CH-(CH₂)₃CH₃ | -CH₂CH₂CH₂CH₃ |
| 227 | -(CH₂)₃CH=CH-(CH₂)₂CH₃ | -CH₂CH₂CH₂CH₃ |
| 228 | -(CH₂)₄CH=CH-CH₂CH₃ | -CH₂CH₂CH₂CH₃ |
| 229 | -(CH₂)₅CH=CH-CH₃ | -CH₂CH₂CH₂CH₃ |
| 230 | -(CH₂)₆CH=CH₂ | -CH₂CH₂CH₂CH₃ |
| 231 | -CH₃ | -CH(CH₃)CH₂CH₃ |
| 232 | -CH₂CH₃ | -CH(CH₃)CH₂CH₃ |
| 233 | -CH₂CH₂CH₃ | -CH(CH₃)CH₂CH₃ |
| 234 | -CH(CH₃)₂ | -CH(CH₃)CH₂CH₃ |
| 235 | -CH₂CH₂CH₂CH₃ | -CH(CH₃)CH₂CH₃ |
| 236 | -CH(CH₃)CH₂CH₃ | -CH(CH₃)CH₂CH₃ |
| 237 | -CH₂CH(CH₃)₂ | -CH(CH₃)CH₂CH₃ |
| 238 | -C(CH₃)₃ | -CH(CH₃)CH₂CH₃ |
| 239 | -CH₂(CH₂)₃CH₃ | -CH(CH₃)CH₂CH₃ |
| 240 | -CH(CH₃)(CH₂)₂CH₃ | -CH(CH₃)CH₂CH₃ |
| 241 | -CH₂CH(CH₃)CH₂CH₃ | -CH(CH₃)CH₂CH₃ |
| 242 | -(CH₂)₂CH(CH₃)₂ | -CH(CH₃)CH₂CH₃ |
| 243 | -CH₂(CH₂)₄CH₃ | -CH(CH₃)CH₂CH₃ |
| 244 | -CH₂(CH₂)₅CH₃ | -CH(CH₃)CH₂CH₃ |
| 245 | -CH₂(CH₂)₆CH₃ | -CH(CH₃)CH₂CH₃ |
| 246 | -CH=CH₂ | -CH(CH₃)CH₂CH₃ |
| 247 | -CH=CHCH₃ | -CH(CH₃)CH₂CH₃ |
| 248 | -CH=C(CH₃)₂ | -CH(CH₃)CH₂CH₃ |
| 249 | -C(CH₃)=CH₂ | -CH(CH₃)CH₂CH₃ |
| 250 | -C(CH₃)=CHCH₃ | -CH(CH₃)CH₂CH₃ |
| 251 | -C(CH₃)=C(CH₃)₂ | -CH(CH₃)CH₂CH₃ |
| 252 | -CH=CH-CH₂CH₃ | -CH(CH₃)CH₂CH₃ |
| 253 | -CH₂CH=CH-CH₃ | -CH(CH₃)CH₂CH₃ |
| 254 | -(CH₂)₂CH=CH₂ | -CH(CH₃)CH₂CH₃ |
| 255 | -CH=CH-(CH₂)₂CH₃ | -CH(CH₃)CH₂CH₃ |
| 256 | -CH₂CH=CH-CH₂CH₃ | -CH(CH₃)CH₂CH₃ |
| 257 | -(CH₂)₂CH=CH-CH₃ | -CH(CH₃)CH₂CH₃ |
| 258 | -(CH₂)₃CH=CH₂ | -CH(CH₃)CH₂CH₃ |
| 259 | -CH=CH-(CH₂)₃CH₃ | -CH(CH₃)CH₂CH₃ |
| 260 | -CH₂CH=CH-(CH₂)₂CH₃ | -CH(CH₃)CH₂CH₃ |
| 261 | -(CH₂)₂CH=CH-CH₂CH₃ | -CH(CH₃)CH₂CH₃ |
| 262 | -(CH₂)₃CH=CH-CH₃ | -CH(CH₃)CH₂CH₃ |
| 263 | -(CH₂)₄CH=CH₂ | -CH(CH₃)CH₂CH₃ |
| 264 | -CH=CH-(CH₂)₃CH₃ | -CH(CH₃)CH₂CH₃ |
| 265 | -CH₂CH=CH-(CH₂)₃CH₃ | -CH(CH₃)CH₂CH₃ |
| 266 | -(CH₂)₂CH=CH-(CH₂)₂CH₃ | -CH(CH₃)CH₂CH₃ |
| 267 | -(CH₂)₃CH=CH-CH₂CH₃ | -CH(CH₃)CH₂CH₃ |
| 268 | -(CH₂)₄CH=CH-CH₃ | -CH(CH₃)CH₂CH₃ |
| 269 | -(CH₂)₅CH=CH₂ | -CH(CH₃)CH₂CH₃ |
| 270 | -CH=CH-(CH₂)₅CH₃ | -CH(CH₃)CH₂CH₃ |
| 271 | -CH₂CH=CH-(CH₂)₄CH₃ | -CH(CH₃)CH₂CH₃ |
| 272 | -(CH₂)₂CH=CH-(CH₂)₃CH₃ | -CH(CH₃)CH₂CH₃ |
| 273 | -(CH₂)₃CH=CH-(CH₂)₂CH₃ | -CH(CH₃)CH₂CH₃ |
| 274 | -(CH₂)₄CH=CH-CH₂CH₃ | -CH(CH₃)CH₂CH₃ |
| 275 | -(CH₂)₅CH=CH-CH₃ | -CH(CH₃)CH₂CH₃ |
| 276 | -(CH₂)₆CH=CH₂ | -CH(CH₃)CH₂CH₃ |
| 277 | -CH₃ | -CH₂CH(CH₃)₂ |
| 278 | -CH₂CH₃ | -CH₂CH(CH₃)₂ |
| 279 | -CH₂CH₂CH₃ | -CH₂CH(CH₃)₂ |
| 280 | -CH(CH₃)₂ | -CH₂CH(CH₃)₂ |
| 281 | -CH₂CH₂CH₂CH₃ | -CH₂CH(CH₃)₂ |
| 282 | -CH(CH₃)CH₂CH₃ | -CH₂CH(CH₃)₂ |
| 283 | -CH₂CH(CH₃)₂ | -CH₂CH(CH₃)₂ |
| 284 | -C(CH₃)₃ | -CH₂CH(CH₃)₂ |
| 285 | -CH₂(CH₂)₃CH₃ | -CH₂CH(CH₃)₂ |
| 286 | -CH(CH₃)(CH₂)₂CH₃ | -CH₂CH(CH₃)₂ |
| 287 | -CH₂CH(CH₃)CH₂CH₃ | -CH₂CH(CH₃)₂ |
| 288 | -(CH₂)₂CH(CH₃)₂ | -CH₂CH(CH₃)₂ |
| 289 | -CH₂(CH₂)₄CH₃ | -CH₂CH(CH₃)₂ |
| 290 | -CH₂(CH₂)₅CH₃ | -CH₂CH(CH₃)₂ |
| 291 | -CH₂(CH₂)₆CH₃ | -CH₂CH(CH₃)₂ |
| 292 | -CH=CH₂ | -CH₂CH(CH₃)₂ |
| 293 | -CH=CHCH₃ | -CH₂CH(CH₃)₂ |
| 294 | -CH=C(CH₃)₂ | -CH₂CH(CH₃)₂ |
| 295 | -C(CH₃)=CH₂ | -CH₂CH(CH₃)₂ |
| 296 | -C(CH₃)=CHCH₃ | -CH₂CH(CH₃)₂ |
| 297 | -C(CH₃)=C(CH₃)₂ | -CH₂CH(CH₃)₂ |
| 298 | -CH=CH-CH₂CH₃ | -CH₂CH(CH₃)₂ |
| 299 | -CH₂CH=CH-CH₃ | -CH₂CH(CH₃)₂ |
| 300 | -(CH₂)₂CH=CH₂ | -CH₂CH(CH₃)₂ |
| 301 | -CH=CH-(CH₂)₂CH₃ | -CH₂CH(CH₃)₂ |
| 302 | -CH₂CH=CH-CH₂CH₃ | -CH₂CH(CH₃)₂ |
| 303 | -(CH₂)₂CH=CH-CH₃ | -CH₂CH(CH₃)₂ |
| 304 | -(CH₂)₃CH=CH₂ | -CH₂CH(CH₃)₂ |
| 305 | -CH=CH-(CH₂)₃CH₃ | -CH₂CH(CH₃)₂ |
| 306 | -CH₂CH=CH-(CH₂)₂CH₃ | -CH₂CH(CH₃)₂ |
| 307 | -(CH₂)₂CH=CH-CH₂CH₃ | -CH₂CH(CH₃)₂ |
| 308 | -(CH₂)₃CH=CH-CH₃ | -CH₂CH(CH₃)₂ |
| 309 | -(CH₂)₄CH=CH₂ | -CH₂CH(CH₃)₂ |
| 310 | -CH=CH-(CH₂)₃CH₃ | -CH₂CH(CH₃)₂ |
| 311 | -CH₂CH=CH-(CH₂)₃CH₃ | -CH₂CH(CH₃)₂ |
| 312 | -(CH₂)₂CH=CH-(CH₂)₂CH₃ | -CH₂CH(CH₃)₂ |
| 313 | -(CH₂)₃CH=CH-CH₂CH₃ | -CH₂CH(CH₃)₂ |
| 314 | -(CH₂)₄CH=CH-CH₃ | -CH₂CH(CH₃)₂ |
| 315 | -(CH₂)₅CH=CH₂ | -CH₂CH(CH₃)₂ |
| 316 | -CH=CH-(CH₂)₅CH₃ | -CH₂CH(CH₃)₂ |
| 317 | -CH₂CH=CH-(CH₂)₄CH₃ | -CH₂CH(CH₃)₂ |
| 318 | -(CH₂)₂CH=CH-(CH₂)₃CH₃ | -CH₂CH(CH₃)₂ |
| 319 | -(CH₂)₃CH=CH-(CH₂)₂CH₃ | -CH₂CH(CH₃)₂ |
| 320 | -(CH₂)₄CH=CH-CH₂CH₃ | -CH₂CH(CH₃)₂ |
| 321 | -(CH₂)₅CH=CH-CH₃ | -CH₂CH(CH₃)₂ |
| 322 | -(CH₂)₆CH=CH₂ | -CH₂CH(CH₃)₂ |
| 323 | -CH₃ | -C(CH₃)₃ |
| 324 | -CH₂CH₃ | -C(CH₃)₃ |
| 325 | -CH₂CH₂CH₃ | -C(CH₃)₃ |
| 326 | -CH(CH₃)₂ | -C(CH₃)₃ |
| 327 | -CH₂CH₂CH₂CH₃ | -C(CH₃)₃ |
| 328 | -CH(CH₃)CH₂CH₃ | -C(CH₃)₃ |
| 329 | -CH₂CH(CH₃)₂ | -C(CH₃)₃ |
| 330 | -C(CH₃)₃ | -C(CH₃)₃ |
| 331 | -CH₂(CH₂)₃CH₃ | -C(CH₃)₃ |
| 332 | -CH(CH₃)(CH₂)₂CH₃ | -C(CH₃)₃ |
| 333 | -CH₂CH(CH₃)CH₂CH₃ | -C(CH₃)₃ |
| 334 | -(CH₂)₂CH(CH₃)₂ | -C(CH₃)₃ |
| 335 | -CH₂(CH₂)₄CH₃ | -C(CH₃)₃ |
| 336 | -CH₂(CH₂)₅CH₃ | -C(CH₃)₃ |
| 337 | -CH₂(CH₂)₆CH₃ | -C(CH₃)₃ |
| 338 | -CH=CH₂ | -C(CH₃)₃ |
| 339 | -CH=CHCH₃ | -C(CH₃)₃ |
| 340 | -CH=C(CH₃)₂ | -C(CH₃)₃ |
| 341 | -C(CH₃)=CH₂ | -C(CH₃)₃ |
| 342 | -C(CH₃)=CHCH₃ | -C(CH₃)₃ |
| 343 | -C(CH₃)=C(CH₃)₂ | -C(CH₃)₃ |
| 344 | -CH=CH-CH₂CH₃ | -C(CH₃)₃ |
| 345 | -CH₂CH=CH-CH₃ | -C(CH₃)₃ |
| 346 | -(CH₂)₂CH=CH₂ | -C(CH₃)₃ |
| 347 | -CH=CH-(CH₂)₂CH₃ | -C(CH₃)₃ |
| 348 | -CH₂CH=CH-CH₂CH₃ | -C(CH₃)₃ |
| 349 | -(CH₂)₂CH=CH-CH₃ | -C(CH₃)₃ |
| 350 | -(CH₂)₃CH=CH₂ | -C(CH₃)₃ |
| 351 | -CH=CH-(CH₂)₃CH₃ | -C(CH₃)₃ |
| 352 | -CH₂CH=CH-(CH₂)₂CH₃ | -C(CH₃)₃ |
| 353 | -(CH₂)₂CH=CH-CH₂CH₃ | -C(CH₃)₃ |
| 354 | -(CH₂)₃CH=CH-CH₃ | -C(CH₃)₃ |
| 355 | -(CH₂)₄CH=CH₂ | -C(CH₃)₃ |
| 356 | -CH=CH-(CH₂)₃CH₃ | -C(CH₃)₃ |
| 357 | -CH₂CH=CH-(CH₂)₃CH₃ | -C(CH₃)₃ |
| 358 | -(CH₂)₂CH=CH-(CH₂)₂CH₃ | -C(CH₃)₃ |
| 359 | -(CH₂)₃CH=CH-CH₂CH₃ | -C(CH₃)₃ |
| 360 | -(CH₂)₄CH=CH-CH₃ | -C(CH₃)₃ |
| 361 | -(CH₂)₅CH=CH₂ | -C(CH₃)₃ |
| 362 | -CH=CH-(CH₂)₅CH₃ | -C(CH₃)₃ |
| 363 | -CH₂CH=CH-(CH₂)₄CH₃ | -C(CH₃)₃ |
| 364 | -(CH₂)₂CH=CH-(CH₂)₃CH₃ | -C(CH₃)₃ |
| 365 | -(CH₂)₃CH=CH-(CH₂)₂CH₃ | -C(CH₃)₃ |
| 366 | -(CH₂)₄CH=CH-CH₂CH₃ | -C(CH₃)₃ |
| 367 | -(CH₂)₅CH=CH-CH₃ | -C(CH₃)₃ |
| 368 | -(CH₂)₆CH=CH₂ | -C(CH₃)₃ |
| 369 | -CH₃ | -CH₂(CH₂)₃CH₃ |
| 370 | -CH₂CH₃ | -CH₂(CH₂)₃CH₃ |
| 371 | -CH₂CH₂CH₃ | -CH₂(CH₂)₃CH₃ |
| 372 | -CH(CH₃)₂ | -CH₂(CH₂)₃CH₃ |
| 373 | -CH₂CH₂CH₂CH₃ | -CH₂(CH₂)₃CH₃ |
| 374 | -CH(CH₃)CH₂CH₃ | -CH₂(CH₂)₃CH₃ |
| 375 | -CH₂CH(CH₃)₂ | -CH₂(CH₂)₃CH₃ |
| 376 | -C(CH₃)₃ | -CH₂(CH₂)₃CH₃ |
| 377 | -CH₂(CH₂)₃CH₃ | -CH₂(CH₂)₃CH₃ |
| 378 | -CH(CH₃)(CH₂)₂CH₃ | -CH₂(CH₂)₃CH₃ |
| 379 | -CH₂CH(CH₃)CH₂CH₃ | -CH₂(CH₂)₃CH₃ |
| 380 | -(CH₂)₂CH(CH₃)₂ | -CH₂(CH₂)₃CH₃ |
| 381 | -CH₂(CH₂)₄CH₃ | -CH₂(CH₂)₃CH₃ |
| 382 | -CH₂(CH₂)₅CH₃ | -CH₂(CH₂)₃CH₃ |
| 383 | -CH₂(CH₂)₆CH₃ | -CH₂(CH₂)₃CH₃ |
| 384 | -CH=CH₂ | -CH₂(CH₂)₃CH₃ |
| 385 | -CH=CHCH₃ | -CH₂(CH₂)₃CH₃ |
| 386 | -CH=C(CH₃)₂ | -CH₂(CH₂)₃CH₃ |
| 387 | -C(CH₃)=CH₂ | -CH₂(CH₂)₃CH₃ |
| 388 | -C(CH₃)=CHCH₃ | -CH₂(CH₂)₃CH₃ |
| 389 | -C(CH₃)=C(CH₃)₂ | -CH₂(CH₂)₃CH₃ |
| 390 | -CH=CH-CH₂CH₃ | -CH₂(CH₂)₃CH₃ |
| 391 | -CH₂CH=CH-CH₃ | -CH₂(CH₂)₃CH₃ |
| 392 | -(CH₂)₂CH=CH₂ | -CH₂(CH₂)₃CH₃ |
| 393 | -CH=CH-(CH₂)₂CH₃ | -CH₂(CH₂)₃CH₃ |
| 394 | -CH₂CH=CH-CH₂CH₃ | -CH₂(CH₂)₃CH₃ |
| 395 | -(CH₂)₂CH=CH-CH₃ | -CH₂(CH₂)₃CH₃ |
| 396 | -(CH₂)₃CH=CH₂ | -CH₂(CH₂)₃CH₃ |
| 397 | -CH=CH-(CH₂)₃CH₃ | -CH₂(CH₂)₃CH₃ |
| 398 | -CH₂CH=CH-(CH₂)₂CH₃ | -CH₂(CH₂)₃CH₃ |
| 399 | -(CH₂)₂CH=CH-CH₂CH₃ | -CH₂(CH₂)₃CH₃ |
| 400 | -(CH₂)₃CH=CH-CH₃ | -CH₂(CH₂)₃CH₃ |
| 401 | -(CH₂)₄CH=CH₂ | -CH₂(CH₂)₃CH₃ |
| 402 | -CH=CH-(CH₂)₃CH₃ | -CH₂(CH₂)₃CH₃ |
| 403 | -CH₂CH=CH-(CH₂)₃CH₃ | -CH₂(CH₂)₃CH₃ |
| 404 | -(CH₂)₂CH=CH-(CH₂)₂CH₃ | -CH₂(CH₂)₃CH₃ |
| 405 | -(CH₂)₃CH=CH-CH₂CH₃ | -CH₂(CH₂)₃CH₃ |
| 406 | -(CH₂)₄CH=CH-CH₃ | -CH₂(CH₂)₃CH₃ |
| 407 | -(CH₂)₅CH=CH₂ | -CH₂(CH₂)₃CH₃ |
| 408 | -CH=CH-(CH₂)₅CH₃ | -CH₂(CH₂)₃CH₃ |
| 409 | -CH₂CH=CH-(CH₂)₄CH₃ | -CH₂(CH₂)₃CH₃ |
| 410 | -(CH₂)₂CH=CH-(CH₂)₃CH₃ | -CH₂(CH₂)₃CH₃ |
| 411 | -(CH₂)₃CH=CH-(CH₂)₂CH₃ | -CH₂(CH₂)₃CH₃ |
| 412 | -(CH₂)₄CH=CH-CH₂CH₃ | -CH₂(CH₂)₃CH₃ |
| 413 | -(CH₂)₅CH=CH-CH₃ | -CH₂(CH₂)₃CH₃ |
| 414 | -(CH₂)₆CH=CH₂ | -CH₂(CH₂)₃CH₃ |
| 415 | -CH₃ | -CH(CH₃)(CH₂)₂CH₃ |
| 416 | -CH₂CH₃ | -CH(CH₃)(CH₂)₂CH₃ |
| 417 | -CH₂CH₂CH₃ | -CH(CH₃)(CH₂)₂CH₃ |
| 418 | -CH(CH₃)₂ | -CH(CH₃)(CH₂)₂CH₃ |
| 419 | -CH₂CH₂CH₂CH₃ | -CH(CH₃)(CH₂)₂CH₃ |
| 420 | -CH(CH₃)CH₂CH₃ | -CH(CH₃)(CH₂)₂CH₃ |
| 421 | -CH₂CH(CH₃)₂ | -CH(CH₃)(CH₂)₂CH₃ |
| 422 | -C(CH₃)₃ | -CH(CH₃)(CH₂)₂CH₃ |
| 423 | -CH₂(CH₂)₃CH₃ | -CH(CH₃)(CH₂)₂CH₃ |
| 424 | -CH(CH₃)(CH₂)₂CH₃ | -CH(CH₃)(CH₂)₂CH₃ |
| 425 | -CH₂CH(CH₃)CH₂CH₃ | -CH(CH₃)(CH₂)₂CH₃ |
| 426 | -(CH₂)₂CH(CH₃)₂ | -CH(CH₃)(CH₂)₂CH₃ |
| 427 | -CH₂(CH₂)₄CH₃ | -CH(CH₃)(CH₂)₂CH₃ |
| 428 | -CH₂(CH₂)₅CH₃ | -CH(CH₃)(CH₂)₂CH₃ |
| 429 | -CH₂(CH₂)₆CH₃ | -CH(CH₃)(CH₂)₂CH₃ |
| 430 | -CH=CH₂ | -CH(CH₃)(CH₂)₂CH₃ |
| 431 | -CH=CHCH₃ | -CH(CH₃)(CH₂)₂CH₃ |
| 432 | -CH=C(CH₃)₂ | -CH(CH₃)(CH₂)₂CH₃ |
| 433 | -C(CH₃)=CH₂ | -CH(CH₃)(CH₂)₂CH₃ |
| 434 | -C(CH₃)=CHCH₃ | -CH(CH₃)(CH₂)₂CH₃ |
| 435 | -C(CH₃)=C(CH₃)₂ | -CH(CH₃)(CH₂)₂CH₃ |
| 436 | -CH=CH-CH₂CH₃ | -CH(CH₃)(CH₂)₂CH₃ |
| 437 | -CH₂CH=CH-CH₃ | -CH(CH₃)(CH₂)₂CH₃ |
| 438 | -(CH₂)₂CH=CH₂ | -CH(CH₃)(CH₂)₂CH₃ |
| 439 | -CH=CH-(CH₂)₂CH₃ | -CH(CH₃)(CH₂)₂CH₃ |
| 440 | -CH₂CH=CH-CH₂CH₃ | -CH(CH₃)(CH₂)₂CH₃ |
| 441 | -(CH₂)₂CH=CH-CH₃ | -CH(CH₃)(CH₂)₂CH₃ |
| 442 | -(CH₂)₃CH=CH₂ | -CH(CH₃)(CH₂)₂CH₃ |
| 443 | -CH=CH-(CH₂)₃CH₃ | -CH(CH₃)(CH₂)₂CH₃ |
| 444 | -CH₂CH=CH-(CH₂)₂CH₃ | -CH(CH₃)(CH₂)₂CH₃ |
| 445 | -(CH₂)₂CH=CH-CH₂CH₃ | -CH(CH₃)(CH₂)₂CH₃ |
| 446 | -(CH₂)₃CH=CH-CH₃ | -CH(CH₃)(CH₂)₂CH₃ |
| 447 | -(CH₂)₄CH=CH₂ | -CH(CH₃)(CH₂)₂CH₃ |
| 448 | -CH=CH-(CH₂)₃CH₃ | -CH(CH₃)(CH₂)₂CH₃ |
| 449 | -CH₂CH=CH-(CH₂)₃CH₃ | -CH(CH₃)(CH₂)₂CH₃ |
| 450 | -(CH₂)₂CH=CH-(CH₂)₂CH₃ | -CH(CH₃)(CH₂)₂CH₃ |
| 451 | -(CH₂)₃CH=CH-CH₂CH₃ | -CH(CH₃)(CH₂)₂CH₃ |
| 452 | -(CH₂)₄CH=CH-CH₃ | -CH(CH₃)(CH₂)₂CH₃ |
| 453 | -(CH₂)₅CH=CH₂ | -CH(CH₃)(CH₂)₂CH₃ |
| 454 | -CH=CH-(CH₂)₅CH₃ | -CH(CH₃)(CH₂)₂CH₃ |
| 455 | -CH₂CH=CH-(CH₂)₄CH₃ | -CH(CH₃)(CH₂)₂CH₃ |
| 456 | -(CH₂)₂CH=CH-(CH₂)₃CH₃ | -CH(CH₃)(CH₂)₂CH₃ |
| 457 | -(CH₂)₃CH=CH-(CH₂)₂CH₃ | -CH(CH₃)(CH₂)₂CH₃ |
| 458 | -(CH₂)₄CH=CH-CH₂CH₃ | -CH(CH₃)(CH₂)₂CH₃ |
| 459 | -(CH₂)₅CH=CH-CH₃ | -CH(CH₃)(CH₂)₂CH₃ |
| 460 | -(CH₂)₆CH=CH₂ | -CH(CH₃)(CH₂)₂CH₃ |
| 461 | -CH₃ | -CH₂CH(CH₃)CH₂CH₃ |
| 462 | -CH₂CH₃ | -CH₂CH(CH₃)CH₂CH₃ |
| 463 | -CH₂CH₂CH₃ | -CH₂CH(CH₃)CH₂CH₃ |
| 464 | -CH(CH₃)₂ | -CH₂CH(CH₃)CH₂CH₃ |
| 465 | -CH₂CH₂CH₂CH₃ | -CH₂CH(CH₃)CH₂CH₃ |
| 466 | -CH(CH₃)CH₂CH₃ | -CH₂CH(CH₃)CH₂CH₃ |
| 467 | -CH₂CH(CH₃)₂ | -CH₂CH(CH₃)CH₂CH₃ |
| 468 | -C(CH₃)₃ | -CH₂CH(CH₃)CH₂CH₃ |
| 469 | -CH₂(CH₂)₃CH₃ | -CH₂CH(CH₃)CH₂CH₃ |
| 470 | -CH(CH₃)(CH₂)₂CH₃ | -CH₂CH(CH₃)CH₂CH₃ |
| 471 | -CH₂CH(CH₃)CH₂CH₃ | -CH₂CH(CH₃)CH₂CH₃ |
| 472 | -(CH₂)₂CH(CH₃)₂ | -CH₂CH(CH₃)CH₂CH₃ |
| 473 | -CH₂(CH₂)₄CH₃ | -CH₂CH(CH₃)CH₂CH₃ |
| 474 | -CH₂(CH₂)₅CH₃ | -CH₂CH(CH₃)CH₂CH₃ |
| 475 | -CH₂(CH₂)₆CH₃ | -CH₂CH(CH₃)CH₂CH₃ |
| 476 | -CH=CH₂ | -CH₂CH(CH₃)CH₂CH₃ |
| 477 | -CH=CHCH₃ | -CH₂CH(CH₃)CH₂CH₃ |
| 478 | -CH=C(CH₃)₂ | -CH₂CH(CH₃)CH₂CH₃ |
| 479 | -C(CH₃)=CH₂ | -CH₂CH(CH₃)CH₂CH₃ |
| 480 | -C(CH₃)=CHCH₃ | -CH₂CH(CH₃)CH₂CH₃ |
| 481 | -C(CH₃)=C(CH₃)₂ | -CH₂CH(CH₃)CH₂CH₃ |
| 482 | -CH=CH-CH₂CH₃ | -CH₂CH(CH₃)CH₂CH₃ |
| 483 | -CH₂CH=CH-CH₃ | -CH₂CH(CH₃)CH₂CH₃ |
| 484 | -(CH₂)₂CH=CH₂ | -CH₂CH(CH₃)CH₂CH₃ |
| 485 | -CH=CH-(CH₂)₂CH₃ | -CH₂CH(CH₃)CH₂CH₃ |
| 486 | -CH₂CH=CH-CH₂CH₃ | -CH₂CH(CH₃)CH₂CH₃ |
| 487 | -(CH₂)₂CH=CH-CH₃ | -CH₂CH(CH₃)CH₂CH₃ |
| 488 | -(CH₂)₃CH=CH₂ | -CH₂CH(CH₃)CH₂CH₃ |
| 489 | -CH=CH-(CH₂)₃CH₃ | -CH₂CH(CH₃)CH₂CH₃ |
| 490 | -CH₂CH=CH-(CH₂)₂CH₃ | -CH₂CH(CH₃)CH₂CH₃ |
| 491 | -(CH₂)₂CH=CH-CH₂CH₃ | -CH₂CH(CH₃)CH₂CH₃ |
| 492 | -(CH₂)₃CH=CH-CH₃ | -CH₂CH(CH₃)CH₂CH₃ |
| 493 | -(CH₂)₄CH=CH₂ | -CH₂CH(CH₃)CH₂CH₃ |
| 494 | -CH=CH-(CH₂)₃CH₃ | -CH₂CH(CH₃)CH₂CH₃ |
| 495 | -CH₂CH=CH-(CH₂)₃CH₃ | -CH₂CH(CH₃)CH₂CH₃ |
| 496 | -(CH₂)₂CH=CH-(CH₂)₂CH₃ | -CH₂CH(CH₃)CH₂CH₃ |
| 497 | -(CH₂)₃CH=CH-CH₂CH₃ | -CH₂CH(CH₃)CH₂CH₃ |
| 498 | -(CH₂)₄CH=CH-CH₃ | -CH₂CH(CH₃)CH₂CH₃ |
| 499 | -(CH₂)₅CH=CH₂ | -CH₂CH(CH₃)CH₂CH₃ |
| 500 | -CH=CH-(CH₂)₅CH₃ | -CH₂CH(CH₃)CH₂CH₃ |
| 501 | -CH₂CH=CH-(CH₂)₄CH₃ | -CH₂CH(CH₃)CH₂CH₃ |
| 502 | -(CH₂)₂CH=CH-(CH₂)₃CH₃ | -CH₂CH(CH₃)CH₂CH₃ |
| 503 | -(CH₂)₃CH=CH-(CH₂)₂CH₃ | -CH₂CH(CH₃)CH₂CH₃ |
| 504 | -(CH₂)₄CH=CH-CH₂CH₃ | -CH₂CH(CH₃)CH₂CH₃ |
| 505 | -(CH₂)₅CH=CH-CH₃ | -CH₂CH(CH₃)CH₂CH₃ |
| 506 | -(CH₂)₆CH=CH₂ | -CH₂CH(CH₃)CH₂CH₃ |
| 507 | -CH₃ | -(CH₂)₂CH(CH₃)₂ |
| 508 | -CH₂CH₃ | -(CH₂)₂CH(CH₃)₂ |
| 509 | -CH₂CH₂CH₃ | -(CH₂)₂CH(CH₃)₂ |
| 510 | -CH(CH₃)₂ | -(CH₂)₂CH(CH₃)₂ |
| 511 | -CH₂CH₂CH₂CH₃ | -(CH₂)₂CH(CH₃)₂ |
| 512 | -CH(CH₃)CH₂CH₃ | -(CH₂)₂CH(CH₃)₂ |
| 513 | -CH₂CH(CH₃)₂ | -(CH₂)₂CH(CH₃)₂ |
| 514 | -C(CH₃)₃ | -(CH₂)₂CH(CH₃)₂ |
| 515 | -CH₂(CH₂)₃CH₃ | -(CH₂)₂CH(CH₃)₂ |
| 516 | -CH(CH₃)(CH₂)₂CH₃ | -(CH₂)₂CH(CH₃)₂ |
| 517 | -CH₂CH(CH₃)CH₂CH₃ | -(CH₂)₂CH(CH₃)₂ |
| 518 | -(CH₂)₂CH(CH₃)₂ | -(CH₂)₂CH(CH₃)₂ |
| 519 | -CH₂(CH₂)₄CH₃ | -(CH₂)₂CH(CH₃)₂ |
| 520 | -CH₂(CH₂)₅CH₃ | -(CH₂)₂CH(CH₃)₂ |
| 521 | -CH₂(CH₂)₆CH₃ | -(CH₂)₂CH(CH₃)₂ |
| 522 | -CH=CH₂ | -(Ch₂)₂CH(CH₃)₂ |
| 523 | -CH=CHCH₃ | -(CH₂)₂CH(CH₃)₂ |
| 524 | -CH=C(CH₃)₂ | -(CH₂)₂CH(CH₃)₂ |
| 525 | -C(CH₃)=CH₂ | -(CH₂)₂CH(CH₃)₂ |
| 526 | -C(CH₃)=CHCH₃ | -(CH₂)₂CH(CH₃)₂ |
| 527 | -C(CH₃)=C(CH₃)₂ | -(CH₂)₂CH(CH₃)₂ |
| 528 | -CH=CH-CH₂CH₃ | -(CH₂)₂CH(CH₃)₂ |
| 529 | -CH₂CH=CH-CH₃ | -(CH₂)₂CH(CH₃)₂ |
| 530 | -(CH₂)₂CH=CH₂ | -(CH₂)₂CH(CH₃)₂ |
| 531 | -CH=CH-(CH₂)₂CH₃ | -(CH₂)₂CH(CH₃)₂ |
| 532 | -CH₂CH=CH-CH₂CH₃ | -(CH₂)₂CH(CH₃)₂ |
| 533 | -(CH₂)₂CH=CH-CH₃ | -(CH₂)₂CH(CH₃)₂ |
| 534 | -(CH₂)₃CH=CH₂ | -(CH₂)₂CH(CH₃)₂ |
| 535 | -CH=CH-(CH₂)₃CH₃ | -(CH₂)₂CH(CH₃)₂ |
| 536 | -CH₂CH=CH-(CH₂)₂CH₃ | -(CH₂)₂CH(CH₃)₂ |
| 537 | -(CH₂)₂CH=CH-CH₂CH₃ | -(CH₂)₂CH(CH₃)₂ |
| 538 | -(CH₂)₃CH=CH-CH₃ | -(CH₂)₂CH(CH₃)₂ |
| 539 | -(CH₂)₄CH=CH₂ | -(CH₂)₂CH(CH₃)₂ |
| 540 | -CH=CH-(CH₂)₃CH₃ | -(CH₂)₂CH(CH₃)₂ |
| 541 | -CH₂CH=CH-(CH₂)₃CH₃ | -(CH₂)₂CH(CH₃)₂ |
| 542 | -(CH₂)₂CH=CH-(CH₂)₂CH₃ | -(CH₂)₂CH(CH₃)₂ |
| 543 | -(CH₂)₃CH=CH-CH₂CH₃ | -(CH₂)₂CH(CH₃)₂ |
| 544 | -(CH₂)₄CH=CH-CH₃ | -(CH₂)₂CH(CH₃)₂ |
| 545 | -(CH₂)₅CH=CH₂ | -(CH₂)₂CH(CH₃)₂ |
| 546 | -CH=CH-(CH₂)₅CH₃ | -(CH₂)₂CH(CH₃)₂ |
| 547 | -CH₂CH=CH-(CH₂)₄CH₃ | -(CH₂)₂CH(CH₃)₂ |
| 548 | -(CH₂)₂CH=CH-(CH₂)₃CH₃ | -(CH₂)₂CH(CH₃)₂ |
| 549 | -(CH₂)₃CH=CH-(CH₂)₂CH₃ | -(CH₂)₂CH(CH₃)₂ |
| 550 | -(CH₂)₄CH=CH-CH₂CH₃ | -(CH₂)₂CH(CH₃)₂ |
| 551 | -(CH₂)₅CH=CH-CH₃ | -(CH₂)₂CH(CH₃)₂ |
| 552 | -(CH₂)₆CH=CH₂ | -(CH₂)₂CH(CH₃)₂ |
| 553 | -CH₃ | -CH₂(CH₂)₄CH₃ |
| 554 | -CH₂CH₃ | -CH₂(CH₂)₄CH₃ |
| 555 | -CH₂CH₂CH₃ | -CH₂(CH₂)₄CH₃ |
| 556 | -CH(CH₃)₂ | -CH₂(CH₂)₄CH₃ |
| 557 | -CH₂CH₂CH₂CH₃ | -CH₂(CH₂)₄CH₃ |
| 558 | -CH(CH₃)CH₂CH₃ | -CH₂(CH₂)₄CH₃ |
| 559 | -CH₂CH(CH₃)₂ | -CH₂(CH₂)₄CH₃ |
| 560 | -C(CH₃)₃ | -CH₂(CH₂)₄CH₃ |
| 561 | -CH₂(CH₂)₃CH₃ | -CH₂(CH₂)₄CH₃ |
| 562 | -CH(CH₃)(CH₂)₂CH₃ | -CH₂(CH₂)₄CH₃ |
| 563 | -CH₂CH(CH₃)CH₂CH₃ | -CH₂(CH₂)₄CH₃ |
| 564 | -(CH₂)₂CH(CH₃)₂ | -CH₂(CH₂)₄CH₃ |
| 565 | -CH₂(CH₂)₄CH₃ | -CH₂(CH₂)₄CH₃ |
| 566 | -CH₂(CH₂)₅CH₃ | -CH₂(CH₂)₄CH₃ |
| 567 | -CH₂(CH₂)₆CH₃ | -CH₂(CH₂)₄CH₃ |
| 568 | -CH=CH₂ | -CH₂(CH₂)₄CH₃ |
| 569 | -CH=CHCH₃ | -CH₂(CH₂)₄CH₃ |
| 570 | -CH=C(CH₃)₂ | -CH₂(CH₂)₄CH₃ |
| 571 | -C(CH₃)=CH₂ | -CH₂(CH₂)₄CH₃ |
| 572 | -C(CH₃)=CHCH₃ | -CH₂(CH₂)₄CH₃ |
| 573 | -C(CH₃)=C(CH₃)₂ | -CH₂(CH₂)₄CH₃ |
| 574 | -CH=CH-CH₂CH₃ | -CH₂(CH₂)₄CH₃ |
| 575 | -CH₂CH=CH-CH₃ | -CH₂(CH₂)₄CH₃ |
| 576 | -(CH₂)₂CH=CH₂ | -CH₂(CH₂)₄CH₃ |
| 577 | -CH=CH-(CH₂)₂CH₃ | -CH₂(CH₂)₄CH₃ |
| 578 | -CH₂CH=CH-CH₂CH₃ | -CH₂(CH₂)₄CH₃ |
| 579 | -(CH₂)₂CH=CH-CH₃ | -CH₂(CH₂)₄CH₃ |
| 580 | -(CH₂)₃CH=CH₂ | -CH₂(CH₂)₄CH₃ |
| 581 | -CH=CH-(CH₂)₃CH₃ | -CH₂(CH₂)₄CH₃ |
| 582 | -CH₂CH=CH-(CH₂)₂CH₃ | -CH₂(CH₂)₄CH₃ |
| 583 | -(CH₂)₂CH=CH-CH₂CH₃ | -CH₂(CH₂)₄CH₃ |
| 584 | -(CH₂)₃CH=CH-CH₃ | -CH₂(CH₂)₄CH₃ |
| 585 | -(CH₂)₄CH=CH₂ | -CH₂(CH₂)₄CH₃ |
| 586 | -CH=CH-(CH₂)₃CH₃ | -CH₂(CH₂)₄CH₃ |
| 587 | -CH₂CH=CH-(CH₂)₃CH₃ | -CH₂(CH₂)₄CH₃ |
| 588 | -(CH₂)₂CH=CH-(CH₂)₂CH₃ | -CH₂(CH₂)₄CH₃ |
| 589 | -(CH₂)₃CH=CH-CH₂CH₃ | -CH₂(CH₂)₄CH₃ |
| 590 | -(CH₂)₄CH=CH-CH₃ | -CH₂(CH₂)₄CH₃ |
| 591 | -(CH₂)₅CH=CH₂ | -CH₂(CH₂)₄CH₃ |
| 592 | -CH=CH-(CH₂)₅CH₃ | -CH₂(CH₂)₄CH₃ |
| 593 | -CH₂CH=CH-(CH₂)₄CH₃ | -CH₂(CH₂)₄CH₃ |
| 594 | -(CH₂)₂CH=CH-(CH₂)₃CH₃ | -CH₂(CH₂)₄CH₃ |
| 595 | -(CH₂)₃CH=CH-(CH₂)₂CH₃ | -CH₂(CH₂)₄CH₃ |
| 596 | -(CH₂)₄CH=CH-CH₂CH₃ | -CH₂(CH₂)₄CH₃ |
| 597 | -(CH₂)₅CH=CH-CH₃ | -CH₂(CH₂)₄CH₃ |
| 598 | -(CH₂)₆CH=CH₂ | -CH₂(CH₂)₄CH₃ |
| 599 | -CH₃ | -CH₂(CH₂)₅CH₃ |
| 600 | -CH₂CH₃ | -CH₂(CH₂)₅CH₃ |
| 601 | -CH₂CH₂CH₃ | -CH₂(CH₂)₅CH₃ |
| 602 | -CH(CH₃)₂ | -CH₂(CH₂)₅CH₃ |
| 603 | -CH₂CH₂CH₂CH₃ | -CH₂(CH₂)₅CH₃ |
| 604 | -CH(CH₃)CH₂CH₃ | -CH₂(CH₂)₅CH₃ |
| 605 | -CH₂CH(CH₃)₂ | -CH₂(CH₂)₅CH₃ |
| 606 | -C(CH₃)₃ | -CH₂(CH₂)₅CH₃ |
| 607 | -CH₂(CH₂)₃CH₃ | -CH₂(CH₂)₅CH₃ |
| 608 | -CH(CH₃)(CH₂)₂CH₃ | -CH₂(CH₂)₅CH₃ |
| 609 | -CH₂CH(CH₃)CH₂CH₃ | -CH₂(CH₂)₅CH₃ |
| 610 | -(CH₂)₂CH(CH₃)₂ | -CH₂(CH₂)₅CH₃ |
| 611 | -CH₂(CH₂)₄CH₃ | -CH₂(CH₂)₅CH₃ |
| 612 | -CH₂(CH₂)₅CH₃ | -CH₂(CH₂)₅CH₃ |
| 613 | -CH₂(CH₂)₆CH₃ | -CH₂(CH₂)₅CH₃ |
| 614 | -CH=CH₂ | -CH₂(CH₂)₅CH₃ |
| 615 | -CH=CHCH₃ | -CH₂(CH₂)₅CH₃ |
| 616 | -CH=C(CH₃)₂ | -CH₂(CH₂)₅CH₃ |
| 617 | -C(CH₃)=CH₂ | -CH₂(CH₂)₅CH₃ |
| 618 | -C(CH₃)=CHCH₃ | -CH₂(CH₂)₅CH₃ |
| 619 | -C(CH₃)=C(CH₃)₂ | -CH₂(CH₂)₅CH₃ |
| 620 | -CH=CH-CH₂CH₃ | -CH₂(CH₂)₅CH₃ |
| 621 | -CH₂CH=CH-CH₃ | -CH₂(CH₂)₅CH₃ |
| 622 | -(CH₂)₂CH=CH₂ | -CH₂(CH₂)₅CH₃ |
| 623 | -CH=CH-(CH₂)₂CH₃ | -CH₂(CH₂)₅CH₃ |
| 624 | -CH₂CH=CH-CH₂CH₃ | -CH₂(CH₂)₅CH₃ |
| 625 | -(CH₂)₂CH=CH-CH₃ | -CH₂(CH₂)₅CH₃ |
| 626 | -(CH₂)₃CH=CH₂ | -CH₂(CH₂)₅CH₃ |
| 627 | -CH=CH-(CH₂)₃CH₃ | -CH₂(CH₂)₅CH₃ |
| 628 | -CH₂CH=CH-(CH₂)₂CH₃ | -CH₂(CH₂)₅CH₃ |
| 629 | -(CH₂)₂CH=CH-CH₂CH₃ | -CH₂(CH₂)₅CH₃ |
| 630 | -(CH₂)₃CH=CH-CH₃ | -CH₂(CH₂)₅CH₃ |
| 631 | -(CH₂)₄CH=CH₂ | -CH₂(CH₂)₅CH₃ |
| 632 | -CH=CH-(CH₂)₃CH₃ | -CH₂(CH₂)₅CH₃ |
| 633 | -CH₂CH=CH-(CH₂)₃CH₃ | -CH₂(CH₂)₅CH₃ |
| 634 | -(CH₂)₂CH=CH-(CH₂)₂CH₃ | -CH₂(CH₂)₅CH₃ |
| 635 | -(CH₂)₃CH=CH-CH₂CH₃ | -CH₂(CH₂)₅CH₃ |
| 636 | -(CH₂)₄CH=CH-CH₃ | -CH₂(CH₂)₅CH₃ |
| 637 | -(CH₂)₅CH=CH₂ | -CH₂(CH₂)₅CH₃ |
| 638 | -CH=CH-(CH₂)₅CH₃ | -CH₂(CH₂)₅CH₃ |
| 639 | -CH₂CH=CH-(CH₂)₄CH₃ | -CH₂(CH₂)₅CH₃ |
| 640 | -(CH₂)₂CH=CH-(CH₂)₃CH₃ | -CH₂(CH₂)₅CH₃ |
| 641 | -(CH₂)₃CH=CH-(CH₂)₂CH₃ | -CH₂(CH₂)₅CH₃ |
| 642 | -(CH₂)₄CH=CH-CH₂CH₃ | -CH₂(CH₂)₅CH₃ |
| 643 | -(CH₂)₅CH=CH-CH₃ | -CH₂(CH₂)₅CH₃ |
| 644 | -(CH₂)₆CH=CH₂ | -CH₂(CH₂)₅CH₃ |
| 645 | -CH₃ | -CH₂(CH₂)₆CH₃ |
| 646 | -CH₂CH₃ | -CH₂(CH₂)₆CH₃ |
| 647 | -CH₂CH₂CH₃ | -CH₂(CH₂)₆CH₃ |
| 648 | -CH(CH₃)₂ | -CH₂(CH₂)₆CH₃ |
| 649 | -CH₂CH₂CH₂CH₃ | -CH₂(CH₂)₆CH₃ |
| 650 | -CH(CH₃)CH₂CH₃ | -CH₂(CH₂)₆CH₃ |
| 651 | -CH₂CH(CH₃)₂ | -CH₂(CH₂)₆CH₃ |
| 652 | -C(CH₃)₃ | -CH₂(CH₂)₆CH₃ |
| 653 | -CH₂(CH₂)₃CH₃ | -CH₂(CH₂)₆CH₃ |
| 654 | -CH(CH₃)(CH₂)₂CH₃ | -CH₂(CH₂)₆CH₃ |
| 655 | -CH₂CH(CH₃)CH₂CH₃ | -CH₂(CH₂)₆CH₃ |
| 656 | -(CH₂)₂CH(CH₃)₂ | -CH₂(CH₂)₆CH₃ |
| 657 | -CH₂(CH₂)₄CH₃ | -CH₂(CH₂)₆CH₃ |
| 658 | -CH₂(CH₂)₅CH₃ | -CH₂(CH₂)₆CH₃ |
| 59 | -CH₂(CH₂)₆CH₃ | -CH₂(CH₂)₆CH₃ |
| 660 | -CH=CH₂ | -CH₂(CH₂)₆CH₃ |
| 661 | -CH=CHCH₃ | -CH₂(CH₂)₆CH₃ |
| 662 | -CH=C(CH₃)₂ | -CH₂(CH₂)₆CH₃ |
| 663 | -C(CH₃)=CH₂ | -CH₂(CH₂)₆CH₃ |
| 664 | -C(CH₃)=CHCH₃ | -CH₂(CH₂)₆CH₃ |
| 665 | -C(CH₃)=C(CH₃)₂ | -CH₂(CH₂)₆CH₃ |
| 666 | -CH=CH-CH₂CH₃ | -CH₂(CH₂)₆CH₃ |
| 667 | -CH₂CH=CH-CH₃ | -CH₂(CH₂)₆CH₃ |
| 668 | -(CH₂)₂CH=CH₂ | -CH₂(CH₂)₆CH₃ |
| 669 | -CH=CH-(CH₂)₂CH₃ | -CH₂(CH₂)₆CH₃ |
| 670 | -CH₂CH=CH-CH₂CH₃ | -CH₂(CH₂)₆CH₃ |
| 671 | -(CH₂)₂CH=CH-CH₃ | -CH₂(CH₂)₆CH₃ |
| 672 | -(CH₂)₃CH=CH₂ | -CH₂(CH₂)₆CH₃ |
| 673 | -CH=CH-(CH₂)₃CH₃ | -CH₂(CH₂)₆CH₃ |
| 674 | -CH₂CH=CH-(CH₂)₂CH₃ | -CH₂(CH₂)₆CH₃ |
| 675 | -(CH₂)₂CH=CH-CH₂CH₃ | -CH₂(CH₂)₆CH₃ |
| 676 | -(CH₂)₃CH=CH-CH₃ | -CH₂(CH₂)₆CH₃ |
| 677 | -(CH₂)₄CH=CH₂ | -CH₂(CH₂)₆CH₃ |
| 678 | -CH=CH-(CH₂)₃CH₃ | -CH₂(CH₂)₆CH₃ |
| 679 | -CH₂CH=CH-(CH₂)₃CH₃ | -CH₂(CH₂)₆CH₃ |
| 680 | -(CH₂)₂CH=CH-(CH₂)₂CH₃ | -CH₂(CH₂)₆CH₃ |
| 681 | -(CH₂)₃CH=CH-CH₂CH₃ | -CH₂(CH₂)₆CH₃ |
| 682 | -(CH₂)₄CH=CH-CH₃ | -CH₂(CH₂)₆CH₃ |
| 683 | -(CH₂)₅CH=CH₂ | -CH₂(CH₂)₆CH₃ |
| 684 | -CH=CH-(CH₂)₅CH₃ | -CH₂(CH₂)₆CH₃ |
| 685 | -CH₂CH=CH-(CH₂)₄CH₃ | -CH₂(CH₂)₆CH₃ |
| 686 | -(CH₂)₂CH=CH-(CH₂)₃CH₃ | -CH₂(CH₂)₆CH₃ |
| 687 | -(CH₂)₃CH=CH-(CH₂)₂CH₃ | -CH₂(CH₂)₆CH₃ |
| 688 | -(CH₂)₄CH=CH-CH₂CH₃ | -CH₂(CH₂)₆CH₃ |
| 689 | -(CH₂)₅CH=CH-CH₃ | -CH₂(CH₂)₆CH₃ |
| 690 | -(CH₂)₆CH=CH₂ | -CH₂(CH₂)₆CH₃ |
| 691 | -CH₃ | -CH=CH₂ |
| 692 | -CH₂CH₃ | -CH=CH₂ |
| 693 | -CH₂CH₂CH₃ | -CH=CH₂ |
| 694 | -CH(CH₃)₂ | -CH=CH₂ |
| 695 | -CH₂CH₂CH₂CH₃ | -CH=CH₂ |
| 696 | -CH(CH₃)CH₂CH₃ | -CH=CH₂ |
| 697 | -CH₂CH(CH₃)₂ | -CH=CH₂ |
| 698 | -C(CH₃)₃ | -CH=CH₂ |
| 699 | -CH₂(CH₂)₃CH₃ | -CH=CH₂ |
| 700 | -CH(CH₃)(CH₂)₂CH₃ | -CH=CH₂ |
| 701 | -CH₂CH(CH₃)CH₂CH₃ | -CH=CH₂ |
| 702 | -(CH₂)₂CH(CH₃)₂ | -CH=CH₂ |
| 703 | -CH₂(CH₂)₄CH₃ | -CH=CH₂ |
| 704 | -CH₂(CH₂)₅CH₃ | -CH=CH₂ |
| 705 | -CH₂(CH₂)₆CH₃ | -CH=CH₂ |
| 706 | -CH=CH₂ | -CH=CH₂ |
| 707 | -CH=CHCH₃ | -CH=CH₂ |
| 708 | -CH=C(CH₃)₂ | -CH=CH₂ |
| 709 | -C(CH₃)=CH₂ | -CH=CH₂ |
| 710 | -C(CH₃)=CHCH₃ | -CH=CH₂ |
| 711 | -C(CH₃)=C(CH₃)₂ | -CH=CH₂ |
| 712 | -CH=CH-CH₂CH₃ | -CH=CH₂ |
| 713 | -CH₂CH=CH-CH₃ | -CH=CH₂ |
| 714 | -(CH₂)₂CH=CH₂ | -CH=CH₂ |
| 715 | -CH=CH-(CH₂)₂CH₃ | -CH=CH₂ |
| 716 | -CH₂CH=CH-CH₂CH₃ | -CH=CH₂ |
| 717 | -(CH₂)₂CH=CH-CH₃ | -CH=CH₂ |
| 718 | -(CH₂)₃CH=CH₂ | -CH=CH₂ |
| 719 | -CH=CH-(CH₂)₃CH₃ | -CH=CH₂ |
| 720 | -CH₂CH=CH-(CH₂)₂CH₃ | -CH=CH₂ |
| 721 | -(CH₂)₂CH=CH-CH₂CH₃ | -CH=CH₂ |
| 722 | -(CH₂)₃CH=CH-CH₃ | -CH=CH₂ |
| 723 | -(CH₂)₄CH=CH₂ | -CH=CH₂ |
| 724 | -CH=CH-(CH₂)₃CH₃ | -CH=CH₂ |
| 725 | -CH₂CH=CH-(CH₂)₃CH₃ | -CH=CH₂ |
| 726 | -(CH₂)₂CH=CH-(CH₂)₂CH₃ | -CH=CH₂ |
| 727 | -(CH₂)₃CH=CH-CH₂CH₃ | -CH=CH₂ |
| 728 | -(CH₂)₄CH=CH-CH₃ | -CH=CH₂ |
| 729 | -(CH₂)₅CH=CH₂ | -CH=CH₂ |
| 730 | -CH=CH-(CH₂)₅CH₃ | -CH=CH₂ |
| 731 | -CH₂CH=CH-(CH₂)₄CH₃ | -CH=CH₂ |
| 732 | -(CH₂)₂CH=CH-(CH₂)₃CH₃ | -CH=CH₂ |
| 733 | -(CH₂)₃CH=CH-(CH₂)₂CH₃ | -CH=CH₂ |
| 734 | -(CH₂)₄CH=CH-CH₂CH₃ | -CH=CH₂ |
| 735 | -(CH₂)₅CH=CH-CH₃ | -CH=CH₂ |
| 736 | -(CH₂)₆CH=CH₂ | -CH=CH₂ |
| 737 | -CH₃ | -C(CH₃)=CH₂ |
| 738 | -CH₂CH₃ | -C(CH₃)=CH₂ |
| 739 | -CH₂CH₂CH₃ | -C(CH₃)=CH₂ |
| 740 | -CH(CH₃)₂ | -C(CH₃)=CH₂ |
| 741 | -CH₂CH₂CH₂CH₃ | -C(CH₃)=CH₂ |
| 742 | -CH(CH₃)CH₂CH₃ | -C(CH₃)=CH₂ |
| 743 | -CH₂CH(CH₃)₂ | -C(CH₃)=CH₂ |
| 744 | -C(CH₃)₃ | -C(CH₃)=CH₂ |
| 745 | -CH₂(CH₂)₃CH₃ | -C(CH₃)=CH₂ |
| 746 | -CH(CH₃)(CH₂)₂CH₃ | -C(CH₃)=CH₂ |
| 747 | -CH₂CH(CH₃)CH₂CH₃ | -C(CH₃)=CH₂ |
| 748 | -(CH₂)₂CH(CH₃)₂ | -C(CH₃)=CH₂ |
| 749 | -CH₂(CH₂)₄CH₃ | -C(CH₃)=CH₂ |
| 750 | -CH₂(CH₂)₅CH₃ | -C(CH₃)=CH₂ |
| 751 | -CH₂(CH₂)₆CH₃ | -C(CH₃)=CH₂ |
| 752 | -CH=CH₂ | -C(CH₃)=CH₂ |
| 753 | -CH=CHCH₃ | -C(CH₃)=CH₂ |
| 754 | -CH=C(CH₃)₂ | -C(CH₃)=CH₂ |
| 755 | -C(CH₃)=CH₂ | -C(CH₃)=CH₂ |
| 756 | -C(CH₃)=CHCH₃ | -C(CH₃)=CH₂ |
| 757 | -C(CH₃)=C(CH₃)₂ | -C(CH₃)=CH₂ |
| 758 | -CH=CH-CH₂CH₃ | -C(CH₃)=CH₂ |
| 759 | -CH₂CH=CH-CH₃ | -C(CH₃)=CH₂ |
| 760 | -(CH₂)₂CH=CH₂ | -C(CH₃)=CH₂ |
| 761 | -CH=CH-(CH₂)₂CH₃ | -C(CH₃)=CH₂ |
| 762 | -CH₂CH=CH-CH₂CH₃ | -C(CH₃)=CH₂ |
| 763 | -(CH₂)₂CH=CH-CH₃ | -C(CH₃)=CH₂ |
| 764 | -(CH₂)₃CH=CH₂ | -C(CH₃)=CH₂ |
| 765 | -CH=CH-(CH₂)₃CH₃ | -C(CH₃)=CH₂ |
| 766 | -CH₂CH=CH-(CH₂)₂CH₃ | -C(CH₃)=CH₂ |
| 767 | -(CH₂)₂CH=CH-CH₂CH₃ | -C(CH₃)=CH₂ |
| 768 | -(CH₂)₃CH=CH-CH₃ | -C(CH₃)=CH₂ |
| 769 | -(CH₂)₄CH=CH₂ | -C(CH₃)=CH₂ |
| 770 | -CH=CH-(CH₂)₃CH₃ | -C(CH₃)=CH₂ |
| 771 | -CH₂CH=CH-(CH₂)₃CH₃ | -C(CH₃)=CH₂ |
| 772 | -(CH₂)₂CH=CH-(CH₂)₂CH₃ | -C(CH₃)=CH₂ |
| 773 | -(CH₂)₃CH=CH-CH₂CH₃ | -C(CH₃)=CH₂ |
| 774 | -(CH₂)₄CH=CH-CH₃ | -C(CH₃)=CH₂ |
| 775 | -(CH₂)₅CH=CH₂ | -C(CH₃)=CH₂ |
| 776 | -CH=CH-(CH₂)₅CH₃ | -C(CH₃)=CH₂ |
| 777 | -CH₂CH=CH-(CH₂)₄CH₃ | -C(CH₃)=CH₂ |
| 778 | -(CH₂)₂CH=CH-(CH₂)₃CH₃ | -C(CH₃)=CH₂ |
| 779 | -(CH₂)₃CH=CH-(CH₂)₂CH₃ | -C(CH₃)=CH₂ |
| 780 | -(CH₂)₄CH=CH-CH₂CH₃ | -C(CH₃)=CH₂ |
| 781 | -(CH₂)₅CH=CH-CH₃ | -C(CH₃)=CH₂ |
| 782 | -(CH₂)₆CH=CH₂ | -C(CH₃)=CH₂ |
| 783 | -CH₃ | -O-CH₃ |
| 784 | -CH₂CH₃ | -O-CH₃ |
| 785 | -CH₂CH₂CH₃ | -O-CH₃ |
| 786 | -CH(CH₃)₂ | -O-CH₃ |
| 787 | -CH₂CH₂CH₂CH₃ | -O-CH₃ |
| 788 | -CH(CH₃)CH₂CH₃ | -O-CH₃ |
| 789 | -CH₂CH(CH₃)₂ | -O-CH₃ |
| 790 | -C(CH₃)₃ | -O-CH₃ |
| 791 | -CH₂(CH₂)₃CH₃ | -O-CH₃ |
| 792 | -CH(CH₃)(CH₂)₂CH₃ | -O-CH₃ |
| 793 | -CH₂CH(CH₃)CH₂CH₃ | -O-CH₃ |
| 794 | -(CH₂)₂CH(CH₃)₂ | -O-CH₃ |
| 795 | -CH₂(CH₂)₄CH₃ | -O-CH₃ |
| 796 | -CH₂(CH₂)₅CH₃ | -O-CH₃ |
| 797 | -CH₂(CH₂)₆CH₃ | -O-CH₃ |
| 798 | -CH=CH₂ | -O-CH₃ |
| 799 | -CH=CHCH₃ | -O-CH₃ |
| 800 | -CH=C(CH₃)₂ | -O-CH₃ |
| 801 | -C(CH₃)=CH₂ | -O-CH₃ |
| 802 | -C(CH₃)=CHCH₃ | -O-CH₃ |
| 803 | -C(CH₃)=C(CH₃)₂ | -O-CH₃ |
| 804 | -CH=CH-CH₂CH₃ | -O-CH₃ |
| 805 | -CH₂CH=CH-CH₃ | -O-CH₃ |
| 806 | -(CH₂)₂CH=CH₂ | -O-CH₃ |
| 807 | -CH=CH-(CH₂)₂CH₃ | -O-CH₃ |
| 808 | -CH₂CH=CH-CH₂CH₃ | -O-CH₃ |
| 809 | -(CH₂)₂CH=CH-CH₃ | -O-CH₃ |
| 810 | -(CH₂)₃CH=CH₂ | -O-CH₃ |
| 811 | -CH=CH-(CH₂)₃CH₃ | -O-CH₃ |
| 812 | -CH₂CH=CH-(CH₂)₂CH₃ | -O-CH₃ |
| 813 | -(CH₂)₂CH=CH-CH₂CH₃ | -O-CH₃ |
| 814 | -(CH₂)₃CH=CH-CH₃ | -O-CH₃ |
| 815 | -(CH₂)₄CH=CH₂ | -O-CH₃ |
| 816 | -CH=CH-(CH₂)₃CH₃ | -O-CH₃ |
| 817 | -CH₂CH=CH-(CH₂)₃CH₃ | -O-CH₃ |
| 818 | -(CH₂)₂CH=CH-(CH₂)₂CH₃ | -O-CH₃ |
| 819 | -(CH₂)₃CH=CH-CH₂CH₃ | -O-CH₃ |
| 820 | -(CH₂)₄CH=CH-CH₃ | -O-CH₃ |
| 821 | -(CH₂)₅CH=CH₂ | -O-CH₃ |
| 822 | -CH=CH-(CH₂)₅CH₃ | -O-CH₃ |
| 823 | -CH₂CH=CH-(CH₂)₄CH₃ | -O-CH₃ |
| 824 | -(CH₂)₂CH=CH-(CH₂)₃CH₃ | -O-CH₃ |
| 825 | -(CH₂)₃CH=CH-(CH₂)₂CH₃ | -O-CH₃ |
| 826 | -(CH₂)₄CH=CH-CH₂CH₃ | -O-CH₃ |
| 827 | -(CH₂)₅CH=CH-CH₃ | -O-CH₃ |
| 828 | -(CH₂)₆CH=CH₂ | -O-CH₃ |
| 829 | -CH₃ | -O-CH₂CH₃ |
| 830 | -CH₂CH₃ | -O-CH₂CH₃ |
| 831 | -CH₂CH₂CH₃ | -O-CH₂CH₃ |
| 832 | -CH(CH₃)₂ | -O-CH₂CH₃ |
| 833 | -CH₂CH₂CH₂CH₃ | -O-CH₂CH₃ |
| 834 | -CH(CH₃)CH₂CH₃ | -O-CH₂CH₃ |
| 835 | -CH₂CH(CH₃)₂ | -O-CH₂CH₃ |
| 836 | -C(CH₃)₃ | -O-CH₂CH₃ |
| 837 | -CH₂(CH₂)₃CH₃ | -O-CH₂CH₃ |
| 838 | -CH(CH₃)(CH₂)₂CH₃ | -O-CH₂CH₃ |
| 839 | -CH₂CH(CH₃)CH₂CH₃ | -O-CH₂CH₃ |
| 840 | -(CH₂)₂CH(CH₃)₂ | -O-CH₂CH₃ |
| 841 | -CH₂(CH₂)₄CH₃ | -O-CH₂CH₃ |
| 842 | -CH₂(CH₂)₅CH₃ | -O-CH₂CH₃ |
| 843 | -CH₂(CH₂)₆CH₃ | -O-CH₂CH₃ |
| 844 | -CH=CH₂ | -O-CH₂CH₃ |
| 845 | -CH=CHCH₃ | -O-CH₂CH₃ |
| 846 | -CH=C(CH₃)₂ | -O-CH₂CH₃ |
| 847 | -C(CH₃)=CH₂ | -O-CH₂CH₃ |
| 848 | -C(CH₃)=CHCH₃ | -O-CH₂CH₃ |
| 849 | -C(CH₃)=C(CH₃)₂ | -O-CH₂CH₃ |
| 850 | -CH=CH-CH₂CH₃ | -O-CH₂CH₃ |
| 851 | -CH₂CH=CH-CH₃ | -O-CH₂CH₃ |
| 852 | -(CH₂)₂CH=CH₂ | -O-CH₂CH₃ |
| 853 | -CH=CH-(CH₂)₂CH₃ | -O-CH₂CH₃ |
| 854 | -CH₂CH=CH-CH₂CH₃ | -O-CH₂CH₃ |
| 855 | -(CH₂)₂CH=CH-CH₃ | -O-CH₂CH₃ |
| 856 | -(CH₂)₃CH=CH₂ | -O-CH₂CH₃ |
| 857 | -CH=CH-(CH₂)₃CH₃ | -O-CH₂CH₃ |
| 858 | -CH₂CH=CH-(CH₂)₂CH₃ | -O-CH₂CH₃ |
| 859 | -(CH₂)₂CH=CH-CH₂CH₃ | -O-CH₂CH₃ |
| 860 | -(CH₂)₃CH=CH-CH₃ | -O-CH₂CH₃ |
| 861 | -(CH₂)₄CH=CH₂ | -O-CH₂CH₃ |
| 862 | -CH=CH-(CH₂)₃CH₃ | -O-CH₂CH₃ |
| 863 | -CH₂CH=CH-(CH₂)₃CH₃ | -O-CH₂CH₃ |
| 864 | -(CH₂)₂CH=CH-(CH₂)₂CH₃ | -O-CH₂CH₃ |
| 865 | -(CH₂)₃CH=CH-CH₂CH₃ | -O-CH₂CH₃ |
| 866 | -(CH₂)₄CH=CH-CH₃ | -O-CH₂CH₃ |
| 867 | -(CH₂)₅CH=CH₂ | -O-CH₂CH₃ |
| 868 | -CH=CH-(CH₂)₅CH₃ | -O-CH₂CH₃ |
| 869 | -CH₂CH=CH-(CH₂)₄CH₃ | -O-CH₂CH₃ |
| 870 | -(CH₂)₂CH=CH-(CH₂)₃CH₃ | -O-CH₂CH₃ |
| 871 | -(CH₂)₃CH=CH-(CH₂)₂CH₃ | -O-CH₂CH₃ |
| 872 | -(CH₂)₄CH=CH-CH₂CH₃ | -O-CH₂CH₃ |
| 873 | -(CH₂)₅CH=CH-CH₃ | -O-CH₂CH₃ |
| 874 | -(CH₂)₆CH=CH₂ | -O-CH₂CH₃ |
| 875 | -CH₃ | -O-CH₂CH₂CH₃ |
| 876 | -CH₂CH₃ | -O-CH₂CH₂CH₃ |
| 877 | -CH₂CH₂CH₃ | -O-CH₂CH₂CH₃ |
| 878 | -CH(CH₃)₂ | -O-CH₂CH₂CH₃ |
| 879 | -CH₂CH₂CH₂CH₃ | -O-CH₂CH₂CH₃ |
| 880 | -CH(CH₃)CH₂CH₃ | -O-CH₂CH₂CH₃ |
| 881 | -CH₂CH(CH₃)₂ | -O-CH₂CH₂CH₃ |
| 882 | -C(CH₃)₃ | -O-CH₂CH₂CH₃ |
| 883 | -CH₂(CH₂)₃CH₃ | -O-CH₂CH₂CH₃ |
| 884 | -CH(CH₃)(CH₂)₂CH₃ | -O-CH₂CH₂CH₃ |
| 885 | -CH₂CH(CH₃)CH₂CH₃ | -O-CH₂CH₂CH₃ |
| 886 | -(CH₂)₂CH(CH₃)₂ | -O-CH₂CH₂CH₃ |
| 887 | -CH₂(CH₂)₄CH₃ | -O-CH₂CH₂CH₃ |
| 888 | -CH₂(CH₂)₅CH₃ | -O-CH₂CH₂CH₃ |
| 889 | -CH₂(CH₂)₆CH₃ | -O-CH₂CH₂CH₃ |
| 890 | -CH=CH₂ | -O-CH₂CH₂CH₃ |
| 891 | -CH=CHCH₃ | -O-CH₂CH₂CH₃ |
| 892 | -CH=C(CH₃)₂ | -O-CH₂CH₂CH₃ |
| 893 | -C(CH₃)=CH₂ | -O-CH₂CH₂CH₃ |
| 894 | -C(CH₃)=CHCH₃ | -O-CH₂CH₂CH₃ |
| 895 | -C(CH₃)=C(CH₃)₂ | -O-CH₂CH₂CH₃ |
| 896 | -CH=CH-CH₂CH₃ | -O-CH₂CH₂CH₃ |
| 897 | -CH₂CH=CH-CH₃ | -O-CH₂CH₂CH₃ |
| 898 | -(CH₂)₂CH=CH₂ | -O-CH₂CH₂CH₃ |
| 899 | -CH=CH-(CH₂)₂CH₃ | -O-CH₂CH₂CH₃ |
| 900 | -CH₂CH=CH-CH₂CH₃ | -O-CH₂CH₂CH₃ |
| 901 | -(CH₂)₂CH=CH-CH₃ | -O-CH₂CH₂CH₃ |
| 902 | -(CH₂)₃CH=CH₂ | -O-CH₂CH₂CH₃ |
| 903 | -CH=CH-(CH₂)₃CH₃ | -O-CH₂CH₂CH₃ |
| 904 | -CH₂CH=CH-(CH₂)₂CH₃ | -O-CH₂CH₂CH₃ |
| 905 | -(CH₂)₂CH=CH-CH₂CH₃ | -O-CH₂CH₂CH₃ |
| 906 | -(CH₂)₃CH=CH-CH₃ | -O-CH₂CH₂CH₃ |
| 907 | -(CH₂)₄CH=CH₂ | -O-CH₂CH₂CH₃ |
| 908 | -CH=CH-(CH₂)₃CH₃ | -O-CH₂CH₂CH₃ |
| 909 | -CH₂CH=CH-(CH₂)₃CH₃ | -O-CH₂CH₂CH₃ |
| 910 | -(CH₂)₂CH=CH-(CH₂)₂CH₃ | -O-CH₂CH₂CH₃ |
| 911 | -(CH₂)₃CH=CH-CH₂CH₃ | -O-CH₂CH₂CH₃ |
| 912 | -(CH₂)₄CH=CH-CH₃ | -O-CH₂CH₂CH₃ |
| 913 | -(CH₂)₅CH=CH₂ | -O-CH₂CH₂CH₃ |
| 914 | -CH=CH-(CH₂)₅CH₃ | -O-CH₂CH₂CH₃ |
| 915 | -CH₂CH=CH-(CH₂)₄CH₃ | -O-CH₂CH₂CH₃ |
| 916 | -(CH₂)₂CH=CH-(CH₂)₃CH₃ | -O-CH₂CH₂CH₃ |
| 917 | -(CH₂)₃CH=CH-(CH₂)₂CH₃ | -O-CH₂CH₂CH₃ |
| 918 | -(CH₂)₄CH=CH-CH₂CH₃ | -O-CH₂CH₂CH₃ |
| 919 | -(CH₂)₅CH=CH-CH₃ | -O-CH₂CH₂CH₃ |
| 920 | -(CH₂)₆CH=CH₂ | -O-CH₂CH₂CH₃ |
| 921 | -CH₃ | -O-CH(CH₃)₂ |
| 922 | -CH₂CH₃ | -O-CH(CH₃)₂ |
| 923 | -CH₂CH₂CH₃ | -O-CH(CH₃)₂ |
| 924 | -CH(CH₃)₂ | -O-CH(CH₃)₂ |
| 925 | -CH₂CH₂CH₂CH₃ | -O-CH(CH₃)₂ |
| 926 | -CH(CH₃)CH₂CH₃ | -O-CH(CH₃)₂ |
| 927 | -CH₂CH(CH₃)₂ | -O-CH(CH₃)₂ |
| 928 | -C(CH₃)₃ | -O-CH(CH₃)₂ |
| 929 | -CH₂(CH₂)₃CH₃ | -O-CH(CH₃)₂ |
| 930 | -CH(CH₃)(CH₂)₂CH₃ | -O-CH(CH₃)₂ |
| 931 | -CH₂CH(CH₃)CH₂CH₃ | -O-CH(CH₃)₂ |
| 932 | -(CH₂)₂CH(CH₃)₂ | -O-CH(CH₃)₂ |
| 933 | -CH₂(CH₂)₄CH₃ | -O-CH(CH₃)₂ |
| 934 | -CH₂(CH₂)₅CH₃ | -O-CH(CH₃)₂ |
| 935 | -CH₂(CH₂)₆CH₃ | -O-CH(CH₃)₂ |
| 936 | -CH=CH₂ | -O-CH(CH₃)₂ |
| 937 | -CH=CHCH₃ | -O-CH(CH₃)₂ |
| 938 | -CH=C(CH₃)₂ | -O-CH(CH₃)₂ |
| 939 | -C(CH₃)=CH₂ | -O-CH(CH₃)₂ |
| 940 | -C(CH₃)=CHCH₃ | -O-CH(CH₃)₂ |
| 941 | -C(CH₃)=C(CH₃)₂ | -O-CH(CH₃)₂ |
| 942 | -CH=CH-CH₂CH₃ | -O-CH(CH₃)₂ |
| 943 | -CH₂CH=CH-CH₃ | -O-CH(CH₃)₂ |
| 944 | -(CH₂)₂CH=CH₂ | -O-CH(CH₃)₂ |
| 945 | -CH=CH-(CH₂)₂CH₃ | -O-CH(CH₃)₂ |
| 946 | -CH₂CH=CH-CH₂CH₃ | -O-CH(CH₃)₂ |
| 947 | -(CH₂)₂CH=CH-CH₃ | -O-CH(CH₃)₂ |
| 948 | -(CH₂)₃CH=CH₂ | -O-CH(CH₃)₂ |
| 949 | -CH=CH-(CH₂)₃CH₃ | -O-CH(CH₃)₂ |
| 950 | -CH₂CH=CH-(CH₂)₂CH₃ | -O-CH(CH₃)₂ |
| 951 | -(CH₂)₂CH=CH-CH₂CH₃ | -O-CH(CH₃)₂ |
| 952 | -(CH₂)₃CH=CH-CH₃ | -O-CH(CH₃)₂ |
| 953 | -(CH₂)₄CH=CH₂ | -O-CH(CH₃)₂ |
| 954 | -CH=CH-(CH₂)₃CH₃ | -O-CH(CH₃)₂ |
| 955 | -CH₂CH=CH-(CH₂)₃CH₃ | -O-CH(CH₃)₂ |
| 956 | -(CH₂)₂CH=CH-(CH₂)₂CH₃ | -O-CH(CH₃)₂ |
| 957 | -(CH₂)₃CH=CH-CH₂CH₃ | -O-CH(CH₃)₂ |
| 958 | -(CH₂)₄CH=CH-CH₃ | -O-CH(CH₃)₂ |
| 959 | -(CH₂)₅CH=CH₂ | -O-CH(CH₃)₂ |
| 960 | -CH=CH-(CH₂)₅CH₃ | -O-CH(CH₃)₂ |
| 961 | -CH₂CH=CH-(CH₂)₄CH₃ | -O-CH(CH₃)₂ |
| 962 | -(CH₂)₂CH=CH-(CH₂)₃CH₃ | -O-CH(CH₃)₂ |
| 963 | -(CH₂)₃CH=CH-(CH₂)₂CH₃ | -O-CH(CH₃)₂ |
| 964 | -(CH₂)₄CH=CH-CH₂CH₃ | -O-CH(CH₃)₂ |
| 965 | -(CH₂)₅CH=CH-CH₃ | -O-CH(CH₃)₂ |
| 966 | -(CH₂)₆CH=CH₂ | -O-CH(CH₃)₂ |

Als weiterhin besonders geeignet haben sich im Rahmen der vorliegenden Erfindung Carvonacetale der Formel 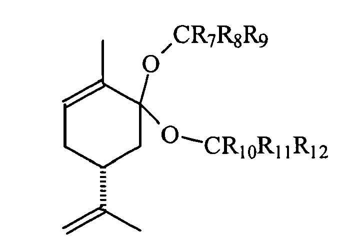 erwiesen, in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, - CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, - C(CH₃)₃

Einzelne Spezies, die der vorstehend genannten Formel genügen, sind hinsichtlich ihrer Reste R7 bis R12 in der nachstehenden Tabelle 3 aufgeführt. Diese sind erfindungsgemäß besonders bevorzugt.

**Tabelle 3: Bevorzugte Carvonacetale**

| **Nr.** | **R7** | **R8** | **R9** | **R10** | **R11** | **R12** |
|---|---|---|---|---|---|---|
| 1 | -H | -H | -H | -H | -H | -H |
| 2 | -CH₃ | -H | -H | -H | -H | -H |
| 3 | -CH₂CH₃ | -H | -H | -H | -H | -H |
| 4 | -CH₂CH₂CH₃ | -H | -H | -H | -H | -H |
| 5 | -CH(CH₃)₂ | -H | -H | -H | -H | -H |
| 6 | -CH₂CH₂CH₂CH₃ | -H | -H | -H | -H | -H |
| 7 | -CH(CH₃)CH₂CH₃ | -H | -H | -H | -H | -H |
| 8 | -CH₂CH(CH₃)₂ | -H | -H | -H | -H | -H |
| 9 | -C(CH₃)₃ | -H | -H | -H | -H | -H |
| 10 | -H | -CH₃ | -H | -H | -H | -H |
| 11 | -CH₃ | -CH₃ | -H | -H | -H | -H |
| 12 | -CH₂CH₃ | -CH₃ | -H | -H | -H | -H |
| 13 | -CH₂CH₂CH₃ | -CH₃ | -H | -H | -H | -H |
| 14 | -CH(CH₃)₂ | -CH₃ | -H | -H | -H | -H |
| 15 | -CH₂CH₂CH₂CH₃ | -CH₃ | -H | -H | -H | -H |
| 16 | -CH(CH₃)CH₂CH₃ | -CH₃ | -H | -H | -H | -H |
| 17 | -CH₂CH(CH₃)₂ | -CH₃ | -H | -H | -H | -H |
| 18 | -C(CH₃)₃ | -CH₃ | -H | -H | -H | -H |
| 19 | -H | -CH₃ | -CH₃ | -H | -H | -H |
| 20 | -CH₃ | -CH₃ | -CH₃ | -H | -H | -H |
| 21 | -CH₂CH₃ | -CH₃ | -CH₃ | -H | -H | -H |
| 22 | -CH₂CH₂CH₃ | -CH₃ | -CH₃ | -H | -H | -H |
| 23 | -CH(CH₃)₂ | -CH₃ | -CH₃ | -H | -H | -H |
| 24 | -CH₂CH₂CH₂CH₃ | -CH₃ | -CH₃ | -H | -H | -H |
| 25 | -CH(CH₃)CH₂CH₃ | -CH₃ | -CH₃ | -H | -H | -H |
| 26 | -CH₂CH(CH₃)₂ | -CH₃ | -CH₃ | -H | -H | -H |
| 27 | -C(CH₃)₃ | -CH₃ | -CH₃ | -H | -H | -H |
| 28 | -H | -CH₂CH₃ | -H | -H | -H | -H |
| 29 | -CH₃ | -CH₂CH₃ | -H | -H | -H | -H |
| 30 | -CH₂CH₃ | -CH₂CH₃ | -H | -H | -H | -H |
| 31 | -CH₂CH₂CH₃ | -CH₂CH₃ | -H | -H | -H | -H |
| 32 | -CH(CH₃)₂ | -CH₂CH₃ | -H | -H | -H | -H |
| 33 | -CH₂CH₂CH₂CH₃ | -CH₂CH₃ | -H | -H | -H | -H |
| 34 | -CH(CH₃)CH₂CH₃ | -CH₂CH₃ | -H | -H | -H | -H |
| 35 | -CH₂CH(CH₃)₂ | -CH₂CH₃ | -H | -H | -H | -H |
| 36 | -C(CH₃)₃ | -CH₂CH₃ | -H | -H | -H | -H |
| 37 | -H | -CH₂CH₃ | -CH₃ | -H | -H | -H |
| 38 | -CH₃ | -CH₂CH₃ | -CH₃ | -H | -H | -H |
| 39 | -CH₂CH₃ | -CH₂CH₃ | -CH₃ | -H | -H | -H |
| 40 | -CH₂CH₂CH₃ | -CH₂CH₃ | -CH₃ | -H | -H | -H |
| 41 | -CH(CH₃)₂ | -CH₂CH₃ | -CH₃ | -H | -H | -H |
| 42 | -CH₂CH₂CH₂CH₃ | -CH₂CH₃ | -CH₃ | -H | -H | -H |
| 43 | -CH(CH₃)CH₂CH₃ | -CH₂CH₃ | -CH₃ | -H | -H | -H |
| 44 | -CH₂CH(CH₃)₂ | -CH₂CH₃ | -CH₃ | -H | -H | -H |
| 45 | -C(CH₃)₃ | -CH₂CH₃ | -CH₃ | -H | -H | -H |
| 46 | -H | -CH₂CH₃ | -CH₂CH₃ | -H | -H | -H |
| 47 | -CH₃ | -CH₂CH₃ | -CH₂CH₃ | -H | -H | -H |
| 48 | -CH₂CH₃ | -CH₂CH₃ | -CH₂CH₃ | -H | -H | -H |
| 49 | -CH₂CH₂CH₃ | -CH₂CH₃ | -CH₂CH₃ | -H | -H | -H |
| 50 | -CH(CH₃)₂ | -CH₂CH₃ | -CH₂CH₃ | -H | -H | -H |
| 51 | -CH₂CH₂CH₂CH₃ | -CH₂CH₃ | -CH₂CH₃ | -H | -H | -H |
| 52 | -CH(CH₃)CH₂CH₃ | -CH₂CH₃ | -CH₂CH₃ | -H | -H | -H |
| 53 | -CH₂CH(CH₃)₂ | -CH₂CH₃ | -CH₂CH₃ | -H | -H | -H |
| 54 | -C(CH₃)₃ | -CH₂CH₃ | -CH₂CH₃ | -H | -H | -H |
| 55 | -H | -CH₂CH₂CH₃ | -H | -H | -H | -H |
| 56 | -CH₃ | -CH₂CH₂CH₃ | -H | -H | -H | -H |
| 57 | -CH₂CH₃ | -CH₂CH₂CH₃ | -H | -H | -H | -H |
| 58 | -CH₂CH₂CH₃ | -CH₂CH₂CH₃ | -H | -H | -H | -H |
| 59 | -CH(CH₃)₂ | -CH₂CH₂CH₃ | -H | -H | -H | -H |
| 60 | -CH₂CH₂CH₂CH₃ | -CH₂CH₂CH₃ | -H | -H | -H | -H |
| 61 | -CH(CH₃)CH₂CH₃ | -CH₂CH₂CH₃ | -H | -H | -H | -H |
| 62 | -CH₂CH(CH₃)₂ | -CH₂CH₂CH₃ | -H | -H | -H | -H |
| 63 | -C(CH₃)₃ | -CH₂CH₂CH₃ | -H | -H | -H | -H |
| 64 | -H | -CH₂CH₂CH₃ | -CH₃ | -H | -H | -H |
| 65 | -CH₃ | -CH₂CH₂CH₃ | -CH₃ | -H | -H | -H |
| 66 | -CH₂CH₃ | -CH₂CH₂CH₃ | -CH₃ | -H | -H | -H |
| 67 | -CH₂CH₂CH₃ | -CH₂CH₂CH₃ | -CH₃ | -H | -H | -H |
| 68 | -CH(CH₃)₂ | -CH₂CH₂CH₃ | -CH₃ | -H | -H | -H |
| 69 | -CH₂CH₂CH₂CH₃ | -CH₂CH₂CH₃ | -CH₃ | -H | -H | -H |
| 70 | -CH(CH₃)CH₂CH₃ | -CH₂CH₂CH₃ | -CH₃ | -H | -H | -H |
| 71 | -CH₂CH(CH₃)₂ | -CH₂CH₂CH₃ | -CH₃ | -H | -H | -H |
| 72 | -C(CH₃)₃ | -CH₂CH₂CH₃ | -CH₃ | -H | -H | -H |
| 73 | -H | -CH₂CH₂CH₃ | -CH₂CH₃ | -H | -H | -H |
| 74 | -CH₃ | -CH₂CH₂CH₃ | -CH₂CH₃ | -H | -H | -H |
| 75 | -CH₂CH₃ | -CH₂CH₂CH₃ | -CH₂CH₃ | -H | -H | -H |
| 76 | -CH₂CH₂CH₃ | -CH₂CH₂CH₃ | -CH₂CH₃ | -H | -H | -H |
| 77 | -CH(CH₃)₂ | -CH₂CH₂CH₃ | -CH₂CH₃ | -H | -H | -H |
| 78 | -CH₂CH₂CH₂CH₃ | -CH₂CH₂CH₃ | -CH₂CH₃ | -H | -H | -H |
| 79 | -CH(CH₃)CH₂CH₃ | -CH₂CH₂CH₃ | -CH₂CH₃ | -H | -H | -H |
| 80 | -CH₂CH(CH₃)₂ | -CH₂CH₂CH₃ | -CH₂CH₃ | -H | -H | -H |
| 81 | -C(CH₃)₃ | -CH₂CH₂CH₃ | -CH₂CH₃ | -H | -H | -H |
| 82 | -H | -CH₂CH₂CH₃ | -CH₂CH₂CH₃ | -H | -H | -H |
| 83 | -CH₃ | -CH₂CH₂CH₃ | -CH₂CH₂CH₃ | -H | -H | -H |
| 84 | -CH₂CH₃ | -CH₂CH₂CH₃ | -CH₂CH₂CH₃ | -H | -H | -H |
| 85 | -CH₂CH₂CH₃ | -CH₂CH₂CH₃ | -CH₂CH₂CH₃ | -H | -H | -H |
| 86 | -CH(CH₃)₂ | -CH₂CH₂CH₃ | -CH₂CH₂CH₃ | -H | -H | -H |
| 87 | -CH₂CH₂CH₂CH₃ | -CH₂CH₂CH₃ | -CH₂CH₂CH₃ | -H | -H | -H |
| 88 | -CH(CH₃)CH₂CH₃ | -CH₂CH₂CH₃ | -CH₂CH₂CH₃ | -H | -H | -H |
| 89 | -CH₂CH(CH₃)₂ | -CH₂CH₂CH₃ | -CH₂CH₂CH₃ | -H | -H | -H |
| 90 | -C(CH₃)₃ | -CH₂CH₂CH₃ | -CH₂CH₂CH₃ | -H | -H | -H |
| 91 | -H | -CH(CH₃)₂ | -H | -H | -H | -H |
| 92 | -CH₃ | -CH(CH₃)₂ | -H | -H | -H | -H |
| 93 | -CH₂CH₃ | -CH(CH₃)₂ | -H | -H | -H | -H |
| 94 | -CH₂CH₂CH₃ | -CH(CH₃)₂ | -H | -H | -H | -H |
| 95 | -CH(CH₃)₂ | -CH(CH₃)₂ | -H | -H | -H | -H |
| 96 | -CH₂CH₂CH₂CH₃ | -CH(CH₃)₂ | -H | -H | -H | -H |
| 97 | -CH(CH₃)CH₂CH₃ | -CH(CH₃)₂ | -H | -H | -H | -H |
| 98 | -CH₂CH(CH₃)₂ | -CH(CH₃)₂ | -H | -H | -H | -H |
| 99 | -C(CH₃)₃ | -CH(CH₃)₂ | -H | -H | -H | -H |
| 100 | -H | -CH(CH₃)₂ | -CH₃ | -H | -H | -H |
| 101 | -CH₃ | -CH(CH₃)₂ | -CH₃ | -H | -H | -H |
| 102 | -CH₂CH₃ | -CH(CH₃)₂ | -CH₃ | -H | -H | -H |
| 103 | -CH₂CH₂CH₃ | -CH(CH₃)₂ | -CH₃ | -H | -H | -H |
| 104 | -CH(CH₃)₂ | -CH(CH₃)₂ | -CH₃ | -H | -H | -H |
| 105 | -CH₂CH₂CH₂CH₃ | -CH(CH₃)₂ | -CH₃ | -H | -H | -H |
| 106 | -CH(CH₃)CH₂CH₃ | -CH(CH₃)₂ | -CH₃ | -H | -H | -H |
| 107 | -CH₂CH(CH₃)₂ | -CH(CH₃)₂ | -CH₃ | -H | -H | -H |
| 108 | -C(CH₃)₃ | -CH(CH₃)₂ | -CH₃ | -H | -H | -H |
| 109 | -H | -CH(CH₃)₂ | -CH₂CH₃ | -H | -H | -H |
| 110 | -CH₃ | -CH(CH₃)₂ | -CH₂CH₃ | -H | -H | -H |
| 111 | -CH₂CH₃ | -CH(CH₃)₂ | -CH₂CH₃ | -H | -H | -H |
| 112 | -CH₂CH₂CH₃ | -CH(CH₃)₂ | -CH₂CH₃ | -H | -H | -H |
| 113 | -CH(CH₃)₂ | -CH(CH₃)₂ | -CH₂CH₃ | -H | -H | -H |
| 114 | -CH₂CH₂CH₂CH₃ | -CH(CH₃)₂ | -CH₂CH₃ | -H | -H | -H |
| 115 | -CH(CH₃)CH₂CH₃ | -CH(CH₃)₂ | -CH₂CH₃ | -H | -H | -H |
| 116 | -CH_{2C}H(CH₃)₂ | -CH(CH₃)₂ | -CH₂CH₃ | -H | -H | -H |
| 117 | -C(CH₃)₃ | -CH(CH₃)₂ | -CH₂CH₃ | -H | -H | -H |
| 118 | -H | -CH(CH₃)₂ | -CH₂CH₂CH₃ | -H | -H | -H |
| 119 | -CH₃ | -CH(CH₃)₂ | -CH₂CH₂CH₃ | -H | -H | -H |
| 120 | -CH₂CH₃ | -CH(CH₃)₂ | -CH₂CH₂CH₃ | -H | -H | -H |
| 121 | -CH₂CH₂CH₃ | -CH(CH₃)₂ | -CH₂CH₂CH₃ | -H | -H | -H |
| 122 | -CH(CH₃)₂ | -CH(CH₃)₂ | -CH₂CH₂CH₃ | -H | -H | -H |
| 123 | -CH₂CH₂CH₂CH₃ | -CH(CH₃)₂ | -CH₂CH₂CH₃ | -H | -H | -H |
| 124 | -CH(CH₃)CH₂CH₃ | -CH(CH₃)₂ | -CH₂CH₂CH₃ | -H | -H | -H |
| 125 | -CH₂CH(CH₃)₂ | -CH(CH₃)₂ | -CH₂CH₂CH₃ | -H | -H | -H |
| 126 | -C(CH₃)₃ | -CH(CH₃)₂ | -CH₂CH₂CH₃ | -H | -H | -H |
| 127 | -H | -CH(CH₃)₂ | -CH(CH₃)₂ | -H | -H | -H |
| 128 | -CH₃ | -CH(CH₃)₂ | -CH(CH₃)₂ | -H | -H | -H |
| 129 | -CH₂CH₃ | -CH(CH₃)₂ | -CH(CH₃)₂ | -H | -H | -H |
| 130 | -CH₂CH₂CH₃ | -CH(CH₃)₂ | -CH(CH₃)₂ | -H | -H | -H |
| 131 | -CH(CH₃)₂ | -CH(CH₃)₂ | -CH(CH₃)₂ | -H | -H | -H |
| 132 | -CH₂CH₂CH₂CH₃ | -CH(CH₃)₂ | -CH(CH₃)₂ | -H | -H | -H |
| 133 | -CH(CH₃)CH₂CH₃ | -CH(CH₃)₂ | -CH(CH₃)₂ | -H | -H | -H |
| 134 | -CH₂CH(CH₃)₂ | -CH(CH₃)₂ | -CH(CH₃)₂ | -H | -H | -H |
| 135 | -C(CH₃)₃ | -CH(CH₃)₂ | -CH(CH₃)₂ | -H | -H | -H |
| | | | | | | |

| **Nr.** | **R10** | **R11** | **R12** | **R7** | **R8** | **R9** |
|---|---|---|---|---|---|---|
| 136 | -H | -H | -H | -H | -H | -H |
| 137 | -CH₃ | -H | -H | -H | -H | -H |
| 138 | -CH₂CH₃ | -H | -H | -H | -H | -H |
| 139 | -CH₂CH₂CH₃ | -H | -H | -H | -H | -H |
| 140 | -CH(CH₃)₂ | -H | -H | -H | -H | -H |
| 141 | -CH₂CH₂CH₂CH₃ | -H | -H | -H | -H | -H |
| 142 | -CH(CH₃)CH₂CH₃ | -H | -H | -H | -H | -H |
| 143 | -CH₂CH(CH₃)₂ | -H | -H | -H | -H | -H |
| 144 | -C(CH₃)₃ | -H | -H | -H | -H | -H |
| 145 | -H | -CH₃ | -H | -H | -H | -H |
| 146 | -CH₃ | -CH₃ | -H | -H | -H | -H |
| 147 | -CH₂CH₃ | -CH₃ | -H | -H | -H | -H |
| 148 | -CH₂CH₂CH₃ | -CH₃ | -H | -H | -H | -H |
| 149 | -CH(CH₃)₂ | -CH₃ | -H | -H | -H | -H |
| 150 | -CH₂CH₂CH₂CH₃ | -CH₃ | -H | -H | -H | -H |
| 151 | -CH(CH₃)CH₂CH₃ | -CH₃ | -H | -H | -H | -H |
| 152 | -CH₂CH(CH₃)₂ | -CH₃ | -H | -H | -H | -H |
| 153 | -C(CH₃)₃ | -CH₃ | -H | -H | -H | -H |
| 154 | -H | -CH₃ | -CH₃ | -H | -H | -H |
| 155 | -CH₃ | -CH₃ | -CH₃ | -H | -H | -H |
| 156 | -CH₂CH₃ | -CH₃ | -CH₃ | -H | -H | -H |
| 157 | -CH₂CH₂CH₃ | -CH₃ | -CH₃ | -H | -H | -H |
| 158 | -CH(CH₃)₂ | -CH₃ | -CH₃ | -H | -H | -H |
| 159 | -CH₂CH₂CH₂CH₃ | -CH₃ | -CH₃ | -H | -H | -H |
| 160 | -CH(CH₃)CH₂CH₃ | -CH₃ | -CH₃ | -H | -H | -H |
| 161 | -CH₂CH(CH₃)₂ | -CH₃ | -CH₃ | -H | -H | -H |
| 162 | -C(CH₃)₃ | -CH₃ | -CH₃ | -H | -H | -H |
| 163 | -H | -CH₂CH₃ | -H | -H | -H | -H |
| 164 | -CH₃ | -CH₂CH₃ | -H | -H | -H | -H |
| 165 | -CH₂CH₃ | -CH₂CH₃ | -H | -H | -H | -H |
| 166 | -CH₂CH₂CH₃ | -CH₂CH₃ | -H | -H | -H | -H |
| 167 | -CH(CH₃)₂ | -CH₂CH₃ | -H | -H | -H | -H |
| 168 | -CH₂CH₂CH₂CH₃ | -CH₂CH₃ | -H | -H | -H | -H |
| 169 | -CH(CH₃)CH₂CH₃ | -CH₂CH₃ | -H | -H | -H | -H |
| 170 | -CH₂CH(CH₃)₂ | -CH₂CH₃ | -H | -H | -H | -H |
| 171 | -C(CH₃)₃ | -CH₂CH₃ | -H | -H | -H | -H |
| 172 | -H | -CH₂CH₃ | -CH₃ | -H | -H | -H |
| 173 | -CH₃ | -CH₂CH₃ | -CH₃ | -H | -H | -H |
| 174 | -CH₂CH₃ | -CH₂CH₃ | -CH₃ | -H | -H | -H |
| 175 | -CH₂CH₂CH₃ | -CH₂CH₃ | -CH₃ | -H | -H | -H |
| 176 | -CH(CH₃)₂ | -CH₂CH₃ | -CH₃ | -H | -H | -H |
| 177 | -CH₂CH₂CH₂CH₃ | -CH₂CH₃ | -CH₃ | -H | -H | -H |
| 178 | -CH(CH₃)CH₂CH₃ | -CH₂CH₃ | -CH₃ | -H | -H | -H |
| 179 | -CH₂CH(CH₃)₂ | -CH₂CH₃ | -CH₃ | -H | -H | -H |
| 180 | -C(CH₃)₃ | -CH₂CH₃ | -CH₃ | -H | -H | -H |
| 181 | -H | -CH₂CH₃ | -CH₂CH₃ | -H | -H | -H |
| 182 | -CH₃ | -CH₂CH₃ | -CH₂CH₃ | -H | -H | -H |
| 183 | -CH₂CH₃ | -CH₂CH₃ | -CH₂CH₃ | -H | -H | -H |
| 184 | -CH₂CH₂CH₃ | -CH₂CH₃ | -CH₂CH₃ | -H | -H | -H |
| 185 | -CH(CH₃)₂ | -CH₂CH₃ | -CH₂CH₃ | -H | -H | -H |
| 186 | -CH₂CH₂CH₂CH₃ | -CH₂CH₃ | -CH₂CH₃ | -H | -H | -H |
| 187 | -CH(CH₃)CH₂CH₃ | -CH₂CH₃ | -CH₂CH₃ | -H | -H | -H |
| 188 | -CH₂CH(CH₃)₂ | -CH₂CH₃ | -CH₂CH₃ | -H | -H | -H |
| 189 | -C(CH₃)₃ | -CH₂CH₃ | -CH₂CH₃ | -H | -H | -H |
| 190 | -H | -CH₂CH₂CH₃ | -H | -H | -H | -H |
| 191 | -CH₃ | -CH₂CH₂CH₃ | -H | -H | -H | -H |
| 192 | -CH₂CH₃ | -CH₂CH₂CH₃ | -H | -H | -H | -H |
| 193 | -CH₂CH₂CH₃ | -CH₂CH₂CH₃ | -H | -H | -H | -H |
| 194 | -CH(CH₃)₂ | -CH₂CH₂CH₃ | -H | -H | -H | -H |
| 195 | -CH₂CH₂CH₂CH₃ | -CH₂CH₂CH₃ | -H | -H | -H | -H |
| 196 | -CH(CH₃)CH₂CH₃ | -CH₂CH₂CH₃ | -H | -H | -H | -H |
| 197 | -CH₂CH(CH₃)₂ | -CH₂CH₂CH₃ | -H | -H | -H | -H |
| 198 | -C(CH₃)₃ | -CH₂CH₂CH₃ | -H | -H | -H | -H |
| 199 | -H | -CH₂CH₂CH₃ | -CH₃ | -H | -H | -H |
| 200 | -CH₃ | -CH₂CH₂CH₃ | -CH₃ | -H | -H | -H |
| 201 | -CH₂CH₃ | -CH₂CH₂CH₃ | -CH₃ | -H | -H | -H |
| 202 | -CH₂CH₂CH₃ | -CH₂CH₂CH₃ | -CH₃ | -H | -H | -H |
| 203 | -CH(CH₃)₂ | -CH₂CH₂CH₃ | -CH₃ | -H | -H | -H |
| 204 | -CH₂CH₂CH₂CH₃ | -CH₂CH₂CH₃ | -CH₃ | -H | -H | -H |
| 205 | -CH(CH₃)CH₂CH₃ | -CH₂CH₂CH₃ | -CH₃ | -H | -H | -H |
| 206 | -CH₂CH(CH₃)₂ | -CH₂CH₂CH₃ | -CH₃ | -H | -H | -H |
| 207 | -C(CH₃)₃ | -CH₂CH₂CH₃ | -CH₃ | -H | -H | -H |
| 208 | -H | -CH₂CH₂CH₃ | -CH₂CH₃ | -H | -H | -H |
| 209 | -CH₃ | -CH₂CH₂CH₃ | -CH₂CH₃ | -H | -H | -H |
| 210 | -CH₂CH₃ | -CH₂CH₂CH₃ | -CH₂CH₃ | -H | -H | -H |
| 211 | -CH₂CH₂CH₃ | -CH₂CH₂CH₃ | -CH₂CH₃ | -H | -H | -H |
| 212 | -CH(CH₃)₂ | -CH₂CH₂CH₃ | -CH₂CH₃ | -H | -H | -H |
| 213 | -CH₂CH₂CH₂CH₃ | -CH₂CH₂CH₃ | -CH₂CH₃ | -H | -H | -H |
| 214 | -CH(CH₃)CH₂CH₃ | -CH₂CH₂CH₃ | -CH₂CH₃ | -H | -H | -H |
| 215 | -CH₂CH(CH₃)₂ | -CH₂CH₂CH₃ | -CH₂CH₃ | -H | -H | -H |
| 216 | -C(CH₃)₃ | -CH₂CH₂CH₃ | -CH₂CH₃ | -H | -H | -H |
| 217 | -H | -CH₂CH₂CH₃ | -CH₂CH₂CH₃ | -H | -H | -H |
| 218 | -CH₃ | -CH₂CH₂CH₃ | -CH₂CH₂CH₃ | -H | -H | -H |
| 219 | -CH₂CH₃ | -CH₂CH₂CH₃ | -CH₂CH₂CH₃ | -H | -H | -H |
| 220 | -CH₂CH₂CH₃ | -CH₂CH₂CH₃ | -CH₂CH₂CH₃ | -H | -H | -H |
| 221 | -CH(CH₃)₂ | -CH₂CH₂CH₃ | -CH₂CH₂CH₃ | -H | -H | -H |
| 222 | -CH₂CH₂CH₂CH₃ | -CH₂CH₂CH₃ | -CH₂CH₂CH₃ | -H | -H | -H |
| 223 | -CH(CH₃)CH₂CH₃ | -CH₂CH₂CH₃ | -CH₂CH₂CH₃ | -H | -H | -H |
| 224 | -CH₂CH(CH₃)₂ | -CH₂CH₂CH₃ | -CH₂CH₂CH₃ | -H | -H | -H |
| 225 | -C(CH₃)₃ | -CH₂CH₂CH₃ | -CH₂CH₂CH₃ | -H | -H | -H |
| 226 | -H | -CH(CH₃)₂ | -H | -H | -H | -H |
| 227 | -CH₃ | -CH(CH₃)₂ | -H | -H | -H | -H |
| 228 | -CH₂CH₃ | -CH(CH₃)₂ | -H | -H | -H | -H |
| 229 | -CH₂CH₂CH₃ | -CH(CH₃)₂ | -H | -H | -H | -H |
| 230 | -CH(CH₃)₂ | -CH(CH₃)₂ | -H | -H | -H | -H |
| 231 | -CH₂CH₂CH₂CH₃ | -CH(CH₃)₂ | -H | -H | -H | -H |
| 232 | -CH(CH₃)CH₂CH₃ | -CH(CH₃)₂ | -H | -H | -H | -H |
| 233 | -CH₂CH(CH₃)₂ | -CH(CH₃)₂ | -H | -H | -H | -H |
| 234 | -C(CH₃)₃ | -CH(CH₃)₂ | -H | -H | -H | -H |
| 235 | -H | -CH(CH₃)₂ | -CH₃ | -H | -H | -H |
| 236 | -CH₃ | -CH(CH₃)₂ | -CH₃ | -H | -H | -H |
| 237 | -CH₂CH₃ | -CH(CH₃)₂ | -CH₃ | -H | -H | -H |
| 238 | -CH₂CH₂CH₃ | -CH(CH₃)₂ | -CH₃ | -H | -H | -H |
| 239 | -CH(CH₃)₂ | -CH(CH₃)₂ | -CH₃ | -H | -H | -H |
| 240 | -CH₂CH₂CH₂CH₃ | -CH(CH₃)₂ | -CH₃ | -H | -H | -H |
| 241 | -CH(CH₃)CH₂CH₃ | -CH(CH₃)₂ | -CH₃ | -H | -H | -H |
| 242 | -CH₂CH(CH₃)₂ | -CH(CH₃)₂ | -CH₃ | -H | -H | -H |
| 243 | -C(CH₃)₃ | -CH(CH₃)₂ | -CH₃ | -H | -H | -H |
| 244 | -H | -CH(CH₃)₂ | -CH₂CH₃ | -H | -H | -H |
| 245 | -CH₃ | -CH(CH₃)₂ | -CH₂CH₃ | -H | -H | -H |
| 246 | -CH₂CH₃ | -CH(CH₃)₂ | -CH₂CH₃ | -H | -H | -H |
| 247 | -CH₂CH₂CH₃ | -CH(CH₃)₂ | -CH₂CH₃ | -H | -H | -H |
| 248 | -CH(CH₃)₂ | -CH(CH₃)₂ | -CH₂CH₃ | -H | -H | -H |
| 249 | -CH₂CH₂CH₂CH₃ | -CH(CH₃)₂ | -CH₂CH₃ | -H | -H | -H |
| 250 | -CH(CH₃)CH₂CH₃ | -CH(CH₃)₂ | -CH₂CH₃ | -H | -H | -H |
| 251 | -CH₂CH(CH₃)₂ | -CH(CH₃)₂ | -CH₂CH₃ | -H | -H | -H |
| 252 | -C(CH₃)₃ | -CH(CH₃)₂ | -CH₂CH₃ | -H | -H | -H |
| 253 | -H | -CH(CH₃)₂ | -CH₂CH₂CH₃ | -H | -H | -H |
| 254 | -CH₃ | -CH(CH₃)₂ | -CH₂CH₂CH₃ | -H | -H | -H |
| 255 | -CH₂CH₃ | -CH(CH₃)₂ | -CH₂CH₂CH₃ | -H | -H | -H |
| 256 | -CH₂CH₂CH₃ | -CH(CH₃)₂ | -CH₂CH₂CH₃ | -H | -H | -H |
| 257 | -CH(CH₃)₂ | -CH(CH₃)₂ | -CH₂CH₂CH₃ | -H | -H | -H |
| 258 | -CH₂CH₂CH₂CH₃ | -CH(CH₃)₂ | -CH₂CH₂CH₃ | -H | -H | -H |
| 259 | -CH(CH₃)CH₂CH₃ | -CH(CH₃)₂ | -CH₂CH₂CH₃ | -H | -H | -H |
| 260 | -CH₂CH(CH₃)₂ | -CH(CH₃)₂ | -CH₂CH₂CH₃ | -H | -H | -H |
| 261 | -C(CH₃)₃ | -CH(CH₃)₂ | -CH₂CH₂CH₃ | -H | -H | -H |
| 262 | -H | -CH(CH₃)₂ | -CH(CH₃)₂ | -H | -H | -H |
| 263 | -CH₃ | -CH(CH₃)₂ | -CH(CH₃)₂ | -H | -H | -H |
| 264 | -CH₂CH₃ | -CH(CH₃)₂ | -CH(CH₃)₂ | -H | -H | -H |
| 265 | -CH₂CH₂CH₃ | -CH(CH₃)₂ | -CH(CH₃)₂ | -H | -H | -H |
| 266 | -CH(CH₃)₂ | -CH(CH₃)₂ | -CH(CH₃)₂ | -H | -H | -H |
| 267 | -CH₂CH₂CH₂CH₃ | -CH(CH₃)₂ | -CH(CH₃)₂ | -H | -H | -H |
| 268 | -CH(CH₃)CH₂CH₃ | -CH(CH₃)₂ | -CH(CH₃)₂ | -H | -H | -H |
| 269 | -CH₂CH(CH₃)₂ | -CH(CH₃)₂ | -CH(CH₃)₂ | -H | -H | -H |
| 270 | -C(CH₃)₃ | -CH(CH₃)₂ | -CH(CH₃)₂ | -H | -H | -H |
| | | | | | | |

| **Nr.** | **R7** | **R8** | **R9** | **R10** | **R11** | **R12** |
|---|---|---|---|---|---|---|
| 271 | -H | -H | -H | -H | -H | -H |
| 272 | -CH₃ | -H | -H | -CH₃ | -H | -H |
| 273 | -CH₂CH₃ | -H | -H | -CH₂CH₃ | -H | -H |
| 274 | -CH₂CH₂CH₃ | -H | -H | -CH₂CH₂CH₃ | -H | -H |
| 275 | -CH(CH₃)₂ | -H | -H | -CH(CH₃)₂ | -H | -H |
| 276 | -CH₂CH₂CH₂CH₃ | -H | -H | -CH₂CH₂CH₂CH₃ | -H | -H |
| 277 | -CH(CH₃)CH₂CH₃ | -H | -H | -CH(CH₃)CH₂CH₃ | -H | -H |
| 278 | -CH₂CH(CH₃)₂ | -H | -H | -CH₂CH(CH₃)₂ | -H | -H |
| 279 | -C(CH₃)₃ | -H | -H | -C(CH₃)₃ | -H | -H |
| 280 | -H | -CH₃ | -H | -H | -CH₃ | -H |
| 281 | -CH₃ | -CH₃ | -H | -CH₃ | -CH₃ | -H |
| 282 | -CH₂CH₃ | -CH₃ | -H | -CH₂CH₃ | -CH₃ | -H |
| 283 | -CH₂CH₂CH₃ | -CH₃ | -H | -CH₂CH₂CH₃ | -CH₃ | -H |
| 284 | -CH(CH₃)₂ | -CH₃ | -H | -CH(CH₃)₂ | -CH₃ | -H |
| 285 | -CH₂CH₂CH₂CH₃ | -CH₃ | -H | -CH₂CH₂CH₂CH₃ | -CH₃ | -H |
| 286 | -H(CH₃)CH₂CH₃ | -CH₃ | -H | -CH(CH₃)CH₂CH₃ | -CH₃ | -H |
| 287 | -CH₂CH(CH₃)₂ | -CH₃ | -H | -CH₂CH(CH₃)₂ | -CH₃ | -H |
| 288 | -C(CH₃)₃ | -CH₃ | -H | -C(CH₃)₃ | -CH₃ | -H |
| 289 | -H | -CH₃ | -CH₃ | -H | -CH₃ | -CH₃ |
| 290 | -CH₃ | -CH₃ | -CH₃ | -CH₃ | -CH₃ | -CH₃ |
| 291 | -CH₂CH₃ | -CH₃ | -CH₃ | -CH₂CH₃ | -CH₃ | -CH₃ |
| 292 | -CH₂CH₂CH₃ | -CH₃ | -CH₃ | -CH₂CH₂CH₃ | -CH₃ | -CH₃ |
| 293 | -CH(CH₃)₂ | -CH₃ | -CH₃ | -CH(CH₃)₂ | -CH₃ | -CH₃ |
| 294 | -CH₂CH₂CH₂CH₃ | -CH₃ | -CH₃ | -CH₂CH₂CH₂CH₃ | -CH₃ | -CH₃ |
| 295 | -H(CH₃)CH₂CH₃ | -CH₃ | -CH₃ | -CH(CH₃)CH₂CH₃ | -CH₃ | -CH₃ |
| 296 | -CH₂CH(CH₃)₂ | -CH₃ | -CH₃ | -CH₂CH(CH₃)₂ | -CH₃ | -CH₃ |
| 297 | -C(CH₃)₃ | -CH₃ | -CH₃ | -C(CH₃)₃ | -CH₃ | -CH₃ |
| 298 | -H | -CH₂CH₃ | -H | -H | -CH₂CH₃ | -H |
| 299 | -CH₃ | -CH₂CH₃ | -H | -CH₃ | -CH₂CH₃ | -H |
| 300 | -CH₂CH₃ | -CH₂CH₃ | -H | -CH₂CH₃ | -CH₂CH₃ | -H |
| 301 | -CH₂CH₂CH₃ | -CH₂CH₃ | -H | -CH₂CH₂CH₃ | -CH₂CH₃ | -H |
| 302 | -CH(CH₃)₂ | -CH₂CH₃ | -H | -CH(CH₃)₂ | -CH₂CH₃ | -H |
| 303 | -CH₂CH₂CH₂CH₃ | -CH₂CH₃ | -H | -CH₂CH₂CH₂CH₃ | -CH₂CH₃ | -H |
| 304 | -H(CH₃)CH₂CH₃ | -CH₂CH₃ | -H | -CH(CH₃)CH₂CH₃ | -CH₂CH₃ | -H |
| 305 | -CH₂CH(CH₃)₂ | -CH₂CH₃ | -H | -CH₂CH(CH₃)₂ | -CH₂CH₃ | -H |
| 306 | -C(CH₃)₃ | -CH₂CH₃ | -H | -C(CH₃)₃ | -CH₂CH₃ | -H |
| 307 | -H | -CH₂CH₃ | -CH₃ | -H | -CH₂CH₃ | -CH₃ |
| 308 | -CH₃ | -CH₂CH₃ | -CH₃ | -CH₃ | -CH₂CH₃ | -CH₃ |
| 309 | -CH₂CH₃ | -CH₂CH₃ | -CH₃ | -CH₂CH₃ | -CH₂CH₃ | -CH₃ |
| 310 | -CH₂CH₂CH₃ | -CH₂CH₃ | -CH₃ | -CH₂CH₂CH₃ | -CH₂CH₃ | -CH₃ |
| 311 | -CH(CH₃)₂ | -CH₂CH₃ | -CH₃ | -CH(CH₃)₂ | -CH₂CH₃ | -CH₃ |
| 312 | -CH₂CH₂CH₂CH₃ | -CH₂CH₃ | -CH₃ | -CH₂CH₂CH₂CH₃ | -CH₂CH₃ | -CH₃ |
| 313 | -H(CH₃)CH₂CH₃ | -CH₂CH₃ | -CH₃ | -CH(CH₃)CH₂CH₃ | -CH₂CH₃ | -CH₃ |
| 314 | -CH₂CH(CH₃)₂ | -CH₂CH₃ | -CH₃ | -CH₂CH(CH₃)₂ | -CH₂CH₃ | -CH₃ |
| 315 | -C(CH₃)₃ | -CH₂CH₃ | -CH₃ | -C(CH₃)₃ | -CH₂CH₃ | -CH₃ |
| 316 | -H | -CH₂CH₃ | -CH₂CH₃ | -H | -CH₂CH₃ | -CH₂CH₃ |
| 317 | -CH₃ | -CH₂CH₃ | -CH₂CH₃ | -CH₃ | -CH₂CH₃ | -CH₂CH₃ |
| 318 | -CH₂CH₃ | -CH₂CH₃ | -CH₂CH₃ | -CH₂CH₃ | -CH₂CH₃ | -CH₂CH₃ |
| 319 | -CH₂CH₂CH₃ | -CH₂CH₃ | -CH₂CH₃ | -CH₂CH₂CH₃ | -CH₂CH₃ | -CH₂CH₃ |
| 320 | -CH(CH₃)₂ | -CH₂CH₃ | -CH₂CH₃ | -CH(CH₃)₂ | -CH₂CH₃ | -CH₂CH₃ |
| 321 | -CH₂CH₂CH₂CH₃ | -CH₂CH₃ | -CH₂CH₃ | -CH₂CH₂CH₂CH₃ | -CH₂CH₃ | -CH₂CH₃ |
| 322 | CH(CH₃)CH₂CH₃ | -CH₂CH₃ | -CH₂CH₃ | -CH(CH₃)CH₂CH₃ | -CH₂CH₃ | -CH₂CH₃ |
| 323 | -CH₂CH(CH₃)₂ | -CH₂CH₃ | -CH₂CH₃ | -CH₂CH(CH₃)₂ | -CH₂CH₃ | -CH₂CH₃ |
| 324 | -C(CH₃)₃ | -CH₂CH₃ | -CH₂CH₃ | -C(CH₃)₃ | -CH₂CH₃ | -CH₂CH₃ |
| 325 | -H | -CH₂CH₂CH₃ | -H | -H | -CH₂CH₂CH₃ | -H |
| 326 | -CH₃ | -CH₂CH₂CH₃ | -H | -CH₃ | -CH₂CH₂CH₃ | -H |
| 327 | -CH₂CH₃ | -CH₂CH₂CH₃ | -H | -CH₂CH₃ | -CH₂CH₂CH₃ | -H |
| 328 | -CH₂CH₂CH₃ | -CH₂CH₂CH₃ | -H | -CH₂CH₂CH₃ | -CH₂CH₂CH₃ | -H |
| 329 | -CH(CH₃)₂ | -CH₂CH₂CH₃ | -H | -CH(CH₃)₂ | -CH₂CH₂CH₃ | -H |
| 330 | -CH₂CH₂CH₂CH₃ | -CH₂CH₂CH₃ | -H | -CH₂CH₂CH₂CH₃ | -CH₂CH₂CH₃ | -H |
| 331 | -H(CH₃)CH₂CH₃ | -CH₂CH₂CH₃ | -H | -CH(CH₃)CH₂CH₃ | -CH₂CH₂CH₃ | -H |
| 332 | -CH₂CH(CH₃)₂ | -CH₂CH₂CH₃ | -H | -CH₂CH(CH₃)₂ | -CH₂CH₂CH₃ | -H |
| 333 | -C(CH₃)₃ | -CH₂CH₂CH₃ | -H | -C(CH₃)₃ | -CH₂CH₂CH₃ | -H |
| 334 | -H | -CH₂CH₂CH₃ | -CH₃ | -H | -CH₂CH₂CH₃ | -CH₃ |
| 335 | -CH₃ | -CH₂CH₂CH₃ | -CH₃ | -CH₃ | -CH₂CH₂CH₃ | -CH₃ |
| 336 | -CH₂CH₃ | -CH₂CH₂CH₃ | -CH₃ | -CH₂CH₃ | -CH₂CH₂CH₃ | -CH₃ |
| 337 | -CH₂CH₂CH₃ | -CH₂CH₂CH₃ | -CH₃ | -CH₂CH₂CH₃ | -CH₂CH₂CH₃ | -CH₃ |
| 338 | -CH(CH₃)₂ | -CH₂CH₂CH₃ | -CH₃ | -CH(CH₃)₂ | -CH₂CH₂CH₃ | -CH₃ |
| 339 | -CH₂CH₂CH₂CH₃ | -CH₂CH₂CH₃ | -CH₃ | -CH₂CH₂CH₂CH₃ | -CH₂CH₂CH₃ | -CH₃ |
| 340 | -H(CH₃)CH₂CH₃ | -CH₂CH₂CH₃ | -CH₃ | -CH(CH₃)CH₂CH₃ | -CH₂CH₂CH₃ | -CH₃ |
| 341 | -CH₂CH(CH₃)₂ | -CH₂CH₂CH₃ | -CH₃ | -CH₂CH(CH₃)₂ | -CH₂CH₂CH₃ | -CH₃ |
| 342 | -C(CH₃)₃ | -CH₂CH₂CH₃ | -CH₃ | -C(CH₃)₃ | -CH₂CH₂CH₃ | -CH₃ |
| 343 | -H | -CH₂CH₂CH₃ | -CH₂CH₃ | -H | -CH₂CH₂CH₃ | -CH₂CH₃ |
| 344 | -CH₃ | -CH₂CH₂CH₃ | -CH₂CH₃ | -CH₃ | -CH₂CH₂CH₃ | -CH₂CH₃ |
| 345 | -CH₂CH₃ | -CH₂CH₂CH₃ | -CH₂CH₃ | -CH₂CH₃ | -CH₂CH₂CH₃ | -CH₂CH₃ |
| 346 | -CH₂CH₂CH₃ | -CH₂CH₂CH₃ | -CH₂CH₃ | -CH₂CH₂CH₃ | -CH₂CH₂CH₃ | -CH₂CH₃ |
| 347 | -CH(CH₃)₂ | -CH₂CH₂CH₃ | -CH₂CH₃ | -CH(CH₃)₂ | -CH₂CH₂CH₃ | -CH₂CH₃ |
| 348 | -CH₂CH₂CH₂CH₃ | -CH₂CH₂CH₃ | -CH₂CH₃ | -CH₂CH₂CH₂CH₃ | -CH₂CH₂CH₃ | -CH₂CH₃ |
| 349 | -H(CH₃)CH₂CH₃ | -CH₂CH₂CH₃ | -CH₂CH₃ | -CH(CH₃)CH₂CH₃ | -CH₂CH₂CH₃ | -CH₂CH₃ |
| 350 | -CH₂CH(CH₃)₂ | -CH₂CH₂CH₃ | -CH₂CH₃ | -CH₂CH(CH₃)₂ | -CH₂CH₂CH₃ | -CH₂CH₃ |
| 351 | -C(CH₃)₃ | -CH₂CH₂CH₃ | -CH₂CH₃ | -C(CH₃)₃ | -CH₂CH₂CH₃ | -CH₂CH₃ |
| 352 | -H | -CH₂CH₂CH₃ | -CH₂CH₂CH₃ | -H | -CH₂CH₂CH₃ | -CH₂CH₂CH₃ |
| 353 | -CH₃ | -CH₂CH₂CH₃ | -CH₂CH₂CH₃ | -CH₃ | -CH₂CH₂CH₃ | -CH₂CH₂CH₃ |
| 354 | -CH₂CH₃ | -CH₂CH₂CH₃ | -CH₂CH₂CH₃ | -CH₂CH₃ | -CH₂CH₂CH₃ | -CH₂CH₂CH₃ |
| 355 | -CH₂CH₂CH₃ | -CH₂CH₂CH₃ | -CH₂CH₂CH₃ | -CH₂CH₂CH₃ | -CH₂CH₂CH₃ | -CH₂CH₂CH₃ |
| 356 | -CH(CH₃)₂ | -CH₂CH₂CH₃ | -CH₂CH₂CH₃ | -CH(CH₃)₂ | -CH₂CH₂CH₃ | -CH₂CH₂CH₃ |
| 357 | -CH₂CH₂CH₂CH₃ | -CH₂CH₂CH₃ | -CH₂CH₂CH₃ | -CH₂CH₂CH₂CH₃ | -CH₂CH₂CH₃ | -CH₂CH₂CH₃ |
| 358 | -CH(CH₃)CH₂CH₃ | -CH₂CH₂CH₃ | -CH₂CH₂CH₃ - | CH(CH₃)CH₂CH₃ | -CH₂CH₂CH₃ | -CH₂CH₂CH₃ |
| 359 | -CH₂CH(CH₃)₂ | -CH₂CH₂CH₃ | -CH₂CH₂CH₃ | -CH₂CH(CH₃)₂ | -CH₂CH₂CH₃ | -CH₂CH₂CH₃ |
| 360 | -C(CH₃)₃ | -CH₂CH₂CH₃ | -CH₂CH₂CH₃ | -C(CH₃)₃ | -CH₂CH₂CH₃ | -CH₂CH₂CH₃ |
| 361 | -H | -CH(CH₃)₂ | -H | -H | -CH(CH₃)₂ | -H |
| 362 | -CH₃ | -CH(CH₃)₂ | -H | -CH₃ | -CH(CH₃)₂ | -H |
| 363 | -CH₂CH₃ | -CH(CH₃)₂ | -H | -CH₂CH₃ | -CH(CH₃)₂ | -H |
| 364 | -CH₂CH₂CH₃ | -CH(CH₃)₂ | -H | -CH₂CH₂CH₃ | -CH(CH₃)₂ | -H |
| 365 | -CH(CH₃)₂ | -CH(CH₃)₂ | -H | -CH(CH₃)₂ | -CH(CH₃)₂ | -H |
| 366 | -CH₂CH₂CH₂CH₃ | -CH(CH₃)₂ | -H | -CH₂CH₂CH₂CH₃ | -CH(CH₃)₂ | -H |
| 367 | -H(CH₃)CH₂CH₃ | -CH(CH₃)₂ | -H | -CH(CH₃)CH₂CH₃ | -CH(CH₃)₂ | -H |
| 368 | -CH₂CH(CH₃)₂ | -CH(CH₃)₂ | -H | -CH₂CH(CH₃)₂ | -CH(CH₃)₂ | -H |
| 369 | -C(CH₃)₃ | -CH(CH₃)₂ | -H | -C(CH₃)₃ | -CH(CH₃)₂ | -H |
| 370 | -H | -CH(CH₃)₂ | -CH₃ | -H | -CH(CH₃)₂ | -CH₃ |
| 371 | -CH₃ | -CH(CH₃)₂ | -CH₃ | -CH₃ | -CH(CH₃)₂ | -CH₃ |
| 372 | -CH₂CH₃ | -CH(CH₃)₂ | -CH₃ | -CH₂CH₃ | -CH(CH₃)₂ | -CH₃ |
| 373 | -CH₂CH₂CH₃ | -CH(CH₃)₂ | -CH₃ | -CH₂CH₂CH₃ | -CH(CH₃)₂ | -CH₃ |
| 374 | -CH(CH₃)₂ | -CH(CH₃)₂ | -CH₃ | -CH(CH₃)₂ | -CH(CH₃)₂ | -CH₃ |
| 375 | -CH₂CH₂CH₂CH₃ | -CH(CH₃)₂ | -CH₃ | -CH₂CH₂CH₂CH₃ | -CH(CH₃)₂ | -CH₃ |
| 376 | -H(CH₃)CH₂CH₃ | -CH(CH₃)₂ | -CH₃ | -CH(CH₃)CH₂CH₃ | -CH(CH₃)₂ | -CH₃ |
| 377 | -CH₂CH(CH₃)₂ | -CH(CH₃)₂ | -CH₃ | -CH₂CH(CH₃)₂ | -CH(CH₃)₂ | -CH₃ |
| 378 | -C(CH₃)₃ | -CH(CH₃)₂ | -CH₃ | -C(CH₃)₃ | -CH(CH₃)₂ | -CH₃ |
| 379 | -H | -CH(CH₃)₂ | -CH₂CH₃ | -H | -CH(CH₃)₂ | -CH₂CH₃ |
| 380 | -CH₃ | -CH(CH₃)₂ | -CH₂CH₃ | -CH₃ | -CH(CH₃)₂ | -CH₂CH₃ |
| 381 | -CH₂CH₃ | -CH(CH₃)₂ | -CH₂CH₃ | -CH₂CH₃ | -CH(CH₃)₂ | -CH₂CH₃ |
| 382 | -CH₂CH₂CH₃ | -CH(CH₃)₂ | -CH₂CH₃ | -CH₂CH₂CH₃ | -CH(CH₃)₂ | -CH₂CH₃ |
| 383 | -CH(CH₃)₂ | -CH(CH₃)₂ | -CH₂CH₃ | -CH(CH₃)₂ | -CH(CH₃)₂ | -CH₂CH₃ |
| 384 | -CH₂CH₂CH₂CH₃ | -CH(CH₃)₂ | -CH₂CH₃ | -CH₂CH₂CH₂CH₃ | -CH(CH₃)₂ | -CH₂CH₃ |
| 385 | -H(CH₃)CH₂CH₃ | -CH(CH₃)₂ | -CH₂CH₃ | -CH(CH₃)CH₂CH₃ | -CH(CH₃)₂ | -CH₂CH₃ |
| 386 | -CH₂CH(CH₃)₂ | -CH(CH₃)₂ | -CH₂CH₃ | -CH₂CH(CH₃)₂ | -CH(CH₃)₂ | -CH₂CH₃ |
| 387 | -C(CH₃)₃ | -CH(CH₃)₂ | -CH₂CH₃ | -C(CH₃)₃ | -CH(CH₃)₂ | -CH₂CH₃ |
| 388 | -H | -CH(CH₃)₂ | -CH₂CH₂CH₃ | -H | -CH(CH₃)₂ | -CH₂CH₂CH₃ |
| 389 | -CH₃ | -CH(CH₃)₂ | -CH₂CH₂CH₃ | -CH₃ | -CH(CH₃)₂ | -CH₂CH₂CH₃ |
| 390 | -CH₂CH₃ | -CH(CH₃)₂ | -CH₂CH₂CH₃ | -CH₂CH₃ | -CH(CH₃)₂ | -CH₂CH₂CH₃ |
| 391 | -CH₂CH₂CH₃ | -CH(CH₃)₂ | -CH₂CH₂CH₃ | -CH₂CH₂CH₃ | -CH(CH₃)₂ | -CH₂CH₂CH₃ |
| 392 | -CH(CH₃)₂ | -CH(CH₃)₂ | -CH₂CH₂CH₃ | -CH(CH₃)₂ | -CH(CH₃)₂ | -CH₂CH₂CH₃ |
| 393 | -CH₂CH₂CH₂CH₃ | -CH(CH₃)₂ | -CH₂CH₂CH₃ | -CH₂CH₂CH₂CH₃ | -CH(CH₃)₂ | -CH₂CH₂CH₃ |
| 394 | -H(CH₃)CH₂CH₃ | -CH(CH₃)₂ | -CH₂CH₂CH₃ - | CH(CH₃)CH₂CH₃ | -CH(CH₃)₂ | -CH₂CH₂CH₃ |
| 395 | -CH₂CH(CH₃)₂ | -CH(CH₃)₂ | -CH₂CH₂CH₃ | -CH₂CH(CH₃)₂ | -CH(CH₃)₂ | -CH₂CH₂CH₃ |
| 396 | -C(CH₃)₃ | -CH(CH₃)₂ | -CH₂CH₂CH₃ | -C(CH₃)₃ | -CH(CH₃)₂ | -CH₂CH₂CH₃ |
| 397 | -H | -CH(CH₃)₂ | -CH(CH₃)₂ | -H | -CH(CH₃)₂ | -CH(CH₃)₂ |
| 398 | -CH₃ | -CH(CH₃)₂ | -CH(CH₃)₂ | -CH₃ | -CH(CH₃)₂ | -CH(CH₃)₂ |
| 399 | -CH₂CH₃ | -CH(CH₃)₂ | -CH(CH₃)₂ | -CH₂CH₃ | -CH(CH₃)₂ | -CH(CH₃)₂ |
| 400 | -CH₂CH₂CH₃ | -CH(CH₃)₂ | -CH(CH₃)₂ | -CH₂CH₂CH₃ | -CH(CH₃)₂ | -CH(CH₃)₂ |
| 401 | -CH(CH₃)₂ | -CH(CH₃)₂ | -CH(CH₃)₂ | -CH(CH₃)₂ | -CH(CH₃)₂ | -CH(CH₃)₂ |
| 402 | -CH₂CH₂CH₂CH₃ | -CH(CH₃)₂ | -CH(CH₃)₂ | -CH₂CH₂CH₂CH₃ | -CH(CH₃)₂ | -CH(CH₃)₂ |
| 403 | -H(CH₃)CH₂CH₃ | -CH(CH₃)₂ | -CH(CH₃)₂ | -CH(CH₃)CH₂CH₃ | -CH(CH₃)₂ | -CH(CH₃)₂ |
| 404 | -CH₂CH(CH₃)₂ | -CH(CH₃)₂ | -CH(CH₃)₂ | -CH₂CH(CH₃)₂ | -CH(CH₃)₂ | -CH(CH₃)₂ |
| 405 | -C(CH₃)₃ | -CH(CH₃)₂ | -CH(CH₃)₂ | -C(CH₃)₃ | -CH(CH₃)₂ | -CH(CH₃)₂ |

Zusammenfassend sind erfindungsgemäße Mittel zum Färben und/oder Aufhellen keratinischer Fasern bevorzugt, die als ein Gefühl von Wärme erzeugende Substanz mindestens einen Scharfstoff aus den Gruppen der
- alkylsubstituierten Dioxane der Formel 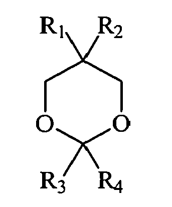 in der R1 und R2 unabhängig voneinander ausgewählt sind aus -H, -CH₃, - CH₂CH₃ und R3 und R4 unabhängig voneinander ausgewählt sind aus -H, -CH₃, -CH₂CH₃, -CH₂CH₂CH₃, -CH(CH₃)₂ und/oder
- Phenylestern der Formel 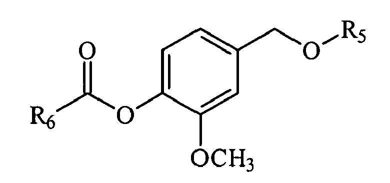 in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht und/oder
- Carvonacetalen der Formel 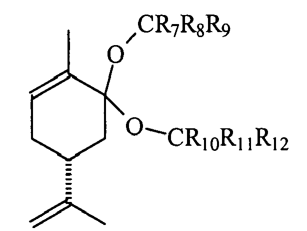 in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃ oder R9 und R10 zusammen eine chemiche Bindung oder eine Gruppe -(CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃
enthalten.

Die erfindungsgemäßen Mittel zum Färben und/oder Aufhellen keratinischer Fasern können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein weiterer Gegenstand der vorliegenden Erfindung ist daher ein erfindungsgemäßes Mittel zum Färben und/oder Aufhellen keratinischer Fasern, insbesondere menschlicher Haare, welches unmittelbar vor dem Aufbringen auf das Haar aus einer fließfähigen Zubereitung A, einer Oxidationsmittelzubereitung B, enthaltend mindestens ein Oxidationsmittel, ausgewählt aus Wasserstoffperoxid und dessen Anlagerungsverbindungen an feste Träger und gegebenenfalls einer Zubereitung C, die miteinander zu einem Färbe- und/oder Aufhellansatz vermischt werden, erhalten wird. Eine oder mehrere dieser Zubereitungen A, B und/oder C enthalten demnach mindestens eine ein Gefühl von Wärme erzeugende Substanz. Oxidationsfarbstoffvorprodukte und/oder direktziehende Farbstoffe sind dabei zumeist in der Zubereitung A enthalten.

Die Mischung der Zubereitungen A, B und C vor der Anwendung führt zu einer Anwendungsmischung, die mindestens eine ein Gefühl von Wärme erzeugende Substanz enthält.

Besonders bevorzugt werden die Zubereitungen A und B miteinander in einem Gewichtsverhältnis von 4:1 bis 1:4, vorzugsweise 3:1 bis 1:3, weiter bevorzugt 2:1 bis 1:2 und insbesondere 1:2 bis 1:1,5 vermischt. Bevorzugte Mischungsverhältnisse von (A+B) zu C liegen im Bereich von 120:1 bis 1:1, vorzugsweise von 100:1 bis 1,5:1, weiter bevorzugt von 80:1 bis 2:1 und insbesondere von 50:1 bis 3:1.

Vorzugsweise werden die erfindungsgemäßen Mittel als Aufhellmittel (nachfolgend auch als Blondiermittel bezeichnet) und/oder als Färbemittel bereitgestellt. Mittel, welche gleichzeitig färbend und aufhellend wirken, werden auch als aufhellende Färbemittel bezeichnet.

Im Falle der Mehrkomponentenmittel gemäß der zweiten Ausführungsform der vorliegenden Erfindung sind die Oxidationsfarbstoffvorprodukte vom Entwickler- und Kupplertyp vorzugsweise in der Zubereitung A enthalten.

Die Oxidationsmittelzubereitung B enthält mindestens ein Oxidationsmittel, ausgewählt aus Wasserstoffperoxid und dessen Anlagerungsverbindungen an feste Träger. Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidonn H₂O₂ (n ist eine positive ganze Zahl größer 0), Harnstoffperoxid und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet. Erfindungsgemäß bevorzugte Mittel zum Färben und/oder Aufhellen keratinischer Fasern sind dadurch gekennzeichnet, daß sie 0,5 bis 15 Gew.-%, vorzugsweise 1 bis 12,5 Gew.-%, besonders bevorzugt 2,5 bis 10 Gew.-% und insbesondere 3 bis 7 Gew.-% Wasserstoffperoxid (berechnet als 100%iges H₂O₂ enthalten.

Die Oxidationsmittelzubereitung B ist vorzugsweise eine wäßrige, fließfähige Oxidationsmittelzubereitung. Demnach sind bevorzugte erfindungsgemäße Mittel dadurch gekennzeichnet, daß die fließfähige Oxidationsmittelzubereitung B - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Vorzugsweise wird der fließfähigen Oxidationsmittelzubereitung B ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind. Diese Stoffe werden weiter unten ausführlich beschrieben.

Die Oxidationsmittelzubereitung kann weitere Oxidationsmittel enthalten, die auch als "Booster" bezeichnet werden. Die Auswahl dieser weiteren Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten. Hier sind erfindungsgemäße Mittel bevorzugt, bei denen die fließfähige Oxidationsmittelzubereitung B zusätzlich mindestens eine Peroxoverbindung enthält, die vorzugsweise ausgewählt ist aus Ammonium- und Alkalimetall-persulfaten und -peroxidisulfaten, wobei bevorzugte Mittel mindestens 2 verschiedene Peroxidisulfate enthalten.

Bei einer Anwendung von Oxidationsmitteln wird das eigentliche Färbemittel wie weiter oben erwähnt, zweckmäßigerweise unmittelbar vor der Anwendung durch Mischung der Zubereitung des Oxidationsmittels B mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-lonen oder Komplexe dieser Ionen enthalten.

Bevorzugte erfindungsgemäße Haarfärbe- und - aufhellungsmittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Die erfindungsgemäßen Mittel zum Färben und/oder Aufhellen keratinischer Fasern können weiterhin alle für solche Zubereitungen bekannten Wirk-, Zusatz- und Hilfsstoffe enthalten. Im falle der Mehrkomponentenmittel können diese Inhaltstoffe sowohl in der Zubereitung A als auch in der Zubereitung B als auch in der Zubereitung C oder nur in einzelnen dieser Zubereitungen enthalten sein.

In vielen Fällen enthalten die erfindungsgemäßen Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 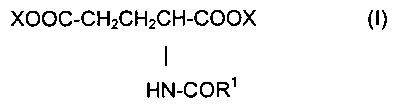 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 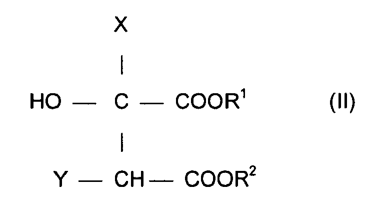 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 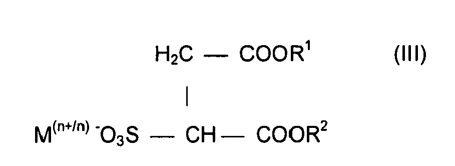 in der M(^{n+/n}) für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.
Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen^{®} HSP (Cognis) oder Sovermol^{®} - Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für Alkyl, Z für Zucker sowie x für die Anzahl der Zuckereinheiten steht. Besonders bevorzugt sind solche Alkylpolyglycoside, bei denen R
   - im wesentlichen aus C₈- und C₁₀-Alkylgruppen,
   - im wesentlichen aus C₁₂- und C₁₄-Alkylgruppen,
   - im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov^{®}68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls^{®} PGPH) oder Triglycerindiisostearat (Lameform^{®} TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Die erfindungsgemäßen Mittel zum Färben und/oder Aufhellen keratinischer Fasern können weiterhin alle für solche Zubereitungen bekannten Wirk-, Zusatz- und Hilfsstoffe enthalten.

In einer weiteren bevorzugten Ausführungsform enthalten die erfindungsgemäßen Zusammensetzungen mindestens einen hautberuhigenden Wirkstoff. Erfindungsgemäß bevorzugte hautberuhigende Wirkstoffe sind ausgewählt aus Allantoin, α-Bisabolol und α-Liponsäure.

Die hautberuhigenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.
In einer weiteren bevorzugten Ausführungsform enthalten die erfindungsgemäßen Zusammensetzungen mindestens eine alpha-Hydroxycarbonsäure, alpha-Ketocarbonsäure oder beta-Hydroxycarbonsäure oder deren Ester-, Lacton- oder Salzform. Erfindungsgemäß geeignete alpha-Hydroxycarbonsäuren oder alphaKetocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte alpha-Hydroxycarbonsäuren sind Milchsäure, Citronensäure, Glycolsäure und Gluconsäure. Eine besonders bevorzugte beta-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die alpha-Hydroxycarbonsäuren, alpha-Ketocarbonsäuren oder beta-Hydroxycarbonsäuren oder ihre Derivate sind vorzugsweise in Mengen von 0,1 - 10 Gew.-%, bevorzugt 0,5 - 5 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-a-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß werden die Flavonoide vorzugsweise in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt. Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin. Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß werden die Isoflavonoide vorzugsweise in Mengen von 0,00001 bis 1 Gew.-%, bevorzugt 0,0005 bis 0,5 Gew.-% und besonders bevorzugt 0,001 bis 0,1 Gew.-%, jeweils bezogen auf die lsoflavonoidaktivsubstanz in der gesamten kosmetischen Zusammensetzung, eingesetzt.

In einer weiteren bevorzugten Ausführungsform enthalten die erfindungsgemäßen Zusammensetzungen mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Erfindungsgemäß werden die Polyphenole vorzugsweise in Mengen von 0,001 bis 10 Gew.-%, bevorzugt 0,005 bis 5 Gew.% und besonders bevorzugt 0,01 bis 3 Gew.-%, jeweils bezogen auf die gesamte kosmetische Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (II) auf: 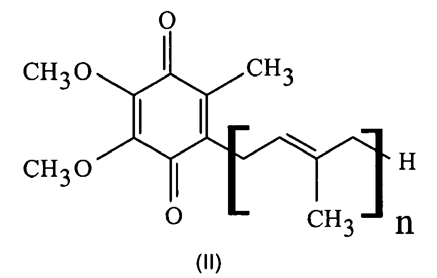 mit n = 6, 7, 8, 9 oder 10.
Besonders bevorzugt ist das Ubichinon der Formel (II) mit n = 10, auch bekannt als Coenzym Q10.
Erfindungsgemäß werden die Ubichinone, Ubichinole oder deren Derivate vorzugsweise in Mengen von 0,0001 bis 1 Gew.-%, bevorzugt 0,001 bis 0,5 Gew.-% und besonders bevorzugt 0,005 bis 0,1 Gew.%, jeweils bezogen auf die gesamte Zusammensetzung, eingesetzt.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß wird Silymarin vorzugsweise in Mengen von 0,0001 bis 1 Gew.-%, bevorzugt 0,001 bis 1,0 Gew.-% und besonders bevorzugt 0,005 bis 0,5 Gew.-%, jeweils bezogen auf die gesamte Zusammensetzung, eingesetzt.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin.
Erfindungsgemäß sind die natürlich vorkommenden Xanthin-Derivate vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß ist Ectoin vorzugsweise in Mengen von 0,0001 bis 1 Gew.-%, bevorzugt 0,001 bis 0,5 Gew.-% und besonders bevorzugt 0,005 bis 0,01 Gew.-%, jeweils bezogen auf die gesamte Zusammensetzung, enthalten.

Schließlich können die erfindungsgemäßen Mittel auch Pflanzenextrakte (L) enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Als weiterhin vorteilhaft hat es sich gezeigt, wenn Polymere (G) in den erfindungsgemäßen Mitteln enthalten sind. In einer bevorzugten Ausführungsform werden den erfindungsgemäßen Mitteln daher Polymere zugesetzt, wobei sich sowohl kationische, anionische, amphotere als auch nichtionische Polymere als wirksam erwiesen haben.

Erfindungsgemäß einsetzbar sind vorzugsweise kationische bzw. amphotere Polymere. Unter kationischen bzw. amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I),

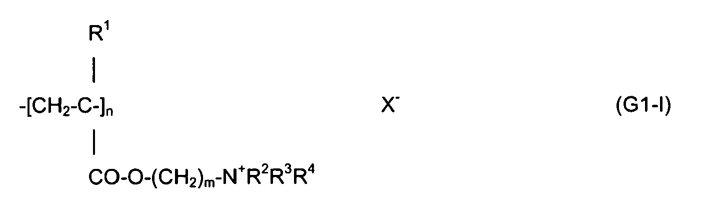

in der R¹ = -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-1) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten:
Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und
Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A(-) (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in denen R⁶ und R⁷ unabhängig voneinander Wasserstoff oder Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z-I), die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z-I) und (Z-II) nach an sich bekannten Polymerisationsverfahren hergestellt. Die Polymerisation kann entweder in wäßriger oder wäßrig-alkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (Z-I) gegenüber den Monomeren der Formel (Z-II) im Überschuß vorlagen. Es ist daher erfindungsgemäß bevorzugt, solche Polymerisate zu verwenden, die aus Monomeren der Formel (Z-I) und die Monomeren der Formel (Z-II) in einem Molverhältnis von 60:40 bis 95:5, insbesondere von 75:25 bis 95:5, bestehen.

Die kationischen bzw. amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Eine besonders bevorzugte Gruppe von Inhaltsstofen stellen die Silikone dar.

Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in derKette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Färbemittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Oxidationsfärbemittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet. Es werden im Rahmen der vorliegenden Erfindung als Silicon der Formel Si-I vorzugsweise die Verbindungen:

(CH₃)₃Si-O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-Si(CH₃)₃

(CH₃)₃Si-[O-(CH₃)₂Si]₆-O-Si(CH₃)₃

(CH₃)₃Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]₉-O-Si(CH₃)₃

(CH₃)₃Si-[O-(CH₃)₂Si]₁₀-O-Si(CH₃)₃

(CH₃)₃Si-[O-(CH₃)₂Si]₁₁-O-Si(CH₃)₃

(CH₃)₃Si-[O-(CH₃)₂Si]₁₂-O-Si(CH₃)₃

(CH₃)₃Si-[O-(CH₃)₂Si]₁₃-O-Si(CH₃)₃

(CH₃)₃Si-[O-(CH₃)₂Si]₁₄-O-Si(CH₃)₃

(CH₃)₃Si-[O-(CH₃)₂Si]₁₅-O-Si(CH₃)₃

(CH₃)₃Si-[O-(CH₃)₂Si]₁₆-O-Si(CH₃)₃

(CH₃)₃Si-[O-(CH₃)₂Si]₁₇-O-Si(CH₃)₃

(CH₃)₃Si-[O-(CH₃)₂Si]₁₈-O-Si(CH₃)₃

(CH₃)₃Si-[O-(CH₃)₂Si]₁₉-O-Si(CH₃)₃

(CH₃)₃Si-[O-(CH₃)₂Si]₂₀-O-Si(CH₃)₃

eingesetzt, wobei (CH₃)₃Si--O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskosiäten von 0,5 bis 1 mm²s⁻¹ besonders bevozugt sind.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(R_{c}SiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, - CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH ₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂ )_{z}NH₂, worin z 1 oder mehr ist. Eine andere mögliche Formel für Z ist -NH(CH₂)_{z}(CH ₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel - CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Oxidationsfärbemittel sind dadurch gekennzeichnet, daß sie ein aminofunktionelles Silikon der Formel (Si-II)

R'ₐG₃₋ₐ-Si(OSiG ₂)ₙ-(OSiG _{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -Q-N(R")-CH₂-CH₂-N(R")₂
   o -Q-N(R")₂
   o -Q-N⁺(R")3A⁻
   o -Q-N⁺H(R")₂ A⁻
   o -Q-N⁺H₂(R")A⁻
   o -Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie mindestens ein aminofunktionelles Silikon der Formel (Si-IIa) 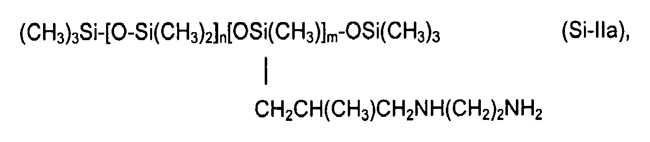 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die ein aminofunktionelles Silikon der Formel (Si-Ilb) 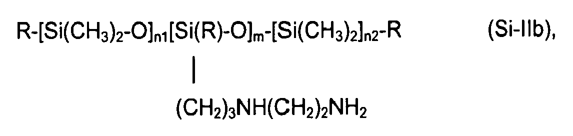 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionalles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Erfindungsgemäß bevorzugte Färbemittel sind dadurch gekennzeichnet, daß sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Färbemittel für keratinische Fasern bevorzugt, die mindestens ein Silikon der Formel Si-III 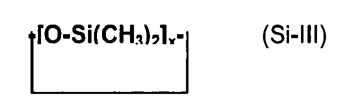 enthalten, in der x für eine Zahl von 2 bis 200, vorzugsweise von 3 bis 10, weiter bevorzugt von 3 bis 7 und insbesondere 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfaßt dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstaoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreakktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, daß durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endtsändigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), vorzugsweise sind die Polymere aber substantiell linear, besonders bevorzugt vollständig linear. Zudem können die Substituenten R ihrereseits substituiert sein, beispielsweise mit N-haltigen Gruppen (beispielsweise Aminogruppen), Epoxygruppen, S-haltige Gruppen, Sihaltige Gruppen, O-haltige Gruppen usw.. Vorzugsweise sind mindestens 80% der Reste R Alkylrste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfaßt, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfaßt, welches mindestens eine Si-OH Gruppe aufweist, kettenverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosyslylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vortehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveräbnderunge bevorzugter Katalysator ist Chloroplatinsäure entweder als kommerziell verfügbares Hexahydrat oder in wasserfreier Form.

Bei einer weiteren bevorzugten Kettenerweiterungsrekation wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethylzinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitanat, Tetraoctadecyltitanat, Titannaphthanat, Ethyltriethanolamin-Titanat, Titani-diisopropyl-diethyl-acetoacetat, Titan-diisopropoxydiacetyl-acetonat und Titani-tetra-Alkoxide, bei denen das Alkoxid Butoxy oder Propoxy ist.

Erfindungsgemäßebenfalls bevorzugte Färbemittel sind dadurch gekennzeichnet, daß sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Färbemittel sind dadurch gekennzeichnet, daß sie mindestens ein wasserlösliches Silikon enthalten.

Ferner können die erfindungsgemäßen Mittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Als weiteren optionalen Bestandteil können die erfindungsgemäßen Mittel 0,01 bis 10 Gew.-% Chitosan und/oder Chitosanderivat(e) enthalten. Chitosan ist das Deacetylierungsprodukt von Chitin, wobei ein definierter Übergang zwischen Chitosan und Chitin nicht existiert. Im Rahmen der vorliegenden Erfindung wird von Chitosan gesprochen, wenn der Deacetylierungsgrad des Chitins mindestens 20 % beträgt. Bevorzugte erfindungsgemäße Mittel enthalten Chitosane (bzw. Derivate davon), deren Deacetylierungsgrad > 30 %, insbesondere >40-50% beträgt. Weiter bevorzugt sind Chitosane und Derivate davon, die in organischen Säuren löslich sind.

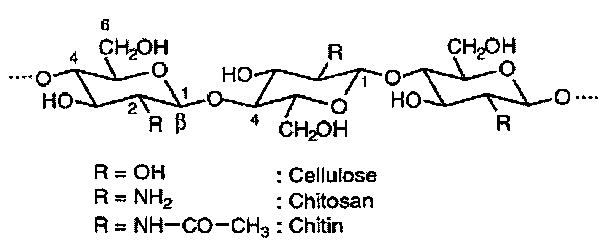

Bevorzugte erfindungsgemäß eingesetzte Chitosane oder Derivate davon weisen Molmassen von 100.000 bis 500.000 Dalton auf. Chitosan besteht aus Ketten von beta-1,4-glycosidisch verknüpften N-Acetyl-D-glucosamin-(NAG-)Resten.

Bevorzugte erfindungsgemäße Mittel zum Färben und/oder Aufhellen keratinischer Fasern sind dadurch gekennzeichnet, daß sie - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 1,5 Gew.-% Chitosan und/oder Chitosanderivat(e) enthalten.

Vorzugsweise sind die in den erfindungsgemäßen Mitteln eingesetzten Chitosane bzw. deren Derivate wasserlöslich. Bevorzugte erfindungsgemäße Mittel zum Färben und/oder Aufhellen keratinischer Fasern sind dadurch gekennzeichnet, daß sie Chitosanderivat(e) enthalten, die eine Wasserlöslichkeit in destilliertem Wasser oberhalb von 1 g/l (20°C, 1013 mbar) aufweisen.

Bevorzugt einzusetzende Chitosanderivate sind an der Aminogruppe derivatisiert. Besonders bevorzugte Chitosanderivate weisen einen an das Stickstoffatom der Aminogruppe gebundenen Säurerest, vorzugsweise den Rest einer organischen Säure, auf. Besonders bevorzugte Chitoanderivate weisen mindestens einen an das Stickstoffatom der Aminogruppe gebundenen Säurerest folgender Säuren auf: Ameisensäure, Essigsäure, Propionsäure, Buttersäure, Valeriansäure, Capronsäure, Heptansäure, Caprylsäure, Pelargonsäure, Caprinsäure, Undecansäure, Laurinsäure, Tridecansäure, Tetradecansäure, Pentadecansäure, Palmitinsäure, Margarinsäure, Stearinsäure, Nonadecansäure, Arachinsäure, Behensäure, Lignocerinsäure, Cerotinsäure, Melissinsäure, Isobuttersäure, Isovaleriansäure, Tubercolostearinsäure, Acrylsäure, Crotonsäure, Palmitoleinsäure, Ölsäure, Erucasäure, Sorbinsäure, Linolsäure, Linolensäure, Elaeostearinsäure, Arachidonsäure, Clupanodonsäure, Docosahexaensäure, Oxalsäure, Malonsäure, Bernsteinsäure, Glutarsäure, Adipinsäure, Pimelinsäure, Azelainsäure, Sebacinsäure, Maleinsäure, Fumarsäure, Muconsäure, Phthalsäure, Terephthalsäure.

Besonders bevorzugte erfindungsgemäße Mittel zum Färben und/oder Aufhellen keratinischer Fasern sind dadurch gekennzeichnet, daß das/die in ihnen enthaltene(n) Chitosanderivat(e) ausgewählt ist/sind aus
- Chitosanglycolat
- N-Acetyl-Chitosan (Chitosanacetat)
- N-Propionyl-Chitosan (Chitosanpropionat)
- N-Butanoyl-Chitosan (Chitosanbutanoat)
- N-Malonyl-Chitosan (Chitosanmalonat)
- N-Succinyl-Chitosan (Chitosansuccinat)
- N-Adipyl-Chitosan (Chitosanadipat).

Zusammenfassend sind erfindungsgemäße Mittel zum Färben und/oder Aufhellen keratinischer Fasern bevorzugt, die zusätzlich - 0,01 bis 5 Gew.-%, vorzugsweise 0,02 bis 2,5 Gew.-%, besonders bevorzugt 0,05 bis 1 Gew.-% und insbesondere 0,1 bis 0,5 Gew.-% Chitosanglycolat oder N-Acetyl-Chitosan oder N-Succinyl-Chitosan enthalten.

Eine weitere bevorzugte Gruppe von Inhaltsstoffen der erfindungsgemäßen Mittel sind Vitamine, Provitamine oder Vitaminvorstufen. Diese werden nachfolgend beschrieben:

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in

Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.
Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Mittel zum Färben und/oder Aufhellen keratinischer Fasern bevorzugt , die zusätzlich Vitamine und/oder Pro-Vitamine und/oder Vitaminvorstufen enthalten, die vorzugsweise den Gruppen A, B, C, E, F und H zugeordnet werden, wobei bevorzugte Mittel die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

Erfindungsgemäß bevorzugte Mittel enthalten zusätzlich mindestens ein 2-Furanonderivat der Formel (I) und/oder der Formel (II). 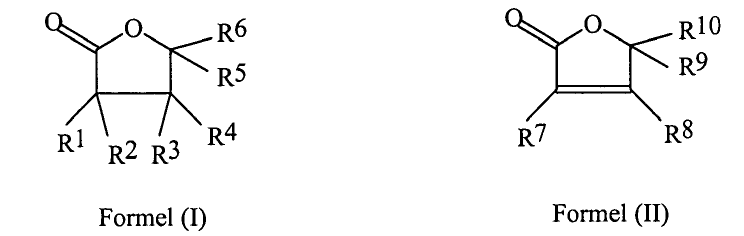 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂- C₄- gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen - C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen - C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

2-Furanone sind bekannte Verbindungen und werden beispielsweise in "Römpps Lexikon der Chemie, Interactive CD-Rom Version 2.0, zum Stichwort "Dihydro-3-hydroxy-4,4-dimethyl-2(3H)-furanon" sowie in "Ullmann's Encyclopedia, sixth edition 1999, electronic release" in den Abschnitten 2.4, 2.7, 3.2, 3.4, 4.3, 6., 11. und 15. und den dort zitierten Schriften bezüglich der Herstellung und Verwendung beschrieben. Auf diese Kapitel und die dort zitierte Literatur wird ausdrücklich Bezug genommen. Die Verbindungen der Formeln (I) und (II) werden als Zwischenstufen in der Naturstoffsynthese sowie der Herstellung von Arzneimitteln und Vitaminen eingesetzt. Die Herstellung der Wirkstoffe gemäß der Formeln (I) und (II) kann beispielsweise durch Umsetzung von primären Alkoholen mit Acrylsäuren erfolgen. Weiterhin gelangt man zu Verbindungen der Formel (I) durch Reaktionen ausgehend von Hydroxypivaldehyd. Ebenfalls führen Carbonylierungen von Alkynen zu substituierten 2-Furanonen der Formel (I) oder (II). Schließlich können die Verbindungen der Formel (I) oder der Formel (II) durch intramolekulare Veresterung der entsprechenden Hydroxycarbonsäuren erhalten werden. Beispielsweise werden die folgenden Verbindungen auf einem der zuvor aufgezeigten Synthesewege erhalten: 2,5-Dihydro-5-methoxy-2-furanon, Tetrahydro-5-oxo-2-furancarbonsäure, Dihydro-3-hydroxy-4,4-dimethyl-2(3H)-furanon, oder 3,4-Dimethyl-5-pentylidenedihydro-2(5H)-furanon oder 4-Hydroxy-2,5-dimethyl-3(2H)-furanon. Die erfindungsgemäßen 2-Furanone umfassen selbstverständlich alle möglichen Stereoisomere wie auch deren Gemische. Durch die erfindungsgemäßen 2-Furanone wird der Geruch der kosmetischen Mittel nicht nachhaltig beeinflußt, so daß eine Parfümierung der Mittel separat erfolgen muß.

Bevorzugte Verbindungen der Formel (I) und/oder der Formel (II) können Verbindungen sein, bei welchen die Substituenten R¹, R² und R⁷ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- einen -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxyalkylrest, oder einen -C₂ - C₃₀ - gesättigten oder ein- oder mehrfach ungesättigten, verzweigten oder linearen Mono-, Di-, Tri- oder Polyaminokohlenwasserstoffrest.

In einer weiteren Ausführungsform der erfindungsgemäßen Lehre hat es sich gezeigt, daß bei den Verbindungen der Formel (I) oder der Formel (II) die Reste R³, R⁴ und R⁸ bevorzugt unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Kohlenwasserstoffrest,
- einen -C₂ - C₄ - gesättigten oder ein- oder zweifach ungesättigten, verzweigten oder linearen Mono-, Di- oder Trihydroxykohlenwasserstoffrest oder
- einen -C₂ - C₄ - gesättigten oder ein- oder zweifach ungesättigten, verzweigten oder linearen Mono-, Di- oder Triaminokohlenwasserstoffrest.
Weiterhin kann es bevorzugt sein, wenn in dem erfindungsgemäßen Wirkstoff gemäß der Formel(I) und/oder der Formel(II) für die Reste R⁵, R⁶, R⁹ und R¹⁰ unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Kohlenwasserstoffrest,
- einen -C₂ - C₄ - gesättigten oder ein- oder zweifach ungesättigten, verzweigten oder linearen Mono-, Di- oder Trihydroxykohlenwasserstoffrest oder
- einen -C₂ - C₄ - gesättigten oder ein- oder zweifach ungesättigten, verzweigten oder linearen Mono-, Di- oder Triaminokohlenwasserstoffrest.

In einer besonders bevorzugten Ausführungsform der erfindungsgemäßen Lehre wird eine Verbindung der Formel (I) eingesetzt. Dabei kann es bevorzugt sein, daß in einer Verbindung der Formel (I) die Reste R¹ und R² unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxykohlenwasserstoffrest. Weiterhin kann es in dieser besonders bevorzugten Ausführungsform der erfindungsgemäßen Lehre vorteilhaft sein, wenn in den Verbindungen der Formel (I) die Reste R³ und R⁴ unabhängig voneinander stehen für:

- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- und/oder Polyhydroxykohlenwasserstoffrest.

In dieser bevorzugten Ausführungsform kann es weiterhin vorteilhaft sein, daß die Verbindungen gemäß Formel (I) für die Reste R5 und R6 unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest.

In einer besonders bevorzugten Ausführungsform der erfindungsgemäßen Lehre wird als Verbindung entsprechend der Formel (I)
- (R)-(-)-4-Hydroxymethyl-γ-butyrolacton und/oder
- D,L-4-Hydroxymethyl-γ-butyrolacton und/oder
- (S)-(+)-4-Hydroxymethyl-γ-butyrolacton und/oder
- R-(-)-2-Hydroxy-3,3-dimethyl-γ-butyrolacton und/oder
- D,L-2-Hydroxy-3,3-dimethyl-γ-butyrolacton und/oder
- S(+)-2-Hydroxy-3,3-dimethyl-γ-butyrolacton und/oder
- 4-Hydroxy-2,5-dimethyl-3(2H)-furanon und/oder
- Tetrahydro-5-oxo-2-furancarbonsäure und/oder
- Tetrahydro-5-oxo-2-furancarbonsäure, Na-Salz und/oder
- Tetrahydro-5-oxo-2-furancarbonsäure, K-Salz und/oder
- 2,5-Dihydro-5-methoxy-2-furanon und/oder
- Dihydro-3-hydroxy-4,4-dimethyl-2(3*H*)-furanon
eingesetzt. In einer ganz besonders bevorzugten Ausführungsform der erfindungsgemäßen Lehre wird als Verbindung entsprechend der Formel (I) Dihydro-3-hydroxy-4,4-dimethyl-2(3H)-furanon eingesetzt.

Zusammenfassend sind bevorzugt erfindungsgemäße Mittel zum Färben und/oder Aufhellen keratinischer Fasern dadurch gekennzeichnet, daß sie zusätzlich 0,05 bis 15 Gew.-% mindestens eines 2-Furanonderivats der Formel (I) und/oder der Formel (II) 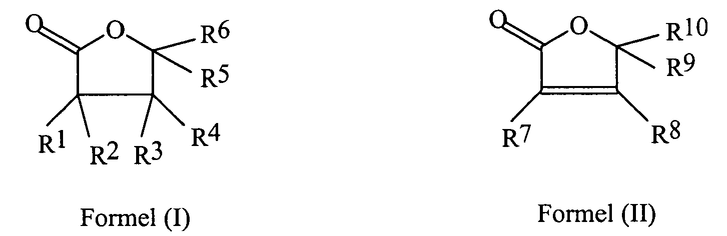 enthalten, in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen - C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

Bevorzugt ist auch der zusätzlich Einsatz von Bisabolol und/oder Bisabololoxiden in den erfindungsgemäßen Mitteln. Hier sind erfindungsgemäße Mittel zum Färben und/oder Aufhellen keratinischer Fasern bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 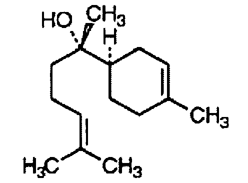 enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, sowie auf die weiter unten stehenden Ausführungen verwiesen.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-trans-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Mittel zum Färben und/oder Aufhellen keratinischer Fasern dadurch gekennzeichnet, daß sie zusätzlich - 0,05 bis 5 Gew.-%, vorzugsweise 0,1 bis 2,5 Gew.%, besonders bevorzugt 0,15 bis 1 Gew.% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Die erfindungsgemäßen Mittel dienen zum Färben und/oder Aufhellen keratinischer Fasern. Ein weiterer Gegenstand der vorliegenden Anmeldung ist daher ein Verfahren zum Färben und/oder Aufhellen keratinischer Fasern, bei dem
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein erfindungsgemäßes Mittel ist.

In erfindungsgemäß bevorzugten Verfahren wird das Haar im Anschluß an die Applikation des ersten Mittels ausgespült, und besonders bevorzugte Verfahren sind dadurch gekennzeichnet, daß die Applikation von Haarfärbemittel(n) auf feuchtes Haar oder die feuchten Haarkonturen erfolgt. "Feucht" im Sinne der vorliegenden Anmeldung ist auch handtuchtrocken.

Bezüglich bevorzugter Ausführungsformen des erfindungsgemäßen Verfahrens gilt mutatis mutandis das zu den erfindungsgemäßen Mitteln Ausgeführte. Insbesondere sind erfindungsgemäße Verfahren bevorzugt, bei denen das Haarfärbemittel mindestens einen direktziehenden Farbstoff enthält.

Auch erfindungsgemäße Verfahren, bei denen das Haarfärbemittel mindestens eine aromatische oder heteroaromatische aktivierte Carbonylverbindung und/oder eine CH-acide Verbindung enthält, sind bevorzugte Ausführungsformen der vorliegenden Erfindung.

Ein weiterer Gegenstand der vorliegenden Erfindung ist die Verwendung mindestens einer Substanz, die ein Gefühl von Wärme erzeugt, in Mitteln zur Behandlung keratinischer Fasern, insbesondere in haarkosmetischen Mitteln für Humanhaar.

Bevorzugt ist die erfindungsgemäße Verwendung in Mitteln zum Färben und/oder Aufhellen keratinischer Fasern, insbesondere menschlicher Haare, so daß die Verwendung mindestens einer Substanz, die ein Gefühl von Wärme erzeugt, in Mitteln zum Färben und/oder Aufhellen keratinischer Fasern ein weiterer Gegenstand der vorliegenden Erfindung ist.

Erfindungsgemäß bevorzugte Verwendungen dienen zur
- Steigerung der Aufhell- bzw. Färbeleistung und/oder
- Steigerung der Hautverträglichkeit von Aufhell- bzw. Färbemitteln und/oder
- Anzeige der Einwirkzeit von Aufhell- bzw. Färbemitteln und/oder
- Verringerung sensorischer Irritationen bei der Anwendung von Aufhell- bzw. Färbemitteln.

Auch bezüglich bevorzugter Ausführungsformen der erfindungsgemäßen Verwendungen gilt mutatis mutandis das zu den erfindungsgemäßen Mitteln Ausgeführte.

### Beispiele:

### A1. Blondiercreme

| | **1** | **2** | **3** | **3** | **4** |
|---|---|---|---|---|---|
| Stenol 1618 | 10,00 | 10,00 | 10,00 | 10,00 | 10,00 |
| Kokoslorol C12-18 | 3,00 | 3,00 | 3,00 | 3,00 | 3,00 |
| Eumulgin B2 | 3,00 | 3,00 | 3,00 | 3,00 | 3,00 |
| Ammoniumsulfat | 1,00 | 1,00 | 1,00 | 1,00 | 1,00 |
| Turpinal SL | 0,20 | 0,20 | 0,20 | 0,20 | 0,20 |
| Gluadin W 40 | 4,00 | 4,00 | 4,00 | 4,00 | 4,00 |
| Ammoniak 25% | 0,62 | 0,62 | 0,62 | 0,62 | 0,62 |
| Vanillyl Butyl Ether | 0,50 | 0,50 | 0,40 | 0,35 | 0,50 |
| Allantoin | | 2,00 | | | |
| DL-alpha Bisabolol | | | 0,20 | | |
| Panthenol 75% | | | 1,00 | | |
| Valin | | | | 1,00 | |
| Hydagen CMF | | | | 5,00 | |
| Ectoin | | | | | 0,50 |
| Aqua | ad.100 | ad.100 | ad.100 | ad.100 | ad.100 |

- Stenol^{®} 1618: C₁₆₋₁₈-Fettalkohol (INCI-Bezeichnung: Cetearyl Alcohol) (Cognis)
- Kokoslorol^{®}: C12-18-Fettalkohol (INCI-Bezeichnung: Coconut Alcohol) (Cognis)
- Eumulgin^{®} B2: Cetylstearylalkohol mit ca. 20 EO-Einheiten (INCI-Bezeichnung: Ceteareth-20) (Cognis)
- Turpinal^{®}SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- Gluadin^{®} W40: Weizenproteinhydrolysat (mind. 40% Festkörper; INCI-Bezeichnung: Aqua (Water), Hydrolyzed Wheat Protein, Sodium Benzoate, Phenoxyethanol, Methylparaben, Propylparaben) (Cognis)
- Hydagen^{®} CMF: deacetyliertes Chitin (INCI-Bezeichnung: Chitosan) (Cognis)

### A2. Entwickler

| | |
|---|---|
| Lorol | 3,60 |
| Eumulgin B2 | 0,90 |
| Disponil FES 77 IS | 2,25 |
| Wasserstoffperoxid 50% | 24,0 |
| Turpinal SL | 1,50 |
| Aculyn 33A | 15,0 |
| Aqua | ad. 100 |

- Eumulgin^{®} B2: Cetylstearylalkohol mit ca. 20 EO-Einheiten (INCI-Bezeichnung: Ceteareth-20) (Cognis)
- Disponil^{®} FES 77: Fettalkoholethersulfat (ca. 31-33% Aktivsubstanzgehalt in Wasser; INCI-Bezeichnung: Sodium Coceth-30 Sulfate) (Cognis)
- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- Aculyn^{®} 33A: Acrylpolymer (ca. 28% Festkörper in Wasser; INCI-Bezeichnung: Acrylates Copolymer)
1:1-Mischungsverhältnis A1: A2

### B1. Färbecreme

| | **1** | **2** | **3** | **4** | **5** | **6** | **7** | **8** |
|---|---|---|---|---|---|---|---|---|
| Hydrenol D | 5,50 | 5,50 | 5,50 | 5,50 | 5,50 | 5,50 | 5,50 | 5,50 |
| Lorol 16 | 2,00 | 2,00 | 2,00 | 2,00 | 2,00 | 2,00 | 2,00 | 2,00 |
| Eumulgin B1 | 0,50 | 0,50 | 0,50 | 0,50 | 0,50 | 0,50 | 0,50 | 0,50 |
| Eumulgin B2 | 0,50 | 0,50 | 0,50 | 0,50 | 0,50 | 0,50 | 0,50 | 0,50 |
| Lamesoft PO 65 | 2,00 | 2,00 | 2,00 | 2,00 | 2,00 | 2,00 | 2,00 | 2,00 |
| Akypo Soft 45 NV | 10,00 | 10,00 | 10,00 | 10,00 | 10,00 | 10,00 | 10,00 | 10,00 |
| Texapon K 14 S 70% | 2,80 | 2,80 | 2,80 | 2,80 | 2,80 | 2,80 | 2,80 | 2,80 |
| Ammoniak 25% | 6,50 | 6,50 | 6,50 | 6,50 | 6,50 | 6,50 | 6,50 | 6,50 |
| Turpinal SL | 0,20 | 0,20 | 0,20 | 0,20 | 0,20 | 0,20 | 0,20 | 0,20 |
| p-Toluylendiamisulfat | 2,50 | 0,40 | 0,30 | 0,05 | 0,30 | 2,50 | 0,40 | 0,30 |
| Resorcin | 0,40 | 0,10 | 1,00 | 0,02 | 0,03 | 0,40 | 0,10 | 1,00 |
| 2,4-Diaminophenoxyethanol 2HCl | 2,00 | | | | | 2,00 | | |
| 2-Methylresorcin | | 1,00 | 0,03 | | 0,50 | | 1,00 | 0,03 |
| 2,4,5,6-Tetraaminopyrimidinsulfat | | 1,50 | | | | | 1,50 | |
| Bis-(5-Amino-2-hydroxyphenyl)methan 2HCl | | 0,06 | | | | | 0,06 | |
| m-Aminophenol | | | 0,01 | 0,03 | 0,02 | | | 0,01 |
| 4-Chorresorcin | | | 0,60 | 0,01 | 0,10 | | | 0,60 |
| Ascorbinsäure | 0,10 | 0,10 | 0,10 | 0,10 | 0,10 | 0,10 | 0,10 | 0,10 |
| Natriumsulfit 96% | 0,40 | 0,40 | 0,40 | 0,40 | 0,40 | 0,40 | 0,40 | 0,40 |
| Vanillyl Butyl Ether | 0,50 | 0,50 | 0,50 | 0,50 | 0,50 | 0,35 | 0,35 | 0,35 |
| Allantoin | | 2,00 | | | | | | |
| DL-alpha Bisabolol | | | 0,20 | | | 0,20 | | |
| Panthenol 75% | | | 1,00 | | | 1,00 | | |
| Valin | | | | 1,00 | | | 1,00 | |
| Hydagen CMF | | | | 5,00 | | | 5,00 | |
| Aqua | ad. 100 | ad. 100 | ad. 100 | ad. 100 | ad. 100 | ad. 100 | ad. 100 | ad. 100 |

- Hydrenol^{®} D: C₁₆₋₁₈-Fettalkohol (INCI-Bezeichnung: Cetearyl alcohol) (Cognis)
- Lorol^{®} C16: INCI-Bezeichnung: Cetyl Alcohol (Cognis)
- Eumulgin^{®} B 1: Cetylstearylalkohol mit ca. 12-EO-Einheiten (INCI-Bezeichnung: Ceteareth-12) (Cognis)
- Eumulgin^{®} B2: Cetylstearylalkohol mit ca. 20 EO-Einheiten (INCI-Bezeichnung: Ceteareth-20) (Cognis)
- Lamesoft^{®} PO65: Alkylpolyglucosid Ölsäuremonoglycerid Gemisch (ca. 65-70% Festkörper; INCI-Bezeichnung: Coco-Glucoside, Glyceryl Oleate, Aqua (Water)) (Cognis)
- Akypo Soft 45 NV^{®}: Laurylalkohol-4.5-EO-Essigsäure-Natrium-Salz (mind. 21% Aktivsubstanzgehalt; INCI-Bezeichnung: Sodium Laureth-6 Carboxylate) (Chem-Y)
- Texapon^{®} K 14 S 70 C: Laurylmyristylethersulfat-Natrium-Salz (ca. 68% bis 73% Aktivsubstanzgehalt'; INCI-Bezeichnung: Sodium Myreth Sulfate) (Cognis)
- Texapon^{®} K 14 S 70 C: Laurylmyristylethersulfat-Natrium-Salz (ca. 68% bis 73% Aktivsubstanzgehalt'; INCI-Bezeichnung: Sodium Myreth Sulfate) (Cognis)
- Hydagen® CMF: deacetyliertes Chitin (INCI-Bezeichnung: Chitosan) (Cognis)

### B2. Entwickler

| | |
|---|---|
| Dipicolinsäure | 0,10 |
| Dinatriumpyrophosphat | 0,03 |
| Turpinal SL | 1,50 |
| Texapon NSO | 2,00 |
| Aculyn 33A | 12,00 |
| Wasserstoffperoxid 50% | 12,00 |
| Ammoniak | 0,62 |
| Aqua | ad. 100 |

- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- Texapon^{®} NSO: Laurylethersulfat, Natriumsalz (ca. 27,5% Aktivsubstanz; INCI-Bezeichnung: Sodium Laureth Sulfate) (Cognis)
- Aculyn^{®} 33A: Acrylpolymer (ca. 28% Festkörper in Wasser; INCI-Bezeichnung: Acrylates Copolymer)
1:1-Mischungsverhältnis B1: B2

### C1. Färbecreme

| | **1** | **2** | **3** | **4** | **5** | **6** | **7** | **8** |
|---|---|---|---|---|---|---|---|---|
| Cetearyl Alkohol | 8,00 | 8,00 | 8,00 | 8,00 | 8,00 | 8,00 | 8,00 | 8,00 |
| Lorol | 3,00 | 3,00 | 3,00 | 3,00 | 3,00 | 3,00 | 3,00 | 3,00 |
| Eumulgin B2 | 2,00 | 2,00 | 2,00 | 2,00 | 2,00 | 2,00 | 2,00 | 2,00 |
| Ascorbinsäure | 0,50 | 0,50 | 0,50 | 0,50 | 0,50 | 0,50 | 0,50 | 0,50 |
| Natriumsulfit 96% | 0,50 | 0,50 | 0,50 | 0,50 | 0,50 | 0,50 | 0,50 | 0,50 |
| Turpinal SL | 0,20 | 0,20 | 0,20 | 0,20 | 0,20 | 0,20 | 0,20 | 0,20 |
| Gluadin W 40 | 4,00 | 4,00 | 4,00 | 4,00 | 4,00 | 4,00 | 4,00 | 4,00 |
| Ammoniak 25% | 6,00 | 6,00 | 6,00 | 6,00 | 6,00 | 6,00 | 6,00 | 6,00 |
| Resorcin | 0,10 | 8,00 | 8,00 | 8,00 | 8,00 | 8,00 | 8,00 | 8,00 |
| p-Toluylendiaminsulfat | 0,15 | 0,70 | 1,80 | 0,15 | 0,70 | 1,80 | 0,70 | 1,80 |
| 4-Cholrresorcin | 0,03 | 0,02 | 0,50 | 0,03 | 0,02 | 0,50 | 0,02 | 0,50 |
| 2-Amino-2-metylamino-6-methoxypyridin | 0,01 | | | 0,01 | | | | |
| 2,6-Dihydroxy-3,4-Dimethylpyridin | | 0,02 | | | 0,02 | | 0,02 | |
| 2,7-Dihydroxynaphthalin | | 0,01 | | | 0,01 | | 0,01 | |
| 2-Methylresorcin | | 0,05 | | | 0,05 | | 0,05 | |
| m-Aminophenol | | 0,10 | | | 0,10 | | 0,10 | |
| 2,4-Diaminophenoxyethanol 2HCl | | 0,01 | | | 0,01 | | 0,01 | |
| 3-Amino-2-methylamino-6-methoxypyridin | | 0,01 | 0,10 | | 0,01 | 0,10 | 0,01 | 0,10 |
| 1,3-Bis-(2,4-diaminophenoxy)propan 4HCl | | | 0,30 | | | 0,30 | | 0,30 |
| Vanillyl Butyl Ether | 0,50 | 0,35 | 0,40 | 0,50 | 0,35 | 0,40 | 0,35 | 0,40 |
| Allantoin | | 2,00 | | | | | | |
| DL-alpha Bisabolol | | | 0,20 | | | | 0,20 | |
| Panthenol 75% | | | 1,00 | | | | 1,00 | |
| Valin | | | | 1,00 | | | | 1,00 |
| Hydagen CMF | | | | 5,00 | | | | 5,00 |
| Aqua | ad. 100 | ad. 100 | ad. 100 | ad. 100 | ad. 100 | ad. 100 | ad. 100 | ad. 100 |

- Lorol^{®} C16: INCI-Bezeichnung: Cetyl Alcohol (Cognis)
- Eumulgin^{®} B2: Cetylstearylalkohol mit ca. 20 EO-Einheiten (INCI-Bezeichnung: Ceteareth-20) (Cognis)
- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- Gluadin^{®} W40: Weizenproteinhydrolysat (mind. 40% Festkörper; INCI-Bezeichnung: Aqua (Water), Hydrolyzed Wheat Protein, Sodium Benzoate, Phenoxyethanol, Methylparaben, Propylparaben) (Cognis)
- Hydagen^{®} CMF: deacetyliertes Chitin (INCI-Bezeichnung: Chitosan) (Cognis)

### C2. Entwickler

| | |
|---|---|
| Lorol | 3,60 |
| Eumulgin B2 | 0,90 |
| Disponil FES 77 IS | 2,25 |
| Wasserstoffperoxid 50% | 12,0 |
| Turpinal SL | 1,50 |
| Aculyn 33A | 15,0 |
| Aqua | ad. 100 |

- Lorol^{®} C16: INCI-Bezeichnung: Cetyl Alcohol (Cognis)
- Eumulgin^{®} B2: Cetylstearylalkohol mit ca. 20 EO-Einheiten (INCI-Bezeichnung: Ceteareth-20) (Cognis)
- Disponil^{®} FES 77: Fettalkoholethersulfat (ca. 31-33% Aktivsubstanzgehalt in Wasser; INCI-Bezeichnung: Sodium Coceth-30 Sulfate) (Cognis)
- Turpinal^{®}SL: 1-Hydroxyethan-1,1-diphosphonsäure (ca. 58 - 61% Aktivsubstanzgehalt; INCI-Bezeichnung: Etidronic Acid, Aqua (Water)) (Solutia)
- Aculyn^{®} 33A: Acrylpolymer (ca. 28% Festkörper in Wasser; INCI-Bezeichnung: Acrylates Copolymer)
1:1-Mischungsverhältnis C1: C2

## Patentansprüche

1. Mittel zum Färben und/oder Aufhellen von Keratinfasern, insbesondere menschlichen Haaren, enthaltend - jeweils bezogen auf sein Gewicht -
c) 0,001 bis 10 Gew.-% eines oder mehrerer Oxidationsfarbstoffvorprodukte und/oder direktziehender Farbstoffe;
d) 0,001 bis 5 Gew.-% mindestens einer ein Gefühl von Wärme erzeugenden Substanz.

2. Mittel zum Färben und/oder Aufhellen keratinischer Fasern nach Anspruch 1, **dadurch gekennzeichnet, daß** es 0,01 bis 4 Gew.-%, vorzugsweise 0,05 bis 3,5 Gew.-%, besonders bevorzugt 0,1 bis 3 Gew.-% und insbesondere 0,2 bis 2,5 Gew.-% mindestens einer ein Gefühl von Wärme erzeugenden Substanz enthält.

3. Mittel zum Färben und/oder Aufhellen keratinischer Fasern nach einem der Ansprüche 1 oder 2, **dadurch gekennzeichnet, daß** es als ein Gefühl von Wärme erzeugende Substanz mindestens einen Ether von Vanillylakohol enthält, vorzugsweise Vanillylalkohol methyl-ether und/oder Vanillylalkohol ethyl-ether, und/oder Vanillylalkohol n-propyl-ether und/oder Vanillylalkohol isopropyl-ether und/oder Vanillylalkohol n-butyl-ether und/oder Vanillylalkohol isobutyl-ether und/oder Vanillylalkohol n-amyl-ether und/oder Vanillylalkohol isoamyl-ether und/oder Vanillylalkohol n-hexyl-ether.

4. Mittel zum Färben und/oder Aufhellen keratinischer Fasern nach einem der Ansprüche 1 bis 3, **dadurch gekennzeichnet, daß** es als ein Gefühl von Wärme erzeugende Substanz mindestens eine Verbindung aus der Gruppe
- der Nicotinsäureester, vorzugsweise Methylnicotinat und/oder Ethylnicotinat und/oder Benzylnicotinat und/oder Butoxyethylnicotinat und/oder Hexylnicotinat und/oder Isopropylnicotinat und/oder Myristylnicotinat und/oder Thurfylniconinat und/oder
- Gingerol und/oder
- Shogaol und/oder
- Paradol und/oder
- Zingeron und/oder
- Capsaicin und/oder Capsaicin-haltiger Extrakte und/oder
- Dihydrocapsaicin und/oder
- Nordihydrocapsaicin und/oder
- Homocapsaicin und/oder
- Homodihydrocapsaicin und/oder
- Norcapsaicin und/oder
- Nordihydrocapsaicin
enthält.

5. Mittel zum Färben und/oder Aufhellen keratinischer Fasern nach einem der Ansprüche 1 bis 4, **dadurch gekennzeichnet, daß** es als ein Gefühl von Wärme erzeugende Substanz mindestens einen Scharfstoff aus der Gruppe der N-Alkyl-substituierte Amide von ungesättigten Carbonsäuren, vorzugsweise
- 2E,6Z,8E-Decatriensäure-N-isobutylamid (Spilanthol) und/oder
- 2E,4E,8Z-Decatriensäure-N-isobutylamid und/oder
- 2E,7Z,9E-Undecatriensäure-N-isobutylamid und/oder
- 2E,4Z-Decadiensäure-N-isobutylamid (cis-Pellitorin) und/oder
- 2E,4E-Decadiensäure-N-isobutylamid (trans-Pellitorin) und/oder
- Ferulasäure-N-Vanillylamid und/oder
- *N*-[2-(4-Hydroxy-3-methoxyphenyl)ethyl]-3-(4-hydroxy-3-methoxyphenyl)- (2*E*)-propensäureamid (trans-Feruloylmethoxytyramin) und/oder
- N-[2- (4-Hydroxy-3-methoxyphenyl)ethyl]-3-(4-hydroxy-3-methoxyphenyl)-(2 Z)-propensäureamid (cis-Feruloylmethoxytyramin) und/oder
- *N*-[2-(4-Hydroxy-3-methoxyphenyl)ethyl]-3-(4-hydroxy-3-methoxyphenyl)- propansäureamid (Dihydroferuloylmethoxytyramin) und/oder
- N-[2-(3,4- Dihydroxyphenyl)ethyl]-3-(4-hydroxy-3-methoxy-phenyl)-(2*E*)-propensäureamid (trans-Feruloyldopamin) und/oder
- *N*-[2-(3,4-Dihydroxyphenyl) ethyl]-3-(4-hydroxy-3-methoxy-phenyl)-(2*Z*)-propensäureamid (cis- Feruloyldopamin) und/oder
- *N*-[2-(4-Hydroxyphenyl)ethyl]-3-(3,4- dihydroxyphenyl)-(2*E*)-propensäureamid (trans-Caffeoyltyramin) und/oder
- N-[2-(4-Hydroxyphenyl)ethyl]-3-(3,4-dihydroxyphenyl)-(2Z)-propensäureamid (cis-Caffeoyltyramin) und/oder
- *N*-[2-(3,4-Dimethoxyphenyl) ethyl]-3-(3,4-dimethoxyphenyl)-(2*E*)-propensäureamid (trans- Rubenamin) und/oder
- *N*-[2-(3,4-dimethoxyphenyl)ethyl]-3-(3,4- dimethoxy-phenyl)-(2*Z*)-propensäureamid (cis-Rubenamin)
enthält.

6. Mittel zum Färben und/oder Aufhellen keratinischer Fasern nach einem der Ansprüche 1 bis 5, **dadurch gekennzeichnet, daß** als ein Gefühl von Wärme erzeugende Substanz mindestens einen Scharfstoff aus den Gruppen der
- alkylsubstituierten Dioxane der Formel 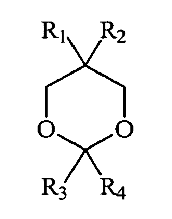 in der R1 und R2 unabhängig voneinander ausgewählt sind aus -H, -CH₃, - CH₂CH₃ und R3 und R4 unabhängig voneinander ausgewählt sind aus -H, -CH₃, -CH₂CH₃, -CH₂CH₂CH₃, -CH(CH₃)₂ und/oder
- Phenylestern der Formel 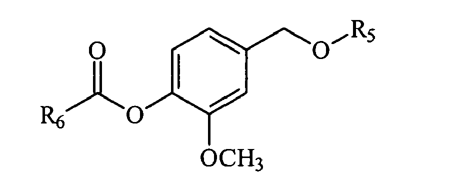 in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht und/oder
- Carvonacetalen der Formel 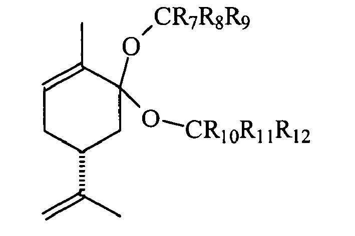 in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃ oder R9 und R10 zusammen eine chemiche Bindung oder eine Gruppe -(CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃,
- CH(CH₃)₂, -CH₂ CH₂CH₂CH₃, -CH₂CH(CH₃)₂, -CH(CH₃)CH₂CH₃, -C(CH₃ₕ enthält.

7. Mittel zum Färben und/oder Aufhellen keratinischer Fasern nach einem der Ansprüche 1 bis 6, **dadurch gekennzeichnet, daß** es zusätzlich Vitamine und/oder Pro-Vitamine und/oder Vitaminvorstufen enthält, die vorzugsweise den Gruppen A, B, C, E, F und H zugeordnet werden, wobei bevorzugte Mittel die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.

8. Mittel zum Färben und/oder Aufhellen keratinischer Fasern nach einem der Ansprüche 1 bis 7, **dadurch gekennzeichnet, daß** es zusätzlich 0,05 bis 15 Gew.-% mindestens eines 2-Furanonderivats der Formel (I) und/oder der Formel (II) 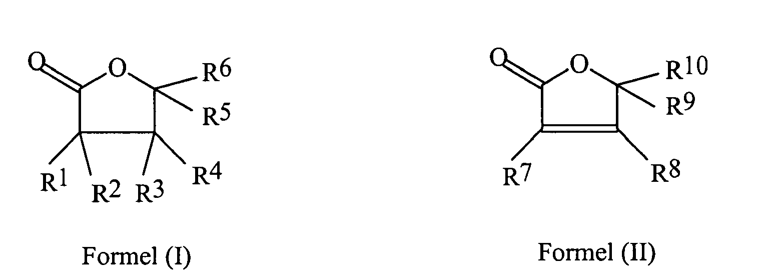 enthält, in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen - C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

9. Mittel zum Färben und/oder Aufhellen keratinischer Fasern nach einem der Ansprüche 1 bis 8, **dadurch gekennzeichnet, daß** es zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 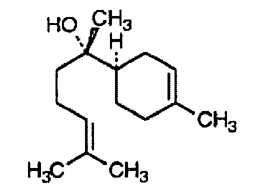 enthält.

10. Mittel zum Färben und/oder Aufhellen keratinischer Fasern nach einem der Ansprüche 1 bis 9, **dadurch gekennzeichnet, daß** es zusätzlich - 0,05 bis 5 Gew.%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthält.

11. Mittel zum Färben und/oder Aufhellen keratinischer Fasern nach einem der Ansprüche 1 bis 10, **dadurch gekennzeichnet, daß** es zusätzlich - 0,01 bis 5 Gew.-%, vorzugsweise 0,02 bis 2,5 Gew.-%, besonders bevorzugt 0,05 bis 1 Gew.-% und insbesondere 0,1 bis 0,5 Gew.-% Chitosanglycolat oder N-Acetyl-Chitosan oder N-Succinyl-Chitosan enthält.

12. Mittel zum Färben und/oder Aufhellen keratinischer Fasern nach einem der Ansprüche 1 bis 11, **dadurch gekennzeichnet, daß** es 0,5 bis 15 Gew.-%, vorzugsweise 1 bis 12,5 Gew.-%, besonders bevorzugt 2,5 bis 10 Gew.-% und insbesondere 3 bis 7 Gew.-% Wasserstoffperoxid (berechnet als 100%iges H₂O₂) enthält.

13. Mittel zum Färben und/oder Aufhellen keratinischer Fasern nach einem der Ansprüche 1 bis 12, **dadurch gekennzeichnet, daß** es als Oxidationsfarbstoffvorprodukt mindestens eine Entwicklerkomponente enthält, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'- diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol.

14. Mittel zum Färben und/oder Aufhellen keratinischer Fasern nach einem der Ansprüche 1 bis 13, **dadurch gekennzeichnet, daß** es als Oxidationsfarbstoffvorprodukt mindestens eine Kupplerkomponente enthält, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

15. Mittel zum Färben und/oder Aufhellen keratinischer Fasern nach einem der Ansprüche 1 bis 14, **dadurch gekennzeichnet, daß** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

16. Verfahren zum Färben und/oder Aufhellen von Keratinfasern, insbesondere menschlichen Haaren, **dadurch gekennzeichnet, daß**
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein Mittel nach einem der Ansprüche 1 bis 14 ist.

17. Verwendung mindestens einer Substanz, die ein Gefühl von Wärme erzeugt, in Mitteln zur Behandlung keratinischer Fasern, insbesondere in haarkosmetischen Mitteln für Humanhaar.

18. Verwendung mindestens einer Substanz, die ein Gefühl von Wärme erzeugt, in Mitteln zum Färben und/oder Aufhellen keratinischer Fasern.

19. Verwendung nach Anspruch 18 zur
- Steigerung der Aufhell- bzw. Färbeleistung und/oder
- Steigerung der Hautverträglichkeit von Aufhell- bzw. Färbemitteln und/oder
- Anzeige der Einwirkzeit von Aufhell- bzw. Färbemitteln und/oder
- Verringerung sensorischer Irritationen bei der Anwendung von Aufhell- bzw. Färbemitteln.
